# EUROPEAN PATENT SPECIFICATION

(11) **EP 1 809 639 B1**
(45) Date of publication and mention of the grant of the patent: **14.10.2009**
(21) Application number: 05857829.5
(22) Date of filing: 10.11.2005
(51) Int. Cl.: C07D 513/04, A61K 31/437, A61P 31/04, A61P 33/02

(54) **8A, 9-DIHYDRO-4A-H-ISOTHIAZOLO[5,4-B] QUINOLINE-3, 4-DIONES AND RELATED COMPOUNDS AS ANTI-INFECTIVE AGENTS**
8A, 9-DIHYDRO-4A-H-ISOTHIAZOLO[5,4-B] CHINOLIN-3, 4-DIONE UND VERWANDTE VERBINDUNGEN ALS INFEKTIONSSCHUTZMITTEL
8A, 9-DIHYDRO-4A-H-ISOTHIAZOLO[5,4-B] QUINOLINE-3, 4-DIONES ET COMPOSES AFFERENTS COMME AGENT ANTIINFECTIEUX

(30) Priority: 11.11.2004 US 626961 P
(43) Date of publication of application: 25.07.2007
(73) Proprietor: Achillion Pharmaceuticals, Inc., New Haven, CT 06511 (US)
(72) Inventor: BRADBURY, Barton, James, Wallingford, CT 06492 (US); DESHPANDE, Milind, Madison, CT 06443 (US); PUCCI, Michael, John, Kensington, CT 06037 (US); WANG, Qiuping, Bethany, CT 06524 (US); WILES, Jason, Allan, Hamden, CT 06518 (US); SONG, Yongsheng, Guilford, CT 06437 (US); HASHIMOTO, Akihiro, Branford, CT 06405 (US); LUCIEN, Edlaine, New Haven, CT 06511 (US)
(74) Representative: Smaggasgale, Gillian Helen
(86) International application number: PCT/US2005/040725
(87) International publication number: WO 2006/118605

(56) References cited:
- EP-A- 0 541 086
- WO-A-02/48138
- US-A- 4 767 762
- US-A- 5 087 621

## Description

### FIELD OF THE INVENTION

The present invention provides an 8a,9-dihydro-4a-H-isothiazolo[5,4-b]quinoline-3,4-diones, many of which are substituted at the 7-position with an oxime substituted heterocycloalkyl substituent, and related compounds, which possess antimicrobial activity. Certain compounds provided herein possess potent antibacterial, antiprotozoal, or antifungal activity. Particular compounds provided herein are also potent and/or selective inhibitors of prokaryotic DNA synthesis and prokaryotic reproduction. The invention provides anti-microbial compositions, including pharmaceutical compositions, containing one or more carrier, diluents, or excipients. The invention provides pharmaceutical compositions containing an 8a,9-dihydro-4a-H-isothiazolo[5,4-b]quinoline-3,4-dione or related compound as the only active agent or containing an 8a,9-dihydro-4a-H-isothiazolo[5,4-b]quinoline-3,4-dione or related compound in combination with one or more other active agent, such as one or more other antimicrobial or antifungal agent. The invention provides methods for treating or preventing microbial infections in eukaryotes by administering an effective amount of an 8a,9-dihydro-4a-H-isothiazolo[5,4-b]quinoline-3,4-dione or related compound to a eukaryote suffering from or susceptible to microbial infection. The invention also provides methods of inhibiting microbial growth and survival by applying an effective amount of an 8a,9-dihydro-4a-H-isothiazolo[5,4-b]quinoline-3,4-dione or related compound.
The invention also provides novel intermediates useful for the synthesis of 8a,9-dihydro-4a-H-isothiazolo[5,4-b]quinoline-3,4-diones and related compounds. The invention also provides methods of synthesis for 8a,9-dihydro-4a-H-isothiazolo[5,4-b]quinoline-3,4-diones and related compounds.

### BACKGROUND OF THE INVENTION

Antimicrobial compounds are compounds capable of destroying or suppressing the growth or reproduction of microorganisms, such as bacteria, protozoa, mycoplasma, yeast, and fungi. The mechanisms by which antimicrobial compounds act vary. However, they are generally believed to function in one or more of the following ways: by inhibiting cell wall synthesis or repair; by altering cell wall permeability; by inhibiting protein synthesis; or by inhibiting synthesis of nucleic acids. For example, beta-lactam antibacterials inhibit the essential penicillin binding proteins (PBPs) in bacteria, which are responsible for cell wall synthesis. Quinolones act, at least in part, by inhibiting synthesis of DNA, thus preventing the cell from replicating.

Many attempts to produce improved antimicrobials yield equivocal results. Indeed, few antimicrobials are produced that are truly clinically acceptable in terms of their spectrum of antimicrobial activity, avoidance of microbial resistance, and pharmacology. Thus there is a continuing need for broad-spectrum antimicrobials, and a particular need for antimicrobials effective against resistant microbes.

Pathogenic bacteria are known to acquire resistance via several distinct mechanisms including inactivation of the antibiotic by bacterial enzymes (e.g., beta-lactamases that hydrolyze penicillin and cephalosporins); removal of the antibiotic using efflux pumps; modification of the target of the antibiotic via mutation and genetic recombination (e.g., penicillin-resistance in *Neiserria gonorrhea*); and acquisition of a readily transferable gene from an external source to create a resistant target (e.g., methicillin-resistance in *Staphylococcus aureus*). There are certain Gram-positive pathogens, such as vancomycin-resistant *Enterococcus faecium,* which are resistant to virtually all commercially available antibiotics.

Resistant organisms of particular note include methicillin-resistant and vancomycin- resistant *Staphylococcus aureus,* penicillin-resistant *Streptococcus pneumoniae,* vancomycin-resistant enterococci, fluoroquinolone-resistant *E*. *coli,* cephalosporin-resistant aerobic gram-negative rods and imipenem- resistant *Pseudomonas aeruginosa.* These organisms are significant causes of nosocomial infections and are clearly associated with increasing morbidity and mortality. The increasing numbers of elderly and immunocompromised patients are particularly at risk for infection with these pathogens. Therefore, there is a large unmet medical need for the development of new antimicrobial agents.
EP 0541086 discloses antibacterial 6-fluoroquinolones having an oxime on the substituent in position 7.

### SUMMARY OF THE INVENTION

The invention provides compounds of Formula I and Formula II (shown below) and includes 8a,9-dihydro-4a-H-isothiazolo[5,4-b]quinoline-3,4-diones and related compounds, which possess antimicrobial activity. The invention provides compounds of Formula I and Formula II, which possess potent and/ or selective antibacterial, antiprotozoal, or antifungal activity. The invention also provides compositions containing one or more compounds of Formula I or Formula II, or a salt, solvate, or prodrug, such as an acylated prodrug of such a compound, and one or more carriers, excipients, or diluents.

The invention further comprises the compounds of formula I or II for use in methods of treating and preventing microbial infections, particularly bacterial and protozoal infections by administering an effective amount of a compound of Formula I or Formula II to a eukaryote suffering from or susceptible to a microbial infection. These microbial infections include bacterial infections, for example *E*. *coli* infections, *Staphylococcus* infections, *Salmonella* infections and protozoal infections, for example *Chlamydia* infections. The invention particularly includes methods of preventing or treating microbial infections in mammals, including humans, but also encompasses methods of preventing or treating microbial infections in other animals, including fish, birds, reptiles, and amphibians.

Methods of treatment include administering a compound of Formula I or Formula II as the single active agent or administering a compound of Formula I or Formula II in combination with one or more other therapeutic agent, such as an antibacterial, an antifungal, an antiviral, an interferon or other immune system modulator, an efflux-pump inhibitor, a beta-lactamase inhibitor, an anti-inflammatory, or another compound of Formula I or Formula II.

The invention also provides the compounds of formula I or II for use in methods of inhibiting microbial growth and survival by applying an effective amount of an 8a,9-dihydro-4a-H-isothiazolo[5,4-b]quinoline-3,4-dione or related compound. The invention includes, for example, the compounds of formula I or II for use in methods of inhibiting microbial growth and survival on medical instruments or on surfaces used for food preparation by applying a composition containing a compound of Formula I or Formula II.

Thus, the invention include compounds and pharmaceutically acceptable salts of Formula I and Formula II 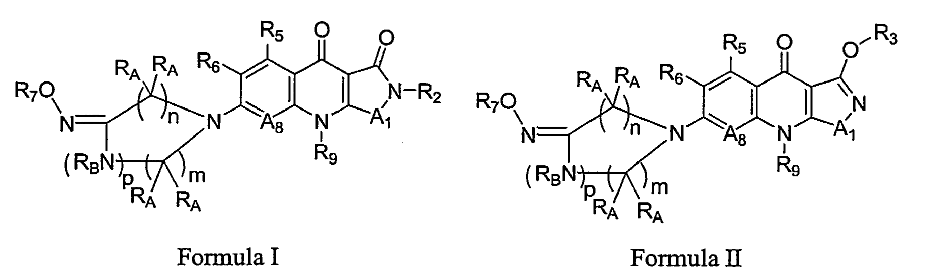 Within Formula I and Formula II:

A₁ is S, O, SO, or SO₂.

R₂ is hydrogen, or R₂ is C₁-C₈alkyl, C₂-C₆alkenyl, C₂-C₆alkynyl, (C₃-C7cycloalkyl)C₀-C₄carbohydryl, (C₄-C₇cycloalkenyl)C₀-C₄carbohydryl, (aryl)C₀-C₄carbohydryl, or (C₂-C₆heterocycloalkyl)C₀-C₄carbohydryl, each of which is substituted with 0 to 5 substituents independently chosen from halogen, hydroxy, amino, cyano, nitro, C₁-C₄alkyl, C₁-C₄alkoxy, C₁-C₂haloalkyl, C₁-C₂haloalkoxy, mono- and di-C₁-C₄alkylamino, C₂-C₄alkanoyl, C₁-C₄alkylthio, -O(C=O)R₁₀, -(C=O)NR₁₀R₁₁, -O(C=O)NR₁₀R₁₁, -(C=O)OR₁₀, -(C=O)NR₁₀OR₁₁, -NR₁₀(C=O)R₁₁, -NR₁₀(C=O)OR₁₁, -NR₁₀(C=O)NR₁₁R₁₂, -NR₁₀(C=S)NR₁₁R₁₂, - NR₁₀NR₁₁R₁₂, -SO₃R₁₀, -(S=O)OR₁₀, -SO₂R₁₃, -SO₂NR₁₀R₁₁, =N-OR₁₀, and -NR₁₀SO₂R₁₃; where each R₁₀, R₁₁, and R₁₂ is independently hydrogen, C₁-C₄alkyl, or aryl, and each R₁₃ is independently C₁-C₄alkyl or aryl.

R₃ is hydrogen, C₁-C₆akyl, C₂-C₆alkanoyl, mono- or di-C₁-C₆alkylcarbamate, C₁-C₆akylphosphate, or C₁-C₆alkylsulfonate; each of which is substituted with 0 to 3 substituents independently chosen from halogen, hydroxy, amino, cyano, nitro, C₁-C₄alkoxy, mono- and di-C₁-C₄alkylamino, C₁-C₂haloalkyl, and C₁-C₂haloalkoxy.

R₅ is hydrogen, halogen, hydroxy, amino, cyano, nitro, -NHNH₂, C₁-C₂haloalkyl, or C₁-C₂haloalkoxy; or

R₅ is C₁-C₄akyl, C₁-C₄alkoxy, mono- or di-C₁-C₄alkylamino, mono-, di-, or tri-C₁-C₄ alkylhydrazinyl, C₂-C₄alkanoyl, or C₁-C₄alkylester,; each of which is substituted with 0 to 3 substituents independently chosen from hydroxy, amino, halogen, oxo, C₁-C₄alkoxy, C₁-C₂haloalkyl, C₁-C₂haloalkoxy, and mono- and di-C₁-C₄alkylamino.

R₆ is hydrogen, halogen, hydroxy, amino, cyano, C₁-C₄alkyl, C₁-C₄alkoxy, mono- or di-C₁-C₄alkylamino, -SO₃R₁₀, -SO₂R₁₀, -SO₂NR₁₀R₁₁; where R₁₀ and R₁₁ carry the definitions set forth above.

n is 1, 2, or 3; m is 1, 2, or 3; and p is 0 or 1.

Each R_{A} is independently (i), (ii), or (iii); where
(i) is hydrogen, hydroxy, amino, or cyano,
(ii) is C₁-C₆alkyl, C₂-C₆alkenyl, C₂-C₆alkynyl, C₁-C₆alkoxyC₀-C₄alkyl, mono- or di-C₁-C₄alkylamino, C₁-C₂haloalkoxy, (C₃-C₇cycloalkyl)C₀-C₄carbohydryl, (C₄-C₇cycloalkenyl)C₀-C₄carbohydryl, (aryl)C₀-C₆carbohydryl, (aryl)C₁-C₄alkoxy, (C₂-C₆heterocycloalkyl)C₀-C₄carbohydryl, (heteroaryl)C₀-C₆carbohydryl, C₁-C₆alkylthio, -(C₀-C₄alkyl)O(C=O)R₁₀, -(C₀-C₄alkyl)(C=O)NR₁₀R₁₁, -(C₀-C₄alkyl)O(C=O)NR₁₀R₁₁, -(C₀-C₄alkyl) (C=O)OR₁₀, -(C₀-C₄alkyl)NR₁₀(C=O)R₁₁, -(C₀-C₄alkyl)NR₁₀(C=O)OR₁₁, -(C₀-C₄alkyl)NR₁₀(C=O)NR₁₁R₁₂, -(C₀-C₄alkyl)NR₁₀(C=S)NR₁₁R₁₂, -(C₀-C₄alkyl)NR₁₀NR₁₁R₁₂, -(C₀-C₄alkyl)N=NR₁₃, -(C₀-C₄alkyl)SO₃R₁₀, -(Co-C₄alkyl)(S=O)OR₁₀, -(C₀-C₄alkyl)SO₂R₁₃, -(C₀-C₄alkyl)SO₂NR₁₀R₁₁, -(Co-C₄alkyl)NR₁₀SO₂R₁₃; =N-OH, or=N-O(C₀-C₄alkyl;
(iii) is -OR_{D}, -(C=O)R_{D}, -SO₂R_{D}, -SO₃R_{D}, or -NR₁₀S0₂R_{D}, where R_{D} is C₁-C₄alkyl, (C₃-C₇cycloalkyl)C₀-C₂alkyl, (C₂-C₆heterocycloalkyl)C₀-C₂alkyl, (aryl)C₀-C₂alkyl, or (heteroaryl)C₀-C₂alkyl; where each of (ii) and (iii) is substituted with 0 to 3 substituents independently chosen from halogen, hydroxy, amino, cyano, nitro, -COOH, -(C=O)OCH₃, - CONH₂, C₁-C₄akyl, C₂-C₄alkenyl, C₂-C₄alkynyl, C₁-C₄alkoxy, (C₃-C₇cycloalkyl)C₀-C₄carbohydryl, (C₃-C₄cycloalkyl)C₀-C₄alkoxy, mono- and di-C₁-C₄alkylamino, C₁-C₂haloalkyl, C₁-C₂haloalkoxy, and C₂-C₄alkanoyl.
Or, any two R_{A} bound to the same carbon atom may be joined to form a C₃-C₇cycloalkyl, or a 3- to 7- membered heterocycloalkyl group; having 1 or 2 heteroatoms independently chosen from N, O, and S; each of which is substituted with 0 to 2 substituents independently chosen from C₁-C₂alkyl, and C₁-C₂alkoxy.
Or, any two R_{A} bound to different carbon atoms may be joined to form a C₃-C₇cycloalkyl or a 3- to 7-membered heterocycloalkyl group having 1 or 2 heteroatoms independently chosen from N, O, and S; each of which is substituted with 0 to 2 substituents independently chosen from C₁-C₂alkyl, and C₁-C₂alkoxy.
R_{B} is hydrogen or C₁-C₄alkyl.

R₇ is hydrogen, or R₇ is C₁-C₈alkyl, C₂-C₆alkenyl, C₂-C₆akynyl, C₂-C₆alkanoyl, (C₃-C₇cycloalkyl)C₀-C₄carbohydryl, (C₄-C₇cycloalkenyl)C₀-C₄carbohydryl, (aryl)C₀-C₄carbohydryl, (aryl)(C=O)-, mono- or di-(C₁-C₆alkyl)carboxamide, C₁-C₆alkylester, or (C₂-C₆heterocycloalkyl)C₀-C₄carbohydryl, each of which is substituted with 0 to 5 substituents independently chosen from halogen, hydroxy, amino, cyano, nitro, C₁-C₄alkyl, C₁-C₄alkoxy, C₁-C₂haloalkyl, C₁-C₂haloalkoxy, mono- and di-C₁-C₄alkylamino, C₂-C₄alkanoyl, C₁-C₄alkylthio, -O(C=O)R₁₀, -(C=O)NR₁₀R₁₁, -O(C=O)NR₁₀R₁₁, -(C=O)OR₁₀, -(C=O)NR₁₀OR₁₁, -NR₁₀(C=O)R₁₁, -NR₁₀(C=O)OR₁₁, -NR₁₀(C=O)NR₁₁R₁₂, -NR₁₀(C=S)NR₁₁R₁₂, -NR₁₀NR₁₁R₁₂, -SO₃R₁₀, -(S=O)OR₁₀, -SO₂R₁₃, -SO₂NR₁₀R₁₁, or -NR₁₀SO₂R₁₃.

A₈ is nitrogen or CR₈; where R₈ is hydrogen, halogen, hydroxy, amino, cyano, nitro, or NHNH₂, or R₈ is C₁-C₄alkyl, C₁-C₄alkoxy, mono- or di-C₁-C₄akylamino, mono-, di-, or tri-C₁-C₄ alkylhydrazinyl, C₂-C₄akanoyl, C₁-C₄alkylester, C₁-C₂haloalkyl, or C₁-C₂haloalkoxy, each of which is substituted with 0 to 3 substituents independently chosen from hydroxy, amino, halogen, oxo, C₁-C₄allcoxy, C₁-C₂haloalkyl, C₁-C₂haloalkoxy, and mono- and di-C₁-C₄alkylamino.

R₉ is C₁-C₈alkyl, (C₃-C₇cycloalkyl)C₀-C₄alkyl, or phenyl, each of which is substituted with 0 to 3 substituents independently chosen from halogen, hydroxy, amino, cyano, nitro, -COOH, -CONH₂, C₁-C₄alkyl, C₂-C₄alkenyl, C₂-C₄alkynyl, C₁-C₄alkoxy, (C₃-C₇cycloalkyl)C₀-C₄alkyl, (C₃-C₇cycloalkyl)C₀-C₄alkoxy, mono- and di-C₁-C₄alkylamino, C₁-C₂haloalkyl, C₁-C₂haloalkoxy, and C₂-C₄alkanoyl.
The invention includes novel intermediates useful for the synthesis of compounds of Formula I and Formula II. The invention provides methods of synthesizing compounds of Formula I and Formula II comprising coupling an intermediate, wherein the intermediate is a compound of Formula I or Formula II in which R₇ is bromo, iodo, -O(SO₂)CF₃, or -N₂BF₄ to an appropriate aryl or heteroaryl boronic acids, aryl or heteroaryl boronic acid esters, or compounds substituted with Li, Mg, B, Al, Si, Zn, Cu, Zr, or Sn at the point of coupling.

### DETAILED DESCRIPTION OF THE INVENTION

### CHEMICAL DESCRIPTION AND TERMINOLOGY

Prior to setting forth the invention in detail, it may be helpful to provide definitions of certain terms to be used herein. Compounds of the present invention are generally described using standard nomenclature.

In certain situations, the compounds of Formula I and Formula II may contain one or more asymmetric elements such as stereogenic centers, stereogenic axes and the like, e.g. asymmetric carbon atoms, so that the compounds can exist in different stereoisomeric forms. These compounds can be, for example, racemates or optically active forms. For compounds with two or more asymmetric elements, these compounds can additionally be mixtures of diastereomers. For compounds having asymmetric centers, it should be understood that all of the optical isomers and mixtures thereof are encompassed. In addition, compounds with carbon-carbon double bonds may occur in Z- and E-forms, with all isomeric forms of the compounds being included in the present invention. In these situations, the single enantiomers, i.e., optically active forms can be obtained by asymmetric synthesis, synthesis from optically pure precursors, or by resolution of the racemates. Resolution of the racemates can also be accomplished, for example, by conventional methods such as crystallization in the presence of a resolving agent, or chromatography, using, for example a chiral HPLC column.

Where a compound exists in various tautomeric forms, the invention is not limited to any one of the specific tautomers, but rather includes all tautomeric forms.

The present invention is intended to include all isotopes of atoms occurring in the present compounds. Isotopes include those atoms having the same atomic number but different mass numbers. By way of general example, and without limitation, isotopes of hydrogen include tritium and deuterium and isotopes of carbon include ¹¹C, ¹³C, and ¹⁴C.

Certain compounds are described herein using a general formula that includes variables, e.g. A₁, R₂, R₃, R₅, R₆, R₇, A₈, and R₉. Unless otherwise specified, each variable within such a formula is defined independently of other variables. Thus, if a group is said to be substituted, e.g. with 0-2 R*, then said group may be substituted with up to two R* groups and R* at each occurrence is selected independently from the definition of R*. Also, combinations of substituents and/or variables are permissible only if such combinations result in stable compounds. When a group is substituted by an "oxo" substituent a carbonyl bond replaces two hydrogen atoms on a carbon. An "oxo" substituent on an aromatic group or heteroaromatic group destroys the aromatic character of that group, e.g. a pyridyl substituted with oxo is a pyridone.

The term "substituted", as used herein, means that any one or more hydrogens on the designated atom or group is replaced with a selection from the indicated group, provided that the designated atom's normal valence is not exceeded. When a substituent is oxo (i.e., =O), then 2 hydrogens on the atom are replaced. Combinations of substituents and/or variables are permissible only if such combinations result in stable compounds or useful synthetic intermediates. A stable compound or stable structure is meant to imply a compound that is sufficiently robust to survive isolation from a reaction mixture, and subsequent formulation into an effective therapeutic agent. Unless otherwise specified substituents are named into the core structure. For example, it is to be understood that when (cycloalkyl)alkyl is listed as a possible substituent the point of attachment of this substituent to the core structure is in the alkyl portion.

The exception to naming substituents into the ring is when the substituted is listed with a dash ("-") or double bond ("=") that is not between two letters or symbols. In that case the dash or double bond symbol is used to indicate a point of attachment for a substituent. For example, -CONH2 is attached through the carbon atom.

As used herein, "alkyl" is intended to include both branched and straight-chain saturated aliphatic hydrocarbon groups, having the specified number of carbon atoms. Thus, the term C₁- C₆ alkyl as used herein includes alkyl groups having from 1 to about 6 carbon atoms. When C₀-Cₙ alkyl is used herein in conjunction with another group, for example, (aryl)C₀-C₄ alkyl, the indicated group, in this case aryl, is either directly bound by a single covalent bond (C₀), or attached by an alkyl chain having the specified number of carbon atoms, in this case from 1 to about 4 carbon atoms. Examples of alkyl include, but are not limited to, methyl, ethyl, n-propyl, isopropyl, n-butyl, t-butyl, n-pentyl, and sec-pentyl.

"Alkenyl" as used herein, indicates a hydrocarbon chain of either a straight or branched configuration having one or more carbon-carbon double bonds, which may occur at any stable point along the chain. Examples of alkenyl groups include ethenyl and propenyl.

"Alkynyl" as used herein, indicates a hydrocarbon chain of either a straight or branched configuration having one or more triple carbon-carbon bonds that may occur in any stable point along the chain, such as ethynyl and propynyl.

"Carbohydryl" as used herein, includes both branched and straight-chain hydrocarbon groups, which are saturated or unsaturated, having the specified number of carbon atoms. When C0-Cn carbohydryl is used herein in conjunction with another group, for example, (aryl)C0-C4carbohydryl, the indicated group, in this case aryl, is either directly bound by a single covalent bond (C0), or attached by a carbohydryl chain, such as an alkyl chain, having the specified number of carbon atoms, in this case from 1 to about 4 carbon atoms. Examples include C1-C6alkyl, such as methyl, 5-butyl, C2-C6alkynyl such as hexynyl, and C2-C6 alkenyl, such as 1-propenyl.

"alkoxy' represents an alkyl group as defined above with the indicated number of carbon atoms attached through an oxygen bridge. Examples of alkoxy include, but are not limited to, methoxy, ethoxy, n- propoxy, i- propoxy, n-butoxy, 2-butoxy, t-butoxy, n-pentoxy, 2-pentoxy, 3-pentoxy, isopentoxy, neopentoxy, n-hexoxy, 2-hexoxy, 3-hexoxy, and 3- methylpentoxy. An "(alkoxy)alkyl group is an alkoxy group as defined herein attached through its oxygen atom to an alkyl bridge where the point of attachment to the substituted group is in the alkyl group.

"Alkanoyl" indicates an alkyl group as defined above, attached through a keto (-(C=O)-) bridge. Alkanoyl groups have the indicated number of carbon atoms, with the carbon of the keto group being included in the numbered carbon atoms. For example a C2alkanoyl group is an acetyl group having the formula CH3(C=O)-.

As used herein, the terms "mono- or di-alkylamino" or "mono- and di-alkylamino" indicate secondary or tertiary alkyl amino groups, wherein the alkyl groups are as defined above and have the indicated number of carbon atoms. The point of attachment of the alkylamino group is on the nitrogen. Examples of mono- and di-alkylamino groups include ethylamino, dimethylamino, and methyl-propyl-amino.

The term "mono- or di-alkylcarbamate" indicates 1 or 2 independently chosen alkyl groups, as defined above, attached through a carbamate (-O(C=O)NRR) linkage where R represents the alkyl groups. Mono-alkylcarbamate groups have the formula (-O(C=O)NHR).

The term "alkylester" indicates an alkyl group as defined above attached through an ester linkage. The ester linkage may be in either orientation, e.g. a group of the formula -O(C=O)alkyl or a group of the formula -(C=O)Oakyl.

The term "mono-, di-, or tri-alkylhydrazinyl" indicates from 1 to 3 independently chosen alkyl groups as defined above attached through a single-bonded nitrogen-nitrogen linkage. At least one of the alkyl groups is attached to the terminal nitrogen (the nitrogen not bound to the core structure). When the term mono- or di-alkylhydrazinyl is used only the terminal nitrogen is alkyl substituted. Examples of alkylhydrazinyl groups include 2-butyl-1-hydrazinyl, 2-butyl-2-methyl-1-hydrazinyl, and 1,2-dimethyl-2-propyl-1-hydrazinyl.

The term "alkylsulfonate" indicates an alkyl group as defined above attached through a sulfonate linkage (e.g. a group of the formula -S(=O)2O-alkyl).

The term "alkylthio" indicates an alkyl group as defined above attached through a sulfur linkage, i.e. a group of the formula alkyl-S-. Examples include ethylthio and pentylthio.

As used herein, the term "aryl" indicates aromatic groups containing only carbon in the aromatic ring or rings. Typical aryl groups contain 1 to 3 separate, fused, or pendant rings and from 6 to about 18 ring atoms, without heteroatoms as ring members. When indicated, such aryl groups may be further substituted with carbon or non-carbon atoms or groups. Such substitution may include fusion to a 5 to 7-membered saturated cyclic group that optionally contains 1 or 2 heteroatoms independently chosen from N, O, and S, to form, for example, a 3,4-methylenedioxy-phenyl group. Aryl groups include, for example, phenyl, naphthyl, including 1- naphthyl and 2-naphthyl, and bi-phenyl.

In the term "(aryl)alkyl", aryl and alkyl are as defined above, and the point of attachment is on the alkyl group. This term encompasses, but is not limited to, benzyl, phenylethyl, and piperonyl. Likewise, in the term (aryl)carbohydryl, aryl and carbohydryl are as defined above and the point of attachment is on the carbohydryl group, for example a phenylpropen-1-yl group. Similarly, in the term (aryl)alkoxy, aryl and alkoxy are as defined above and the point of attachment is through the oxygen atom of the alkoxy group; if the alkoxy is a C₀ alkoxy the aryl is attached through an oxygen bridge.

"Cycloalkyl" as used herein, indicates saturated hydrocarbon ring groups, having the specified number of carbon atoms, usually from 3 to about 8 ring carbon atoms, or from 3 to about 7 carbon atoms. Examples of cycloalkyl groups include cyclopropyl, cyclobutyl, cyclopentyl, or cyclohexyl as well as bridged or caged saturated ring groups such as norborane or adamantane.

"Cycloalkenyl" as used herein, indicates an unsaturated, but not aromatic, hydrocarbon ring having at least one carbon-carbon double bond. Cycloalkenyl groups contain from 4 to about 8 carbon atoms, usually from 4 to about 7 carbon atoms. Examples include cyclohexenyl and cyclobutenyl.

In the terms "(cycloalkyl)alkyl," "(cycloalkyl)carbohydryl," and "(cycloalkyl)alkoxy" the terms cycloalkyl, alkyl, carbohydryl, and alkoxy are as defined above, and the point of attachment is on the alkyl, carbohydryl, or alkoxy group respectively. These terms include examples such as cyclopropylmethyl, cyclohexylmethyl, cyclohexylpropenyl, and cyclopentylethyoxy.

In the terms "(cycloalkenyl)alkyl" "(cycloalkenyl)carbohydryl" the terms cycloakenyl, alkyl, and carbohydryl are as defined above, and the point of attachment is on the alkyl, or carbohydryl group respectively. These terms include examples such as cyclobutenylmethyl, cyclohexenylmethyl, and cyclohexylpropenyl.

"Haloalkyl" indicates both branched and straight-chain saturated aliphatic hydrocarbon groups having the specified number of carbon atoms, substituted with 1 or more halogen atoms, generally up to the maximum allowable number of halogen atoms. Examples of haloalkyl include, but are not limited to, trifluoromethyl, difluoromethyl, 2-fluoroethyl, and penta-fluoroethyl.

"Haloalkoxy" indicates a haloalkyl group as defined above attached through an oxygen bridge.

"Halo" or "halogen" as used herein refers to fluoro, chloro, bromo, or iodo.

As used herein, "heteroaryl" indicates a stable 5- to 7-membered monocyclic or 7-to 10- membered bicyclic heterocyclic ring which contains at least 1 aromatic ring that contains from 1 to 4, or preferably from 1 to 3, heteroatoms chosen from N, O, and S, with remaining ring atoms being carbon. When the total number of S and O atoms in the heteroaryl group exceeds 1, these heteroatoms are not adjacent to one another. It is preferred that the total number of S and O atoms in the heteroaryl group is not more than 2. It is particularly preferred that the total number of S and O atoms in the heteroaryl group is not more than 1. A nitrogen atom in a heteroaryl group may optionally be quaternized. When indicated, such heteroaryl groups may be further substituted with carbon or non-carbon atoms or groups. Such substitution may include fusion to a 5 to 7-membered saturated cyclic group that optionally contains 1, 2, or 3 heteroatoms independently chosen from N, O, and S, to form, for example, a [1,3]dioxolo[4,5-c]pyridyl group. Examples of heteroaryl groups include, but are not limited to, pyridyl, indolyl, pyrimidinyl, pyridizinyl, pyrazinyl, imidazolyl, oxazolyl, furanyl, thiophenyl, thiazolyl, triazolyl, tetrazolyl, isoxazolyl, quinolinyl, pyrrolyl, pyrazolyl, benz[b]thiophenyl, isoquinolinyl, quinazolinyl, quinoxalinyl, thienyl, isoindolyl, and 5,6,7,8-tetrahydroisoquinoline.

In the terms "(heteroarylalkyl" and "heteroaryl(carbohydryl)," heteroaryl, alkyl, and carbohydryl are as defined above, and the point of attachment is on the alkyl or carbohydryl group respectively. These terms include such examples as pyridylmethyl, thiophenylmethyl, and pyrrolyl(1-ethyl).

The term "heterocycloalkyl" indicates a saturated cyclic group containing from 1 to about 3 heteroatoms chosen from N, O, and S, with remaining ring atoms being carbon. Heterocycloalkyl groups have from 3 to about 8 ring atoms, and more typically have from 5 to 7 ring atoms. A C₂-C₇heterocycloalkyl group contains from 2 to about 7 carbon ring atoms and at least one ring atom chosen from N, O, and S. Examples of heterocycloalkyl groups include morpholinyl, piperazinyl, piperidinyl, and pyrrolidinyl groups. A nitrogen in a heterocycloalkyl group may optionally be quaternized.

The term "heterocyclic group" indicates a 5-6 membered saturated, partially unsaturated, or aromatic ring containing from 1 to about 4 heteroatoms chosen from N, O, and S, with remaining ring atoms being carbon or a 7-10 membered bicyclic saturated, partially unsaturated, or aromatic heterocylic ring system containing at least 1 heteroatom in the two ring system chosen from N, O, and S and containing up to about 4 heteroatoms independently chosen from N, O, and S in each ring of the two ring system. Unless otherwise indicated, the heterocyclic ring may be attached to its pendant group at any heteroatom or carbon atom that results in a stable structure. When indicated the heterocyclic rings described herein may be substituted on carbon or on a nitrogen atom if the resulting compound is stable. A nitrogen atom in the heterocycle may optionally be quaternized. It is preferred that the total number of heteroatoms in a heterocyclic groups is not more than 4 and that the total number of S and O atoms in a heterocyclic group is not more than 2, more preferably not more than 1. Examples of heterocyclic groups include, pyridyl, indolyl, pyrimidinyl, pyridizinyl, pyrazinyl, imidazolyl, oxazolyl, furanyl, thiophenyl, thiazolyl, triazolyl, tetrazolyl, isoxazolyl, quinolinyl, pyrrolyl, pyrazolyl, benz[b]thiophenyl, isoquinolinyl, quinazolinyl, quinoxalinyl, thienyl, isoindolyl, dihydroisoindolyl, 5,6,7,8-tetrahydroisoquinoline, pyridinyl, pyrimidinyl, furanyl, thienyl, pyrrolyl, pyrazolyl, pyrrolidinyl, morpholinyl, piperazinyl, piperidinyl, and pyrrolidinyl.

Additional examples of heterocyclic groups include, but are not limited to, phthalazinyl, oxazolyl, indolizinyl, indazolyl, benzothiazolyl, benzimidazolyl, benzofuranyl, benzoisoxolyl, dihydro-benzodioxinyl, oxadiazolyl, thiadiazolyl, triazolyl, tetrazolyl, oxazolopyridinyl, imidazopyridinyl, isothiazolyl, naphthyridinyl, cinnolinyl, carbazolyl, beta-carbolinyl, isochromanyl, chromanonyl, chromanyl, tetrahydroisoquinolinyl, isoindolinyl, isobenzotetrahydrofuranyl, isobenzotetrahydrothienyl, isobenzothienyl, benzoxazolyl, pyridopyridinyl, benzotetrahydrofuranyl, benzotetrahydrothienyl, purinyl, benzodioxolyl, triazinyl, phenoxazinyl, phenothiazinyl, 5 pteridinyl, benzothiazolyl, imidazopyridinyl, imidazothiazolyl, dihydrobenzisoxazinyl, benzisoxazinyl, benzoxazinyl, dihydrobenzisothiazinyl, benzopyranyl, benzothiopyranyl, coumarinyl, isocoumarinyl, chromanyl, tetrahydroquinolinyl, dihydroquinolinyl, dihydroquinolinonyl, dihydroisoquinolinonyl, dihydrocoumarinyl, dihydroisocoumarinyl, isoindolinonyl, benzodioxanyl, benzoxazolinonyl, pyrrolyl N-oxide, pyrimidinyl N-oxide, pyridazinyl N-oxide, pyrazinyl N-oxide, quinolinyl N-oxide, indolyl N-oxide, indolinyl N oxide, isoquinolyl N-oxide, quinazolinyl N-oxide, quinoxalinyl N-oxide, phthalazinyl N-oxide, imidazolyl N-oxide, isoxazolyl N-oxide, oxazolyl N- oxide, thiazolyl N-oxide, indolizinyl N oxide, indazolyl N-oxide, benzothiazolyl N-oxide, benzimidazolyl N-oxide, pyrrolyl N-oxide, oxadiazolyl N-oxide, thiadiazolyl N-oxide, tetrazolyl N- oxide, benzothiopyranyl S-oxide, and benzothiopyranyl S,S-dioxide.

As used herein "active agents" are compounds that have pharmaceutical utility, e.g. may be used to treat a patient suffering from a disease or condition, or may be used prophylacticly to prevent the onset of a disease or condition in a patient , or that may be used to enhance the pharmaceutical activity of other compounds.

"Pharmaceutical compositions" are compositions comprising at least one active agent, such as a compound or salt of Formula I or Formula II, and at least one other substance, such as a carrier, excipient, or diluent. Pharmaceutical compositions meet the U.S. FDA's GMP (Good Manufacturing Practice) standards for human or non-human drugs.

"Salts" of the compounds of the present invention include inorganic and organic acid and base addition salts. The salts of the present compounds can be synthesized from a parent compound that contains a basic or acidic moiety by conventional chemical methods. Generally, such salts can be prepared by reacting free acid forms of these compounds with a stoichiometric amount of the appropriate base (such as Na, Ca, Mg, or K hydroxide, carbonate, bicarbonate, or the like), or by reacting free base forms of these compounds with a stoichiometric amount of the appropriate acid. Such reactions are typically carried out in water or in an organic solvent, or in a mixture of the two. Generally, non-aqueous media like ether, ethyl acetate, ethanol, isopropanol, or acetonitrile are preferred, where practicable. Salts of the present compounds further include solvates of the compounds and of the compound salts.

"Pharmaceutically acceptable salts" includes derivatives of the disclosed compounds wherein the parent compound is modified by making non-toxic acid or base salts thereof, and further refers to pharmaceutically acceptable solvates of such compounds and such salts. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts and the quaternary ammonium salts of the parent compound formed, for example, from non-toxic inorganic or organic acids. For example, conventional non-toxic acid salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, HOOC-(CH₂)ₙ-COOH where n is 0-4, and the like. Lists of additional suitable salts may be found, e.g., in Remington's Pharmaceutical Sciences, 17th ed., Mack Publishing Company, Easton, Pa., p. 1418 (1985).

The term "therapeutically effective amount" of a compound of this invention means an amount effective, when administered to a human or non-human patient, to provide a therapeutic benefit such as an amelioration of symptoms, e.g., an amount effective to decrease the symptoms of a microbial infection, and/ or an amount sufficient to reduce the symptoms of a bacterial, fungal, or protozoal infection. In certain circumstances a patient suffering from a microbial infection may not present symptoms of being infected. Thus a therapeutically effective amount of a compound is also an amount sufficient to provide a positive effect on any indicia of disease, e.g. an amount sufficient to prevent a significant increase or significantly reduce the detectable level of virus or viral antibodies in the patient's blood, serum, other bodily fluids, or tissues. The invention also includes using compounds of Formula I and Formula II in prophylactic therapies. In the context of prophylactic or preventative treatment a "therapeutically effective amount" is an amount sufficient to significantly decrease the treated animal's risk of contracting a microorganism infection. A significant reduction is any detectable negative change that is statistically significant in a standard parametric test of statistical significance such as Student's T-test, where p < 0.05.

### ANTIMICROBIAL COMPOUNDS

For the purposes of this document, the following numbering system will apply to the core 9H-isothiazolo[5,4-b]quinoline-3,4-dione (when A₁ = sulfur) structure or core 9H-isoxazolo[5,4-b]quinoline-3,4-dione (when A₁= oxygen) structure. The numbers 1 through 9 refer specifically to positions within the tricyclic ring system whereas the letters A, B and C refer to the specific six (rings A and B) or five (ring C) member rings as shown below.

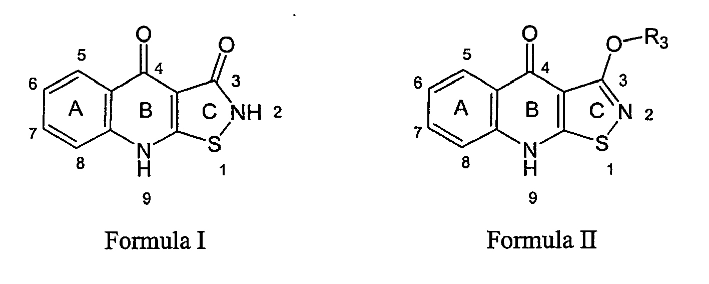

In addition to the compounds of Formula I and Formula **II,** described above the invention also includes compounds of Formula I and Formula II in which the variables (e.g. A₁, R₂, R₃, R₄, etc. ) carry definitions other than those set forth above. Embodiments in which one or more of the following conditions is met are included in the invention:

### The A₁ variable

(1) A₁ is S; e.g. compounds and salts of Formula III and Formula IV are included herein.

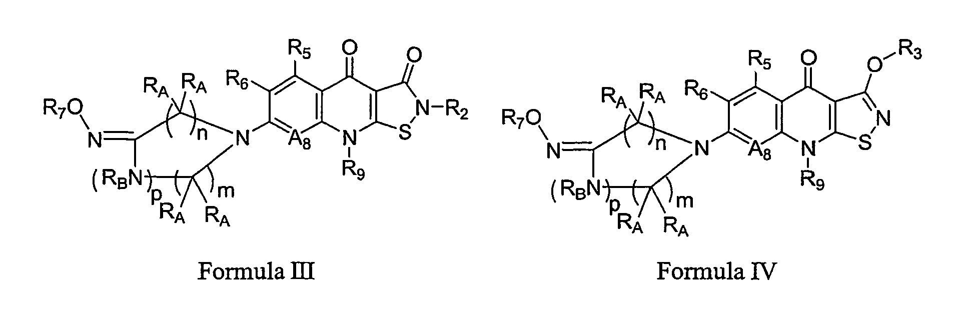

(2) A₁ is SO; e.g. compounds and salts of Formula V and VI are included herein:

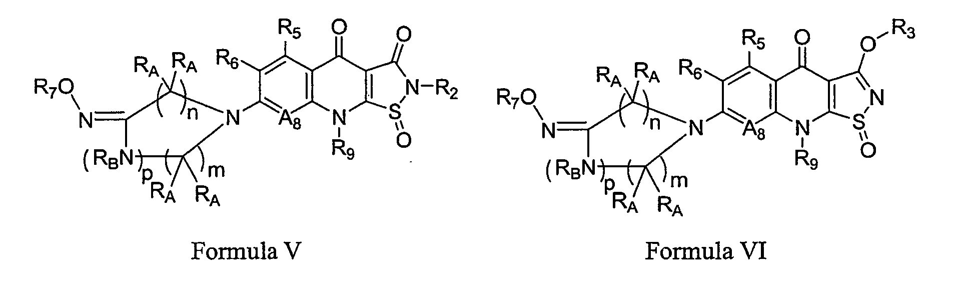

(3) A₁ is SO₂ e.g. compounds and salts of Formula VII and VIII are included herein.

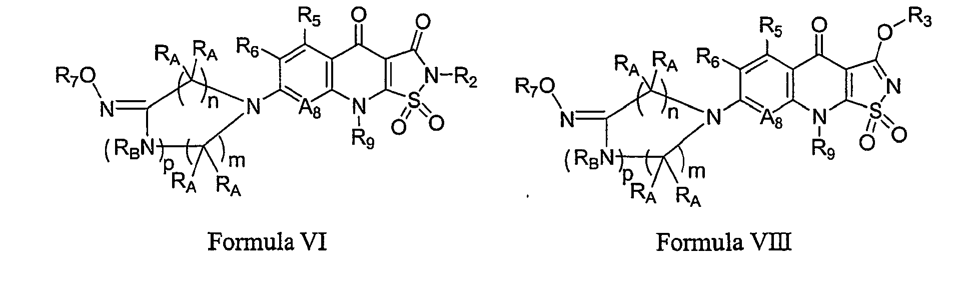

(4) A₁ is O; e.g. compounds and salts of Formula IX and X are included herein.

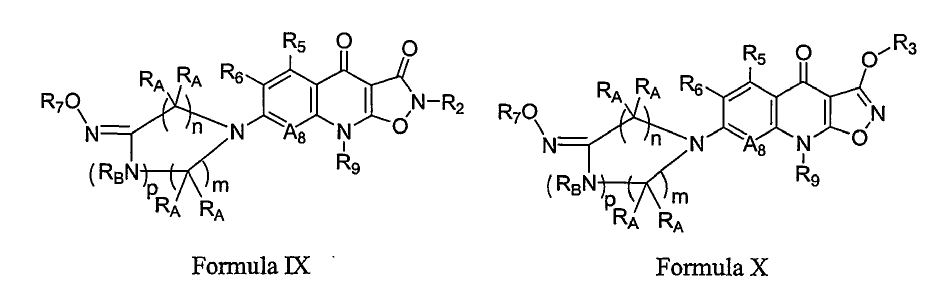

### The R₂ variable (compounds and salts of Formula I):

(1) R₂ is hydrogen, or R₂ is C₁-C₆alkyl or (C₃-C₇cycloalkyl)C₀-C₄alkyl, each of which is substituted with at least one substituent chosen from hydroxy, amino, - COOH, -(C=O)NR₁₀OR₁₁, and -CONH₂; and is substituted with 0 to 3 substituents independently chosen from halogen, hydroxy, amino, cyano, nitro, -COOH, -CONH₂, C₁-C₄alkyl, C₁-C₄alkoxy, C₁-C₂haloalkyl, C₁-C₂haloalkoxy, and mono- and di-C₁-C₄alkylamino, and C₂-C₄alkanoyl.

(2) R₂ is hydrogen.

### The R₃ variable (compounds and salts of Formula II):

(1) R₃ is C₁-C₄alkyl or C₂-C₄alkanoyl.

### The R₅ variable

(1°) R₅ is hydrogen, amino, C₁-C₂alkyl, C₁-C₂alkoxy, mono- or di-C₁-C₄alkylamino, or mono- or di-C₁-C₄ alkylhydrazinyl.

(2) R₅ is hydrogen, amino, mono- or di-C₁-C₂alkylamino, or mono- or di-C₁-C₂ alkylhydrazinyl.

(3) R₅ is hydrogen.

### The R₆ variable

(1) R₆ is hydrogen, halogen, or amino.

(2) R₆ is fluoro.

### The R₇ variable

(1) R₇ is hydrogen or R₇ is C₁-C₈alkyl, C₂-C₆alkanoyl, (C₃-C₇cycloalkyl)C₀-C₄carbohydryl, (aryl)C₀-C₄carbohydryl, (aryl)(C=O)-, mono- or di-(C₁-C₆alkyl)carboxamide, C₁-C₆alkylester, or (C₂-C₆heterocycloalkyl)C₀-C₄carbohydryl, each of which is substituted with 0 to 5 substituents independently chosen from halogen, hydroxy, amino, cyano, nitro, C₁-C₄alkyl, C₁-C₄alkoxy, C₁-C₂haloakyl, C₁-C₂haloalkoxy, mono- and di-C₁-C₄alkylamino, C₂-C₄alkanoyl, C₁-C₄alkylthio, -O(C=O)R₁₀, -(C=O)NR₁₀R₁₁, -O(C=O)NR₁₀R₁₁, -(C=O)OR₁₀, -(C=O)NR₁₀OR₁₁, -NR₁₀(C=O)R₁₁, -NR₁₀(C=O)OR₁₁, -NR₁₀(C=O)NR₁₁R₁₂, -NR₁₀(C=S)NR₁₁R₁₂, -NR₁₀NR₁₁R₁₂, -SO₃R₁₀, -(S=O)OR₁₀, -SO₂R₁₃, -SO₂NR₁₀R₁₁, or -NR₁₀SO₂R₁₃.

(2) R₇ is hydrogen or R₇ is C₁-C₈alkyl, (C₃-C₇cycloalkyl)C₀-C₄carbohydryl, (aryl)C₀-C₄carbohydryl, or (C₂-C₆heterocycloalkyl)C₀-C₄carbohydryl, each of which is substituted with 0 to 5 substituents independently chosen from halogen, hydroxy, amino, cyano, C₁-C₄alkyl, C₁-C₄alkoxy, C₁-C₂haloalkyl, C₁-C₂haloalkoxy, mono- and di-C₁-C₄alkylamino, C₂-C₄alkanoyl, -(C=O)NR₁₀R₁₁, and -NR₁₀(C=O)R₁₁.

(3) R₇ is hydrogen or R₇ is C₁-C₈akyl or (aryl)C₀-C₄alkyl, each of which is substituted with 0 to 5 substituents independently chosen from halogen, hydroxy, amino, cyano, C₁-C₄alkyl, C₁-C₄alkoxy, C₁-C₂haloalkyl, C₁-C₂haloalkoxy, mono- and di-C₁-C₄alkylamino, C₂-C₄alkanoyl, -(C=O)NR₁₀R₁₁, and -NR₁₀(C=O)R₁₁.

(4) R₇ is hydrogen, C₁-C₂alkyl, or benzyl.

### The variables n, m, and p

(1) n is 1; m is 2, p is 1 and R_{B} is hydrogen; e.g. when A₁ is S, the compounds and salts of Formula XI and Formula XII are included herein.

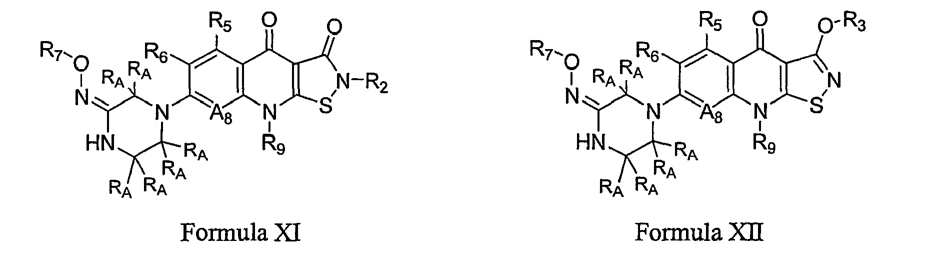

(2) n is 1; m is 1, p is 1 and R_{B} is hydrogen.

(3) p is 0, e.g. when A₁ is S, the compounds and salts of Formula XIII and XIV are included herein.

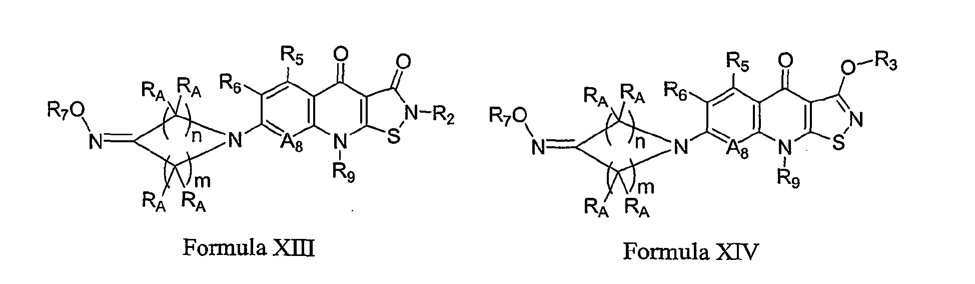

(4) p is 0, n is 1, and m is 2, e.g. when A₁ is S, the compounds and salts for Formula XV and XVI are included herein.

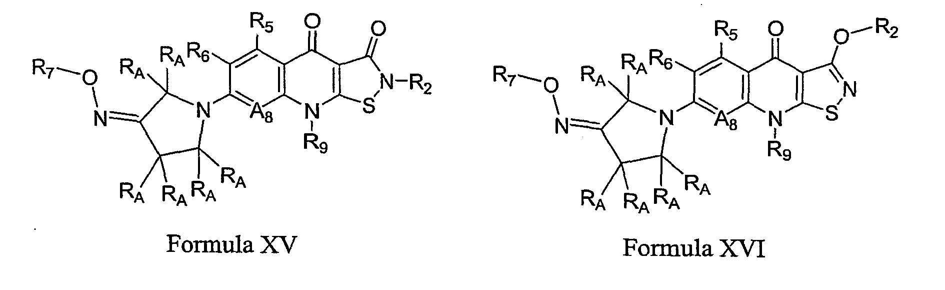

(5) p is 0, n is 2, and m is 2, e.g. when A₁ is S, the compounds and salts for Formula XVII and XVIII are included herein.

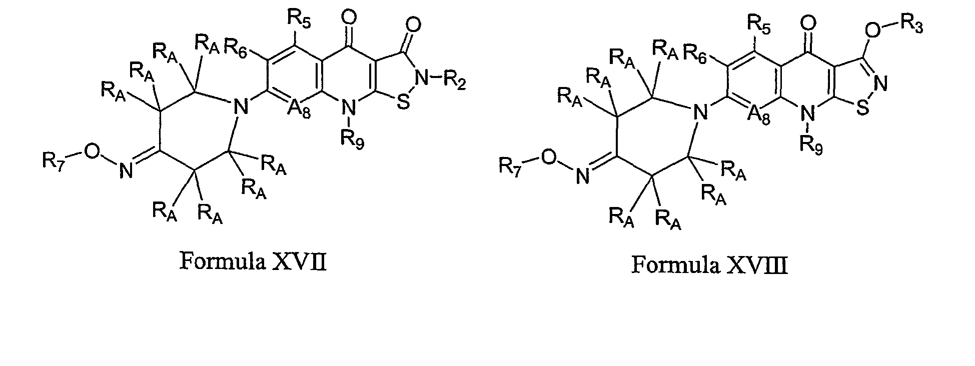

### The variable R_{A}

(1) R_{A} is independently (i), (ii), or (iii).
Where (i) is hydrogen, hydroxy, amino, or cyano, (ii) is C₁-C₆alkyl, C₂-C₆alkenyl, C₂-C₆alkynyl, (C₁-C₆alkoxy)C₀-C₄alkyl, mono- or di-C₁-C₄alkylamino, C₁-C₂haloalkoxy, (C₃-C₇cycloallzyl)C₀-C₄carbohydryl, (C₄-C₇crycloalkenyl)C₀-C₄carbohydryl, (aryl)Co-C₆carbohydryl, (aryl)C₁-C₄alkoxy, (C₂-C₆heterocycloalkyl)C₀-C₄carbohydryl, (heteroaryl)C₀-C₆carbohydryl, C₁-C₆alkylthio, -(C₀-C₄akyl)(C=O)NR₁₀R₁₁, -(C₀-C₄alkyl)NR₁₀(C=O)R₁₁, =N-OH, or =N-O(C₀-C₄alkyl); and (iii) is -OR_{D} or -(C=O)R_{D}, where R_{D} is C₁-C₄alkyl, (C₃-C₇cycloalkyl)C₀-C₂alkyl, (C₂-C₆heterocycloalkyl)C₀-C₂alkyl, (aryl)C₀-C₂alkyl, or (heteroaryl)C₀-C₂alkyl.

Each of (ii) and (iii) is substituted with 0 to 3 substituents independently chosen from halogen, hydroxy, amino, cyano, nitro, -COOH, -(C=O)OCH₃, -CONH₂, C₁-C₄alkyl, C₂-C₄alkenyl, C₂-C₄alkynyl, C₁-C₄alkoxy, (C₃-C₇cycloalkyl)C₀-C₄carbohydryl, (C₃-C₇cycloalkyl)C₀-C₄alkoxy, mono- and di-C₁-C₄alkylamino, C₁-C₂haloalkyl, C₁-C₂haloalkoxy, and C₂-C₄alkanoyl.

Or, any two R_{A} bound to the same carbon atom may be joined to form a C₃-C₇cycloalkyl or a 3- to 7- membered heterocycloalkyl group having 1 or 2 heteroatoms independently chosen from N, O, and S; each of which is substituted with 0 to 2 substituents independently chosen from C₁-C₂alkyl, and C₁-C₂alkoxy.

Or, any two R_{A} bound to different carbon atoms may be joined to form a C₃-C₇cycloalkyl or a 3- to 7-membered heterocycloalkyl group having 1 or 2 heteroatoms independently chosen from N, O, and S; each of which is substituted with 0 to 2 substituents independently chosen from C₁-C₂alkyl and C₁-C₂alkoxy.

(2) Each R_{A} is independently (i) or (ii).
Where (i) is hydrogen or amino, (ii) is C₁-C₆alkyl, (C₁-C₆alkoxy)C₀-C₄alkyl, mono- or di-C₁-C₄alkylamino, (C₃-C₇cycloalkyl)C₀-C₄carbohydryl, (aryl)C₀-C₆carbohydryl, (C₂-C₆heterocycloalkyl)C₀-C₄carbohydryl, (heteroaryl)C₀-C₆carbohydryl, -(C₀-C₄alkyl)(C=O)NR₁₀R₁₁, -(C₀-C₄alkyl)NR₁₀(C=O)R₁₁, =N-OH, or N-O(C₀-C₄alkyl).

Where each of (ii) is substituted with 0 to 3 substituents independently chosen from halogen, hydroxy, amino, cyano, nitro, -COOH, -(C=O)OCH₃, -CONH₂, C₁-C₄alkyl, C₂-C₄alkenyl, C₂-C₄alkynyl, C₁-C₄alkoxy, (C₃-C₇cycloalkyl)C₀-C₄carbohydryl, (C₃-C₇cycloakyl)C₀-C₄alkoxy, mono- and di-C₁-C₄alkylamino, C₁-C₂haloalkyl, C₁-C₂haloalkoxy, and C₂-C₄alkanoyl.

Or, any two R_{A} bound to the same carbon atom may be joined to form a C₃-C₇cycloalkyl or a 3- to 7-membered heterocycloalkyl group having 1 or 2 heteroatoms independently chosen from N, O, and S; each of which is substituted with 0 to 2 substituents independently chosen from C₁-C₂alkyl, and C₁-C₂alkoxy.

Or, any two R_{A} bound to different carbon atoms may be joined to form a C₃-C₇cycloalkyl or heterocycloalkyl group having 1 or 2 heteroatoms independently chosen from N, O, and S; each of which is substituted with 0 to 2 substituents independently chosen from C₁-C₂alkyl, and C₁-C₂akoxy.

(3) Each R_{A} is independently (i) or (ii). Where (i) is hydrogen or amino; and (ii) is C₁-C₆alkyl, mono- or di-C₁-C₄alkylamino, (C₃-C₇cycloalkyl)C₀-C₄alkyl, (C₂-C₆heterocycloalkyl)C₀-C₄alkyl, -(C₀-C₄alkyl)(C=O)NR₁₀R₁₁, -(C₀-C₄alkyl)NR₁₀(C=O)R₁₁, =N-OH, or N-O(C₀-C₄alkyl).

Each of (ii) is substituted with 0 to 3 substituents independently chosen from hydroxy, amino, cyano, and -CONH₂.

Any two R_{A} bound to the same carbon atom may be joined to form a C₃-C₇cycloalkyl or heterocycloalkyl group having 1 or 2 heteroatoms independently chosen from N, O, and S; each of which is substituted with 0 to 2 substituents independently chosen from C₁-C₂alkyl and C₁-C₂alkoxy.

(4) Each R_{A} is independently (i) or (ii); Where (i) is hydrogen or amino, and (ii) is C₁-C₆alkyl, (C₃-C₇cycloalkyl)C₀-C₂alkyl, or mono- or di-C₁-C₄alkylamino.

Where, each of (ii) is substituted with 0 to 3 amino.

Any two R_{A} bound to the same carbon atom may be joined to form a C₃-C₇cycloalkyl or a 3- to 7- membered heterocycloalkyl group having 1 or 2 heteroatoms independently chosen from N, O, and S; each of which is substituted with 0 to 2 substituents independently chosen from C₁-C₂alkyl and C₁-C₂alkoxy.
Or, any two R_{A} bound to different carbon atoms may be joined to form a C₃-C₇cycloalkyl or a membered heterocycloalkyl group having 1 or 2 heteroatoms independently chosen from N, O, and S; each of which is substituted with 0 to 2 substituents independently chosen from C₁-C₂alkyl and C₁-C₂alkoxy.

(5) R_{A} is independently hydrogen, C₁-C₄alkyl, or C₁-C₄alkyl substituted with one amino substituent.

(6) R_{A} is independently hydrogen, C₁-C₄alkyl, or C₁-C₄alkyl, and one or two R_{A} is C₁-C₄alkyl substituted with one amino substituent and the remaining R_{A} are hydrogen.

(7) Two R_{A} bound to the same carbon atom are joined to form a C₃-C₇cycloalkyl or a 3- to 7- membered heterocycloalkyl group having 1 or 2 heteroatoms independently chosen from N, O, and S; each of which is substituted with 0 to 2 substituents independently chosen from C₁-C₂alkyl and C₁-C₂alkoxy; and the remaining R_{A} are independently chosen from hydrogen, C₁-C₂alkyl and C₁-C₂alkoxy.

(8) Two R_{A} bound to the same carbon atom are joined to form a C₃-C₄cycloalkyl or a 3- to 4- membered heterocycloalkyl group having 1 N atom; each of which is substituted with 0 to 2 substituents independently chosen from C₁-C₂alkyl and C₁-C₂alkoxy; and the remaining R_{A} independently chosen from hydrogen, C₁-C₂alkyl and C₁-C₂alkoxy.

### The A₈ variable

(1) A₈ is nitrogen.

(2) A₈ is CR₈.

(3) A₈ is CR₈, and R₈ is hydrogen, halogen, C₁-C₂alkyl, C₁-C₂alkoxy, C₁-C₂haloalkyl, or C₁-C₂haloalkoxy.

(4) A₈ is CR₈ and R₈ is hydrogen or methoxy.

(5) A₈ is CR₈ and R₈ is hydrogen.

### The R₉ Variable

(1) R₉ is C₁-C₄alkyl, cyclopropyl, or phenyl, each of which is substituted with 0 to 3 substituents independently chosen from halogen, hydroxy, amino, C₁-C₂alkyl, C₁-C₂alkoxy, mono- and di-C₁-C₂alkylamino, C₁-C₂haloalkyl, and C₁-C₂haloalkoxy.

(2) R₉ is C₁-C₄alkyl or cyclopropyl, or R₉ is phenyl substituted with 2 substituents chosen from halogen, hydroxy, amino, C₁-C₂alkyl, C₁-C₂alkoxy, mono- and di-C₁-C₂alkylamino, C₁-C₂haloalkyl, and C₁-C₂haloalkoxy.

(3) R₉ is ethyl, t-butyl, cyclopropyl, or 2,4-difluorophenyl.

(4) R₉ is cyclopropyl.

Included herein are all embodiments described by any combination of the variable definitions given above that result in a stable compounds. For example, the invention includes compounds of Formula III (also shown above) 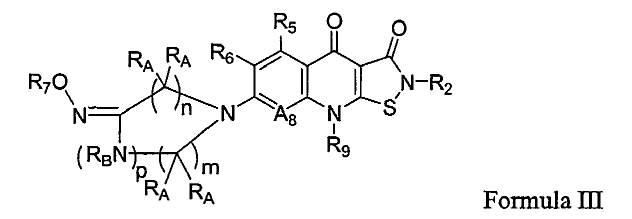 wherein condition (2) is met for the R₂ variable, the R₅ variable, R₆ variable, and the R₇ variable condition (2) is met, for the A₈ variable and R₉ variable condition (3) is met, for the p, n, and m variables condition (4) is met and for the R_{A} variable condition (5) is met. Thus, the invention includes compounds of Formula III in which:

R₂ is hydrogen (condition (2) for the R₂ variable)

R₅ is hydrogen, amino, mono- or di-C₁-C₂alkylamino, or mono- or di-C1-C2 alkylhydrazinyl (condition (2) for the R₅ variable);

R₆ is fluoro (condition (2) for the R₆ variable);

R₇ is hydrogen or R₇ is C₁-C₈alkyl, (C₃-C₇cycloalkyl)C₀-C₄carbohydryl, (aryl)C₀-C₄carbohydryl, or (C₂-C₆heterocycloalkyl)C₀-C₄carbohydryl, each of which is substituted with 0 to 5 substituents independently chosen from halogen, hydroxy, amino, cyano, C₁-C₄alkyl, C₁-C₄alkoxy, C₁-C₂haloalkyl, C₁-C₂haloakoxy, mono- and di-C₁-C₄alkylamino, C₂-C₄alkanoyl, -(C=O)NR₁₀R₁₁, and -NR₁₀(C=O)R₁₁ (condition (2) for the R₇ variable);

A₈ is CR₈, and R₈ is hydrogen, halogen, C₁-C₂alkyl, C₁-C₂alkoxy, C₁-C₂haloalkyl, or C₁-C₂haloalkoxy (condition (3) for the A₈ varaible);

R₉ is ethyl, t-butyl, cyclopropyl, or 2,4-difluorophenyl (condition (3) for the R₉ variable);

p is 0, n is 1, and m is 2 (condition (4) for these variables); and

R_{A} is independently hydrogen, C₁-C₄alkyl, or C₁-C₄alkyl substituted with one amino substituent (condition (5) for the R_{A} variable).

Also included herein are compounds of Formula I and Formula III in which:

R₂ is hydrogen or C₁-C₆alkyl substituted with one substituent chosen from hydroxy, amino, -COOH, -(C=O)NR₁₀OR₁₁, and -CONH₂;

R₅ is hydrogen;

R₆ is halogen or amino;

R₇ is hydrogen, C₁-C₂alkyl, or benzyl;

A₈ is nitrogen or CR₈, and when A₈ is CR₈, R₈ is hydrogen or methoxy

R₉ is ethyl, t-butyl, cyclopropyl, or 2,4-difluorophenyl; and

Each R_{A} is independently hydrogen or C₁-C₄alkyl optionally substituted with one amino substituent; or any two R_{A} bound to the same carbon atom may be joined to form a C₃-C₇cycloalkyl or a 3- to 7-membered heterocycloalkyl group having 1 or 2 heteroatoms independently chosen from N, O, and S; each of which is substituted with 0 to 2 substituents independently chosen from C₁-C₂alkyl, and C₁-C₂alkoxy; or any two R_{A} bound to different carbon atoms may be joined to form a C₃-C₇cycloalkyl or heterocycloalkyl group having 1 or 2 heteroatoms independently chosen from N, O, and S; each of which is substituted with 0 to 2 substituents independently chosen from C₁-C₂alkyl, and C₁-C₂alkoxy.

Certain compounds of Formula I and Formula II exhibit possess potent antibacterial, antifungal, and/or antiprotozoal activity. Particular compounds of the invention exhibit Minimum Inhibitory Concentrations (MIC) of 64 µg/ ml or less against *Staphyloccocus aureus* and/ or *Eschericia coli* in a standard assay for determining the MIC of a compound against these bacteria, such as the assay provided in Example 5 below. Preferred compounds of the Formula I and II exhibit MIC values of 10 µg/ ml or less against *Staphyloccocus aureus* and/ or *Eschericia coli.* More preferred compound of the Formula I and II exhibit MIC values of 4 µg/ ml or less, or even more preferably 1 µg/ ml or less, against *Staphyloccocus aureus* and/ or *Eschericia coli.*

Certain compounds of Formula I and Formula II are selective antimicrobial agents; having the ability to kill or inhibit the growth or reproduction of microbial organisms, while having little or no effect on the cells of fish, amphibians, reptiles, birds, or mammals. The selectivity of compounds of Formula I and Formula II may be assessed by determining the CC₅₀ (the concentration at which 50% of the cells are killed) for cultured cells of a higher animal, such as a fish, reptiles, amphibian, bird, or mammal. Certain compounds of the invention exhibit a CC₅₀ of greater than 100 micromolar for mammalian cells. Certain compounds of the invention exhibit a CC₅₀ of greater than 100 micromolar for cultured human hepatocytes, and also exhibit MIC values of 64 µg/ ml or less, preferably 10 µg/ ml or less, or more preferably 4 µg/ ml or less, or still more preferably 1 µg/ ml or less against *Staphyloccocus aureus* and/ or *Eschericia coli.*

Without wishing to be bound to any particular theory it is believed that the antimicrobial properties of compounds of Formula I and Formula II are due to the ability of these compounds to inhibit the activity of microbial DNA gyrases while having little or no effect on the analogous enzyme, Topoisomerase II, present in higher organisms. Certain preferred compound of the invention are 100-fold or more selective for bacterial DNA gyrases than for mammalian, particularly human, Topoisomerase II.

### SYNTHETIC INTERMEDIATES

The invention includes novel intermediates useful for the synthesis of antimicrobial compounds of Formula I and Formula II. The intermediates include compounds of the Formula (a) and Formula (b):

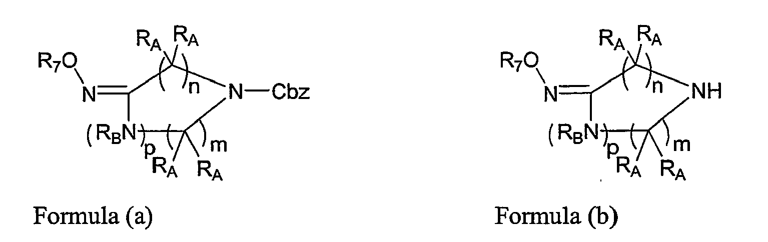

Compounds of Formula a are deprotected to form compounds of Formula b. Compounds of Formula (b) are coupled to compound of Formula (c) or Formula (d)

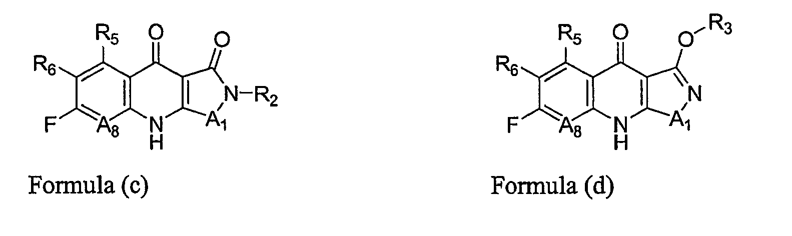

The variables R₇, R_{A}, and R_{B} carry the definitions set forth for these variables in Formula I and Formula II, and in the embodiments described in the immediately preceding "Antimicrobial compounds" section. When R_{A} or R₇ is an aminoalkyl group, the amino alkyl group may be protected, and then deprotected after the coupling reaction between the intermediate of Formula (b) and the compound of Formula (c) or Formula (d) is complete.

Coupling may be effected by dissolving a compound of Formula (c) or Formula (d) in dimethylformamide (DMF), adding an intermediate of Formula (b), and heating the reaction mixture.

Alternately the coupling may be effected by refluxing a mixture of boric acid, acetic anhydride, and acetic acid in a solvent, such as toluene. A compound of Formula (c) or (d) is added and the reaction mixture is refluxed for several more hours. The volatiles are then removed *in vacuo.* An intermediate of formula (b), triethyl amine (1 ml), and acetonitrile (10 ml) are then added to the residue, and the reaction mixture is refluxed for several hours.

If the intermediate substituents, such as R₇ and R_{A}, have been protected these groups are then deprotected.

### PHARMACEUTICAL PREPARATIONS

Compounds and salts of Formula I and Formula II can be administered as the neat chemical, but are preferably administered as a pharmaceutical composition or formulation. Accordingly, the invention provides pharmaceutical formulations comprising a compound or pharmaceutically acceptable salt of Formula I or Formula II, together with one or more pharmaceutically acceptable carrier, excipients, adjuvant, diluent, or other ingredient.

Compounds of general Formula I and Formula II may be administered orally, topically, parenterally, by inhalation or spray, sublingually, transdermally, via buccal administration, rectally, as an ophthalmic solution, or by other means, in dosage unit formulations containing conventional non-toxic pharmaceutically acceptable carriers, excipients, adjuvants, and vehicles.

In addition to the subject compound, the compositions of the invention may contain a pharmaceutically acceptable carrier, one or more compatible solid or liquid filler diluents or encapsulating substances, which are suitable for administration to an animal. Carriers must be of sufficiently high purity and sufficiently low toxicity to render them suitable for administration to the animal being treated. The carrier can be inert or it can possess pharmaceutical benefits of its own. The amount of carrier employed in conjunction with the compound is sufficient to provide a practical quantity of material for administration per unit dose of the compound.

Exemplary pharmaceutically acceptable carriers or components thereof are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, and corn oil; polyols such as propylene glycol, glycerine, sorbitol, mannitol, and polyethylene glycol; alginic acid; emulsifiers, such as the TWEENS; wetting agents, such sodium lauryl sulfate; coloring agents; flavoring agents; tableting agents, stabilizers; antioxidants; preservatives; pyrogen-free water; isotonic saline; and phosphate buffer solutions.

In particular, pharmaceutically acceptable carriers for systemic administration include sugars, starches, cellulose and its derivatives, malt, gelatin, talc, calcium sulfate, vegetable oils, synthetic oils, polyols, alginic acid, phosphate buffer solutions, emulsifiers, isotonic saline, and pyrogen-free water. Preferred carriers for parenteral administration include propylene glycol, ethyl oleate, pyrrolidone, ethanol, and sesame oil.

Optional active agents may be included in a pharmaceutical composition, which do not substantially interfere with the activity of the compound of the present invention.

Effective concentrations of one or more of the compounds of the invention including pharmaceutically acceptable salts, esters or other derivatives thereof are mixed with a suitable pharmaceutical carrier, excipients, adjuvant, or vehicle. In instances in which the compounds exhibit insufficient solubility, methods for solubilizing compounds may be used. Such methods are known to those of skill in this art, and include, but are not limited to, using cosolvents, such as dimethylsulfoxide (DMSO), using surfactants, such as Tween, or dissolution in aqueous sodium bicarbonate. Derivatives of the compounds, such as salts of the compounds or prodrugs of the compounds may also be used in formulating effective pharmaceutical compositions.

Upon mixing or addition of the compound(s) of Formula I and/or Formula II, the resulting mixture may be a solution, suspension, emulsion or the like. The form of the resulting mixture depends upon a number of factors, including the intended mode of administration and the solubility of the compound in the chosen carrier or vehicle. The effective concentration sufficient for ameliorating the symptoms of the disease, disorder or condition treated and may be empirically determined.

The pharmaceutical compositions containing compounds of general Formula I and/ or Formula II may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents, such as sweetening agents, flavoring agents, coloring agents and preserving agents, in order to provide pharmaceutically elegant and palatable preparations. Oral formulations contain between 0.1 and 99% of a compound of the invention and usually at least about 5% (weight %) of a compound of the present invention. Some embodiments contain from about 25% to about 50% or from 5% to 75 % of a compound of invention.

### Liquids formulations

Compounds of the invention can be incorporated into oral liquid preparations such as aqueous or oily suspensions, solutions, emulsions, syrups, or elixirs, for example. Moreover, formulations containing these compounds can be presented as a dry product for constitution with water or other suitable vehicle before use. Such liquid preparations can contain conventional additives, such as suspending agents (e.g., sorbitol syrup, methyl cellulose, glucose/sugar, syrup, gelatin, hydroxyethyl cellulose, carboxymethyl cellulose, aluminum stearate gel, and hydrogenated edible fats), emulsifying agents (e.g., lecithin, sorbitan monsoleate, or acacia), non-aqueous vehicles, which can include edible oils (e.g., almond oil, fractionated coconut oil, silyl esters, propylene glycol and ethyl alcohol), and preservatives (e.g., methyl or propyl p-hydroxybenzoate and sorbic acid).

Orally administered compositions also include liquid solutions, emulsions, suspensions, powders, granules, elixirs, tinctures, syrups, and the like. The pharmaceutically acceptable carriers suitable for preparation of such compositions are well known in the art. Oral formulations may contain preservatives, flavoring agents, sweetening agents, such as sucrose or saccharin, taste-masking agents, and coloring agents.

Typical components of carriers for syrups, elixirs, emulsions and suspensions include ethanol, glycerol, propylene glycol, polyethylene glycol, liquid sucrose, sorbitol and water. Syrups and elixirs may be formulated with sweetening agents, for example glycerol, propylene glycol, sorbitol or sucrose. Such formulations may also contain a demulcent. *Suspensions*

For a suspension, typical suspending agents include methylcellulose, sodium carboxymethyl cellulose, Avicel RC-591, tragacanth and sodium alginate; typical wetting agents include lecithin and polysorbate 80; and typical preservatives include methyl paraben and sodium benzoate.

Aqueous suspensions contain the active material(s) in admixture with excipients suitable for the manufacture of aqueous suspensions. Such excipients are suspending agents, for example sodium carboxymethylcellulose, methylcellulose, hydropropylmethylcellulose, sodium alginate, polyvinylpyrrolidone, gum tragacanth and gum acacia; dispersing or wetting agents; may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol substitute, or condensation products of ethylene oxide with partial esters derived from fatty acids and hexitol anhydrides, for example polyethylene sorbitan substitute. The aqueous suspensions may also contain one or more preservatives, for example ethyl, or n- propyl p-hydroxybenzoate.

Oily suspensions may be formulated by suspending the active ingredients in a vegetable oil, for example peanut oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

### Emulsions

Pharmaceutical compositions of the invention may also be in the form of oil-in-water emulsions. The oily phase may be a vegetable oil, for example olive oil or peanut oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monoleate, and condensation products of the said partial esters with ethylene oxide, for example polyoxyethylene sorbitan monoleate.

### Dispersible powders

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above.

### Tablets and Capsules

Tablets typically comprise conventional pharmaceutically compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. Glidants such as silicon dioxide can be used to improve flow characteristics of the powder mixture. Coloring agents, such as the FD&C dyes, can be added for appearance. Sweeteners and flavoring agents, such as aspartame, saccharin, menthol, peppermint, and fruit flavors, are useful adjuvants for chewable tablets. Capsules (including time release and sustained release formulations) typically comprise one or more solid diluents disclosed above. The selection of carrier components often depends on secondary considerations like taste, cost, and shelf stability.

Such compositions may also be coated by conventional methods, typically with pH or time-dependent coatings, such that the subj ect compound is released in the gastrointestinal tract in the vicinity of the desired topical application, or at various times to extend the desired action. Such dosage forms typically include, but are not limited to, one or more of cellulose acetate phthalate, polyvinylacetate phthalate, hydroxypropyl methylcellulose phthalate, ethyl cellulose, Eudragit coatings, waxes and shellac.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

### Injectable and Parenteral formulations

Pharmaceutical compositions may be in the form of a sterile injectable aqueous or oleaginous suspension. This suspension may be formulated according to the known art using those suitable dispersing or wetting agents and suspending agents that have been mentioned above. The sterile injectable preparation may also be sterile injectable solution or suspension in a non-toxic parentally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid are useful in the preparation of injectables.

Compounds of Formula I and Formula II may be administered parenterally in a sterile medium. Parenteral administration includes subcutaneous injections, intravenous, intramuscular, intrathecal injection or infusion techniques. The drug, depending on the vehicle and concentration used, can either be suspended or dissolved in the vehicle. Advantageously, adjuvants such as local anesthetics, preservatives and buffering agents can be dissolved in the vehicle. In compositions for parenteral administration the carrier comprises at least about 90% by weight of the total composition.

### Suppositories

Compounds of Formula I and Formula II may also be administered in the form of suppositories for rectal administration of the drug. These compositions can be prepared by mixing the drug with a suitable non- irritating excipient that is solid at ordinary temperatures but liquid at the rectal temperature and will therefore melt in the rectum to release the drug. Such materials are cocoa butter and polyethylene glycols.

### Topical formulations

Compounds of the invention may be formulated for local or topical application, such as for topical application to the skin and mucous membranes, such as in the eye, in the form of gels, creams, and lotions and for application to the eye or for intracisternal or intraspinal application. Topical compositions of the present invention may be in any form including, for example, solutions, creams, ointments, gels, lotions, milks, cleansers, moisturizers, sprays, skin patches, and the like.

Such solutions may be formulated as 0.01% -10% isotonic solutions, pH about 5-7, with appropriate salts. Compounds of the invention may also be formulated for transdermal administration as a transdermal patch.

Topical compositions containing the active compound can be admixed with a variety of carrier materials well known in the art, such as, for example, water, alcohols, aloe vera gel, allantoin, glycerine, vitamin A and E oils, mineral oil, propylene glycol, PPG-2 myristyl propionate, and the like.

Other materials suitable for use in topical carriers include, for example, emollients, solvents, humectants, thickeners and powders. Examples of each of these types of materials, which can be used singly or as mixtures of one or more materials, are as follows: Emollients, such as stearyl alcohol, glyceryl monoricinoleate, glyceryl monostearate, propane-1,2-diol, butane-1,3-diol, mink oil, cetyl alcohol, iso-propyl isostearate, stearic acid, iso-butyl palmitate, isocetyl stearate, oleyl alcohol, isopropyl laurate, hexyl laurate, decyl oleate, octadecan-2-ol, isocetyl alcohol, cetyl palmitate, dimethylpolysiloxane, di-n-butyl sebacate, iso-propyl myristate, iso-propyl palmitate, iso-propyl stearate, butyl stearate, polyethylene glycol, triethylene glycol, lanolin, sesame oil, coconut oil, arachis oil, castor oil, acetylated lanolin alcohols, petroleum, mineral oil, butyl myristate, isostearic acid, palmitic acid, isopropyl linoleate, lauryl lactate, myristyl lactate, decyl oleate, and myristyl myristate; propellants, such as propane, butane, iso-butane, dimethyl ether, carbon dioxide, and nitrous oxide; solvents, such as ethyl alcohol, methylene chloride, iso-propanol, castor oil, ethylene glycol monoethyl ether, diethylene glycol monobutyl ether, diethylene glycol monoethyl ether, dimethyl sulphoxide, dimethyl formamide, tetrahydrofuran; humectants, such as glycerin, sorbitol, sodium 2-pyrrolidone-5-carboxylate, soluble collagen, dibutyl phthalate, and gelatin; and powders, such as chalk, talc, fullers earth, kaolin, starch, gums, colloidal silicon dioxide, sodium polyacrylate, tetra alkyl ammonium smectites, trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, and ethylene glycol monostearate.

The compounds of the invention may also be topically administered in the form of liposome delivery systems, such as small unilamellar vesicles, large unilamellar vesicles, and multilamellar vesicles. Liposomes can be formed from a variety of phospholipids, such as cholesterol, stearylamine or phosphatidylcholines.

### Other formulations

Other compositions useful for attaining systemic delivery of the subject compounds include sublingual, buccal and nasal dosage forms. Such compositions typically comprise one or more of soluble filler substances such as sucrose, sorbitol and mannitol, and binders such as acacia, microcrystalline cellulose, carboxymethyl cellulose and hydroxypropyl methylcellulose. Glidants, lubricants, sweeteners, colorants, antioxidants and flavoring agents disclosed above may also be included.

Compositions for inhalation typically can be provided in the form of a solution, suspension or emulsion that can be administered as a dry powder or in the form of an aerosol using a conventional propellant (e.g., dichlorodifluoromethane or trichlorofluoromethane).

### Additional components

The compositions of the present invention may also optionally comprise an activity enhancer. The activity enhancer can be chosen from a wide variety of molecules that function in different ways to enhance antimicrobial effects of compounds of the present invention. Particular classes of activity enhancers include skin penetration enhancers and absorbtion enhancers.
Pharmaceutical compositions of the invention may also contain additional active agents can be chosen from a wide variety of molecules, which can function in different ways to enhance the antimicrobial or therapeutic effects of a compound of the present invention. These optional other active agents, when present, are typically employed in the compositions of the invention at a level ranging from about 0.01 % to about 15%. Some embodiments contain from about 0.1% to about 10% by weight of the composition. Other embodiments contain from about 0.5% to about 5% by weight of the composition.

### Packaged Formulations

The invention includes packaged pharmaceutical formulations. Such packaged formulations include a pharmaceutical composition containing one or more compounds or salts of Formula I in a container and instructions for using the composition to treat an animal (typically a human patient) suffering from a microorganism infection) or prevent a microorganism infection in an animal.

In all of the foregoing the compounds of the invention can be administered alone or as mixtures, and the compositions may further include additional drugs or excipients as appropriate for the indication.

### METHODS OF TREATMENT

The invention includes methods of preventing and treating microorganism infections, particularly bacterial and protozoal infections, by administering an effective amount of one or more compounds of Formula I and of Formula II to an animal at risk for a microorganism infection or suffering from a microorganism infection. The animal may be a fish, amphibian, reptile or bird, but is preferably a mammal. Methods of treating and preventing microorganism infections in livestock animals, companion animals, and human patients are particularly preferred.

The compounds disclosed herein are useful for preventing and treating bacterial infections in animals. Furthermore compounds of the invention may be used to treat a variety of conditions not attributed to bacterial infections. These include diseases and disorders caused fungal infections, mycoplasma infections, protozoal infections, or other conditions involving infectious organisms.

In some circumstances an effective amount of a compound of Formula I or Formula II may be an amount sufficient to reduce the symptoms of the microorganism infection. Alternatively an effective amount of a Compound of Formula I may be an amount sufficient to significantly reduce the amount of microorganism or antibodies against the detectable in a patient's tissues or bodily fluids.

Methods of treatment also include inhibiting microorganism replication in vivo, in an animal at risk for a microorganism infection or suffering from such an infection, by administering a sufficient concentration of a compound of Formula I or Formula II to inhibit bacterial survival *in vitro.* By "sufficient concentration" of a compound administered to the patient is meant the concentration of the compound available in the animal's system to prevent or combat the infection. Such a concentration by be ascertained experimentally, for example by assaying blood concentration of the compound, or theoretically, by calculating bioavailability. The amount of a compound sufficient to inhibit bacterial survival *in vitro* may be determined with a conventional assay for bacterial survival such as the Minimum Inhibitory Concentration (MIC) Assay disclosed in Example 5, which follows.

The invention also includes using compounds of Formula I and Formula I in prophylactic therapies. In the context of prophylactic or preventative treatment an effective amount of a compound of the invention is an amount sufficient to significantly decrease the treated animal's risk of contracting a microorganism infection.

Compounds of the invention are particularly useful for treating and preventing infectious disorders. These include for example: ocular infections such as conjunctivitis; urinary tract and genital infections, such as complicated urinary tract infections, acute urinary tract and genital infections, such as pyelonephritis, cervical gonococcal infections, cystitis, urethral chlamydial infections, cervical chlamydial infections, urethral gonococcal infections, and prostatitis, respiratory infections, such as lower respiratory tract infections, acute sinusitis, acute exacerbations of chronic bronchitis, community-acquired pneumonia, and nosocomial pneumonia, skin infections, such as skin-structure infections, impetigo, folliculitis, boils, scalded skin syndrome, and cellulites, and other infections such as bone infections, joint infections, infectious diarrhea, typhoid fever, intra-abdominal infections, gynecologic infections, including toxic shock syndrome, pelvic infections, and post-surgical infections.

The disclosed compounds are useful for treating infections caused by the following microorganisms:
Aerobic Gram-positive Microorganisms: Including but not limited to *Enterococcus faecalis, Enterococcus faecium, Staphylococcus aureus, Staphylococcus epidermidis, Staphylococcus saprophyticus, Streptococcus pneumoniae, Streptococcus pyogenes, Staphylococcus haemolyticus,* and *Staphylococcus hominis.*
Aerobic Gram-negative Microorganisms: Including but not limited to *Campylobacter jejuni, Citrobacter diversus, Citrobacter freundii, Enterobacter cloacae, Escherichia coli, Haemophilus influenzae, Haemophilus parainfluenzae, Klebsiella pneumoniae, Moraxella catarrhalis, Morganella morganii, Neisseria gonorrhoea, Proteus mirabilis, Proteus vulgaris, Providencia rettgeri, Providencia stuartii, Pseudomonas aeruginosa, Stenotrophomonas maltophila, Salmonella typhi, Serratia marcescens, Shigella boydii, Shigella dysenteriae, Shigella flexneri, Shigella sonnei. Acinetobacter Iwoffi, Aeromonas hydrophila, Edwardsiella tarda, Enterobacter aerogenes, Klebsiella oxytoca, Legionella pneumophila, Pasteurella multocida, Salmonella enteritidis, Vibrio cholerae, Vibrio parahaemolyticus, Vibrio vulnificus, Yersinia enterocolitica* and *H. Pylorii.*
Non-bacterial microorganisms: *Mycoplasma, Legionella* and *Chlamydia.*

Dosage levels of the order of from about 0.1 mg to about 140 mg per kilogram of body weight per day are useful in the treatment of the above-indicated conditions (about 0.5 mg to about 7 g per patient per day). The amount of active ingredient that may be combined with the carrier materials to produce a single dosage form will vary depending upon the host treated and the particular mode of administration. Dosage unit forms will generally contain between from about 1 mg to about 500 mg of an active ingredient.

Frequency of dosage may also vary depending on the compound used and the particular disease treated. However, for treatment of most infectious disorders, a dosage regimen of 4 times daily or less is preferred and a dosage regimen of 1 or 2 times daily is particularly preferred.

It will be understood, however, that the specific dose level for any particular patient will depend upon a variety of factors including the activity of the specific compound employed, the age, body weight, general health, sex, diet, time of administration, route of administration, and rate of excretion, drug combination and the severity of the particular disease undergoing therapy.

### COMBINATION ADMINISTRATION

The compounds of the invention may also be useful in combination with other pharmaceutically active agents such as antibacterial agents, antiviral agents, antifungal agents, anti-inflammatories, interferon, efflux-pump inhibitors, and beta-lactamase inhibitors. Antibiotic agents include any molecule that tends to prevent, inhibit or destroy life and as such, includes anti-bacterial agents, anti-fungicides, anti-viral agents, and anti-parasitic agents.

Pharmaceutical compositions of the invention include single dosage forms containing of a compound of Formula I and/or Formula II and one or more other active agent, dosage forms containing more than one compound of Formula I and/ or Formula II, and separate administration of a compound of Formula I and/or Formula II with another active agent.

The following active agents, which are useful in combinations of the invention, may be isolated from an organism that produces the agent or synthesized by methods known to those of ordinary skill in the art of medicinal chemistry or purchased from a commercial source.

Anti-bacterial antibiotic agents include, but are not limited to, penicillins, cephalosporins, carbacephems, cephamycins, carbapenems, monobactams, aminoglycosides, glycopeptides, quinolones, tetracyclines, macrolides, and fluoroquinolones (see Table below). Examples of antibiotic agents include, but are not limited to, Penicillin G (CAS Registry No.: 61-33-6); Methicillin (CAS Registry No.: 61-32-5); Nafcillin (CAS Registry No.: 147-52-4); Oxacillin (CAS Registry No.: 66- 79-5); Cloxacillin (CAS Registry No.: 61-72-3); Dicloxacillin (CAS Registry No.: 3116-76-5); Ampicillin (CAS Registry No.: 69-53-4); Amoxicillin (CAS Registry No.: 26787-78-0); Ticarcillin (CAS Registry No.: 34787-01-4); Carbenicillin (CAS Registry No.: 4697-36-3); Mezlocillin (CAS Registry No.: 51481-65-3); Azlocillin (CAS Registry No.: 37091-66-0); Piperacillin (CAS Registry No.: 61477-96-1); Imipenem (CAS Registry No.: 74431-23-5); Aztreonam (CAS Registry No.: 78110-38-0); Cephalothin (CAS Registry No.: 153-61-7); Cefazolin (CAS Registry No.: 25953-19-9); Cefaclor (CAS Registry No.: 70356-03-5); Cefamandole formate sodium (CAS Registry No.: 42540-40-9); Cefoxitin (CAS Registry No.: 35607-66-0); Cefuroxime (CAS Registry No.: 55268-75-2); Cefonicid (CAS Registry No.: 61270-58-4); Cefmetazole (CAS Registry No.: 56796-20-4); Cefotetan (CAS Registry No.: 69712-56-7); Cefprozil (CAS Registry No.: 92665-29-7); Loracarbef (CAS Registry No.: 121961-22-6); Cefetamet (CAS Registry No.: 65052-63-3); Cefoperazone (CAS Registry No.: 62893-19-0); Cefotaxime (CAS Registry No.: 63527-52-6); Ceftizoxime (CAS Registry No.: 68401-81-0); Ceftriaxone (CAS Registry No.: 73384-59-5); Ceftazidime (CAS Registry No.: 72558-82-8); Cefepime (CAS Registry No.: 88040-23-7); Cefixime (CAS Registry No.: 79350-37-1); Cefpodoxime (CAS Registry No.: 80210-62-4); Cefsulodin (CAS Registry No.: 62587-73-9); Fleroxacin (CAS Registry No.: 79660-72-3); Nalidixic acid (CAS Registry No.: 389-08-2); Norfloxacin (CAS Registry No.: 70458-96-7); Ciprofloxacin (CAS Registry No.: 85721-33- 1); Ofloxacin (CAS Registry No.: 82419-36-1); Enoxacin (CAS Registry No.: 74011-58-8); Lomefloxacin (CAS Registry No.: 98079-51-7); Cinoxacin (CAS Registry No.: 28657-80-9); Doxycycline (CAS Registry No.: 564-25-0); Minocycline (CAS Registry No.: 10118-90-8); Tetracycline (CAS Registry No. : 60-54-8); Amikacin (CAS Registry No.: 37517-28-5); Gentamicin (CAS Registry No.: 1403-66-3); Kanamycin (CAS Registry No.: 8063-07-8); Netilmicin (CAS Registry No.: 56391-56-1); Tobramycin (CAS Registry No.: 32986-56-4); Streptomycin (CAS Registry No.: 57-92-1); Azithromycin (CAS Registry No.: 83905-01-5); Clarithromycin (CAS Registry No.: 81103-11-9); Erythromycin (CAS Registry No.: 114-07-8); Erythromycin estolate (CAS Registry No.: 3521-62-8); Erythromycin ethyl succinate (CAS Registry No.: 41342-53-4); Erythromycin glucoheptonate (CAS Registry No.: 23067-13-2); Erythromycin lactobionate (CAS Registry No.: 3847-29-8); Erythromycin stearate (CAS Registry No.: 643-22-1); Vancomycin (CAS Registry No.: 1404- 90-6); Teicoplanin (CAS Registry No.: 61036-64-4); Chloramphenicol (CAS Registry No.: 56-75-7); Clindamycin (CAS Registry No.: 18323-44-9); Trimethoprim (CAS Registry No.: 738-70-5); Sulfamethoxazole (CAS Registry No.: 723-46-6); Nitrofurantoin (CAS Registry No.: 67-20-9); Rifampin (CAS Registry No.: 13292-46-1); Mupirocin (CAS Registry No.: 12650-69-0); Metronidazole (CAS Registry No.: 443-48-1); Cephalexin (CAS Registry No.: 15686-71-2); Roxithromycin (CAS Registry No.: 80214-83-1); Co- amoxiclavuanate; combinations of Piperacillin and Tazobactam; and their various salts, acids, bases, and other derivatives.

Anti-fungals agents include but are not limited to Amphotericin B, Candicidin, Dermostatin, Filipin, Fungichromin, Hachimycin, Hamycin, Lucensomycin, Mepartricin, Natamycin, Nystatin, Pecilocin, Perimycin, Azaserine, Griseofulvin, Oligomycins, Neomycin, Pyrrolnitrin, Siccanin, Tubercidin, Viridin, Butenafine, Naftifine, Terbinafine, Bifonazole, Butoconazole, Chlordantoin, Chlormidazole, Cloconazole, Clotrimazole, Econazole, Enilconazole, Fenticonazole, Flutrimazole, Isoconazole, Ketoconazole, Lanoconazole, Miconazole, Omoconazole, Oxiconazole, Sertaconazole, Sulconazole, Tioconazole, Tolciclate, Tolindate, Tolnaftate, Fluconawle, Itraconazole, Saperconazole, Terconazole, Acrisorcin, Amorolfine, Biphenamine, Bromosalicylchloranilide, Buclosamide, Calcium Propionate, Chlorphenesin, Ciclopirox, Cloxyquin, Coparaffinate, Diamthazole, Exalamide, Flucytosine, Halethazole, Hexetidine, Loflucarban, Nifuratel, Potassium Iodide, Propionic Acid, Pyrithione, Salicylanilide, Sodium Propionate, Sulbentine, Tenonitrozole, Triacetin, Ujothion, Undecylenic Acid, and Zinc Propionate.

Antiviral agents include, but are not limited to, Acyclovir, Cidofovir, Cytarabine, Dideoxyadenosine, Didanosine, Edoxudine, Famciclovir, Floxuridine, Ganciclovir, Idoxuridine, Inosine Pranobex, Lamivudine, MADU, Penciclovir, Sorivudine, Stavudine, Trifluridine, Valacyclovir, Vidarabine, ZaIcitabine, Zidovudine, Acemannan, Acetylleucine, Amantadine, Amidinomycin, Delavirdine, Foscarnet, Indinavir, Interferon-α, Interferon-β, Interferon-γ, Kethoxal, Lysozyme, Methisazone, Moroxydine, Nevirapine, Podophyllotoxin, Ribavirin, Rimantadine, Ritonavir2, Saquinavir, Stailimycin, Statolon, Tromantadine, and Xenazoic Acid.

Antiinflammatory agents include, but are not limited to, Enfenamic Acid, Etofenamate, FIufenamic Acid, Isonixin, Meclofenamic Acid, Mefenamic Acid, Niflumic Acid, Talniflumate, Terofenamate, Tolfenamic Acid, Aceclofenac, Acemetacin, Alclofenac, Amfenac, Amtolmetin Guacil, Bromfenac, Bufexamac, Cinmetacin, Clopirac, Diclofenac, Etodolac, Felbinac, Fenclozic Acid, Fentiazac, Glucametacin, Ibufenac, Indomethacin, Isofezolac, Isoxepac, Lonazolac, Metiazinic Acid, Mofezolac, Oxametacine, Pirazolac, Proglumetacin, Sulindac, Tiaramide, Tolmetin, Tropesin, Zomepirac, Bumadizon, Butibufen, Fenbufen, Xenbucin, Clidanac, Ketorolac, Tinoridine, Alminoprofen, Benoxaprofen, Bermoprofen, Bucloxic Acid, Carprofen, Fenoprofen, Flunoxaprofen, Flurbiprofen, Ibuprofen, Ibuproxam, Indoprofen, Ketoprofen, Loxoprofen, Naproxen, Oxaprozin, Piketoprofen, Pirprofen, Pranoprofen, Protizinic Acid, Suprofen, Tiaprofenic Acid, Ximoprofen, Zaltoprofen, Difenamizole, Epirizole, Apazone, Benzpiperylon, Feprazone, Mofebutazone, Morazone, Oxyphenbutazone, Phenylbutazone, Pipebuzone, Propyphenazone, Ramifenazone, Suxibuzone, Thiazolinobutazone, Acetaminosalol, Aspirin, Benorylate, Bromosaligenin, Calcium Acetylsalicylate, Diflunisal, Etersalate, Fendosal, Gentisic Acid, Glycol Salicylate, Imidazole Salicylate, Lysine Acetylsalicylate, Mesalamine, Morpholine Salicylate, I-Naphthyl Salicylate, Olsalazine, Parsalmide, Phenyl Acetylsalicylate, Phenyl Salicylate, Salacetamide, Salicylamide O-Acetic Acid, Salicylsulfuric Acid, Salsalate, Sulfasalazine, Ampiroxicam, Droxicam, Isoxicam, Lornoxicam, Piroxicam, Tenoxicam, epsilon-Acetamidocaproic Acid, S-Adenosylmethionine, 3-Amino-4-hydroxybutyric Acid, Amixetrine, Bendazac, Benzydamine, alpha-Bisabolol, Bucolome, Difenpiramide, Ditazol, Emorfazone, Fepradinol, Guaiazulene, Nabumetone, Nimesulide, Oxaceprol, Paranyline, Perisoxal, Proquazone, Superoxide Dismutase, Tenidap, Zileuton, 21-Acetoxypregnenolone, Alclometasone, Algestone, Amcinonide, Beclomethasone, Betamethasone, Budesonide, Chloroprednisone, Clobetasol, Clobetasone, Clocortolone, Cloprednol, Corticosterone, Cortisone, Cortivazol, Deflazacort, Desonide, Desoximetasone, Dexamethasone, Diflorasone, Diflucortolone, Difluprednate, Enoxolone, Fluazacort, Flucloronide, Flumethasone, Flunisolide, Fluocinolone Acetonide, Fluocinonide, Fluocortin Butyl, Fluocortolone, Fluorometholone, Fluperolone Acetate, Fluprednidene Acetate, Fluprednisolone, Flurandrenolide, Fluticasone Propionate, Formocortal, Halcinonide, Halobetasol Propionate, Halometasone, Halopredone Acetale, Hydrocortamate, Hydrocortisone, Loteprednol Etabonale, Mazipredone, Medrysone, Meprednisone, Methylprednisolone, Mometasone Furoate, Paramethasone, Prednicarbate, Prednisolone, Prednisolone 25-Diethylamino-acetate, Prednisolone Sodium Phosphate, Prednisone, Prednival, Prednylidene, Rimexolone, Tixocortol, Triamcinolone, Triamcinolone Acetonide, Triamcinolone Benetonide, and Triamcinolone Hexacetonide.

Compounds of the invention may be combined with one or more Beta lactamase inhibitor when used in combination with a beta-lactam class antibiotic, such as penicillin or cephalosporins. Beta-lactamase inhibitors include, but are not limited to Clavulanic acid, Sulbactam, Sultamacillin, and Tazobactam.

Compounds of the invention may also be combined with one or more efflux pump inhibitor, such as a quinazolinone efflux pump inhibitors, d-ornithine-d-homophenylalanine-3-aminoquinoline, Phe-Arg-b-naphthylamide, propafenone, a phenothiazine or thioxanthene efflux pump inhibitor, 1-aza-9-oxafluorenes, N-[4-[2-(3,4-dihydro-6,7-dimethoxy-2(1H)-isoquiolinyl)ethyl]phenyl]-9,10-dihydro-5-methoxy-9-oxo-4-Acridinecarboxamide, reserpine, Milbemycin, Cinchonine, Verapamil, L-phenylalanyl-N-2-naphthalenyl-L-Argininamide (and analogs), 5'-methoxyhydnocarpin-D, methylxanthines, FK506, a cyclosporine efflux pump inhibitor, Nocardamine and other siderophores, Amiodarone, Cyclosporin A, Ro11-2933 (DMDP), Quinidine, and the optical isomers of Propranolol, Quinine (SQ1) and Quinidine, Quinine-10,11-epoxide, Quercetin, Amitriptyline, Taxuspine C derivatives, Emodin, MC-002434; Agosterol A; Pheophorbide; pyridoquinolines such as 2,2'-[(2,8,10-trimethylpyrido[3,2-g]quinoline-4,6-diyl)bis(oxy)]bis[N,N-dimethyl-ethanamine, Gitonavir, and Gemfibrozil.

### SYNTHESIS OF COMPOUNDS

The compounds of the invention are prepared according to methods well-known to those skilled in the art of organic chemical synthesis. The starting materials used in preparing the compounds of the invention are known, made by known methods, or are commercially available.

It is recognized that the skilled artisan in the art of organic chemistry can readily carry out standard manipulations of organic compounds without further direction. Examples of such manipulations are discussed in standard texts such as J. March, Advanced Organic Chemistry, John Wiley & Sons, 1992.

The skilled artisan will readily appreciate that certain reactions are best carried out when other functionalities are masked or protected in the compound, thus increasing the yield of the reaction and/or avoiding any undesirable side reactions. Often, the skilled artisan utilizes protecting groups to accomplish such increased yields or to avoid the undesired reactions. These reactions are found in the literature and are also well within the scope of the skilled artisan. Examples of many such manipulations can be found in, for example, T. Greene, Protecting Groups in Organic Synthesis, John Wiley & Sons, 1981.

The compounds of the invention may have one or more chiral center. As a result, one may selectively prepare one optical isomer, including diastereomers and enantiomers, over another, for example by chiral starting materials, catalysts or solvents, or may prepare both stereoisomers or both optical isomers, including diastereomers and enantiomers at once (a racemic mixture). Since the compounds of the invention may exist as racemic mixtures, mixtures of optical isomers, including diastereomers and enantiomers, or stereoisomers may be separated using known methods, such as through the use of, for example, chiral salts and chiral chromatography.

In addition, it is recognized that one optical isomer, including a diastereomer and enantiomer, or a stereoisomer, may have favorable properties over the other. When a racemic mixture is discussed herein, it is clearly contemplated to include both optical isomers, including diastereomers and enantiomers, or one stereoisomer substantially free of the other.

The invention also includes all energetically accessible conformational and torsional isomers of the compounds disclosed.

When the substituent R₇ in a compound of Formula I or Formula II is attached via an unsaturated aliphatic group, for example when R₇ is phenyl (C₂-C₆alkenyl), all geometric isomers of the compound are included.

This invention is further illustrated by the following examples that should not be construed as limiting.

### EXAMPLES

### ABBREVIATIONS

The following abbreviations are used in the reaction schemes and synthetic examples, which follow. This list is not meant to be an all-inclusive list of abbreviations used in the application as additional standard abbreviations, which are readily understood by those skilled in the art of organic synthesis, may also be used in the synthetic schemes and examples.
(Boc)₂O- Di-t-butyl dicarbonate
Cbz-Cl - Benzyloxycarbonyl chloride
DMF - N,N-Dimethylformamide
DMSO - Dimethylsulfoxide
Et₃N - Triethyl amine
Et₂O - Diethyl ether
EtOH - ethanol
EtOAc - Ethyl acetate
PPh₃ - Triphenyl phosphate
PTLC - Preparative thin layer chromatography
t-BuOK - *tert*-Butyl oxide
TsCl - Tosyl chloride
THF - Tetrahydrofuran

### GENERAL METHODS

All nonaqueous reactions are performed under an atmosphere of dry argon gas (99.99%) using oven- or flame-dried glassware. Microwave-assisted syntheses are conducted in a commercial microwave reactor (Discover System, CEM Corporation). The progress of reactions is monitored using thin-layer chromatography on glass plates coated with Merck silica gel 60 (F₂₅₄). Flash column chromatography is performed on Merck silica gel 60 (230-400 mesh). Melting points are recorded on an Electrothermal Model IA9100 digital melting point apparatus; the reported values are the average of three measurements. NMR spectra are recorded at ambient temperature using a Bruker Avance 300 spectrometer (¹H at 300.1 MHz, ¹³C at 75.5 MHz, and ¹⁹F at 282.4 MHz). The chemical shifts for ¹H and ¹³C are reported in parts per million (δ) relative to external tetramethylsilane and are referenced to signals of residual protons in the deuterated solvent. The chemical shifts for ¹⁹F are reported in parts per million (δ) relative to external fluorotrichloromethane. Assignment of ¹H and ¹³C NMR data is based on extensive two-dimensional correlation experiments (¹H-¹H COSY, ¹H-¹³C HMQC, ¹H-¹³C HMBC, and ¹H-¹H NOESY) and the usual principles of NMR spectroscopy (the magnitudes of coupling constants and chemical shifts). Analytical HPLC is performed using a YMC Pack Pro C18 50 x 4.6 mm 5 µm column with an isocratic elution of 0.24 min at 90:10 H₂O:CH₃CN containing 0.1% TFA followed by a 4-min linear gradient elution from 90:10 to 10:90 at a flow rate of 2.5 mL/min with UV detection at 254 nm. Preparative HPLC is performed using a YMC Pack Pro C18 150 x 20.0 mm 5 µm column with an isocratic elution of 0.24 min at 97:3 H₂O:CH₃CN containing 0.1% TFA followed by a 10-min linear gradient elution from 97:3 to 0:100 at a flow rate of 18.0 mL/min with UV detection at 254 nm. Low-resolution mass spectra are recorded on a Thermo Finnigan Surveyor MSQ instrument (operating in APCI mode) equipped with a Gilson liquid chromatograph. Unless noted otherwise, the quasi-molecular ions, [M + H]⁺, observed in the low-resolution mass spectra are the base peaks. Elemental analysis is performed at Prevalere Life Sciences, Inc. (Whitesboro, NY).

### EXAMPLE 1. METHOD FOR THE PREPARATION OF 7-[(4Z)-3-AMINOMETHYL-4-METHOXYIMINO-PYRROLIDIN-1-YL)]-9-CYCLOPROPYL-6-FLUORO-9H-ISOTHIAZOLO [5,4-B]QUINOLINE-3,4-DIONE (12).

7-[(4Z)-3-Aminomethyl-4-methoxyimino-pyrrolidin-1-yl)]-9-cyclopropyl-6-fluoro-9H-isothiazolo[5,4-b]quinoline-3,4-dione (**12**) is prepared in accordance with the synthetic scheme set forth below.

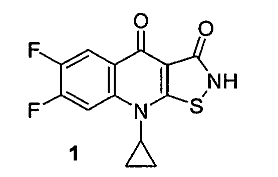

### Step 1. 9-Cyclopropyl-6,7-difluoro-9H-isothiazolo[5,4-b]quinoline-3,4-dione (1).

9-Cyclopropyl-6,7-difluoro-9H-isothiazolo[5,4-b]quinoline-3,4-dione is prepared from 2,4,5-trifluorobenzoic acid using the procedure of Chu *(*J. Heterocyclic Chem., (1990) 27: 839.). LCMS (API) m/z calcd for C₁₃H₈F₂N₂O₂S 294 ([M⁺]); found 295 ([M+H]⁺). ¹H NMR (300 MHz, DMSO-d6): δ 1.25 (m, 2H), 1.36(m, 2H), 3.56(m, 1H), 8.04(dd, J_{H-F6}= 11 Hz, J_{H-F7}=8.8Hz, 1H), 8.10 (dd, J_{H-F7}= 11.6Hz, J_{H-F6}= 5.8 Hz, 1H). ¹⁹F NMR (DMSO-*d6*): δ 143.30 (d, J = 27 Hz), 130.12 (d, J = 27 Hz).

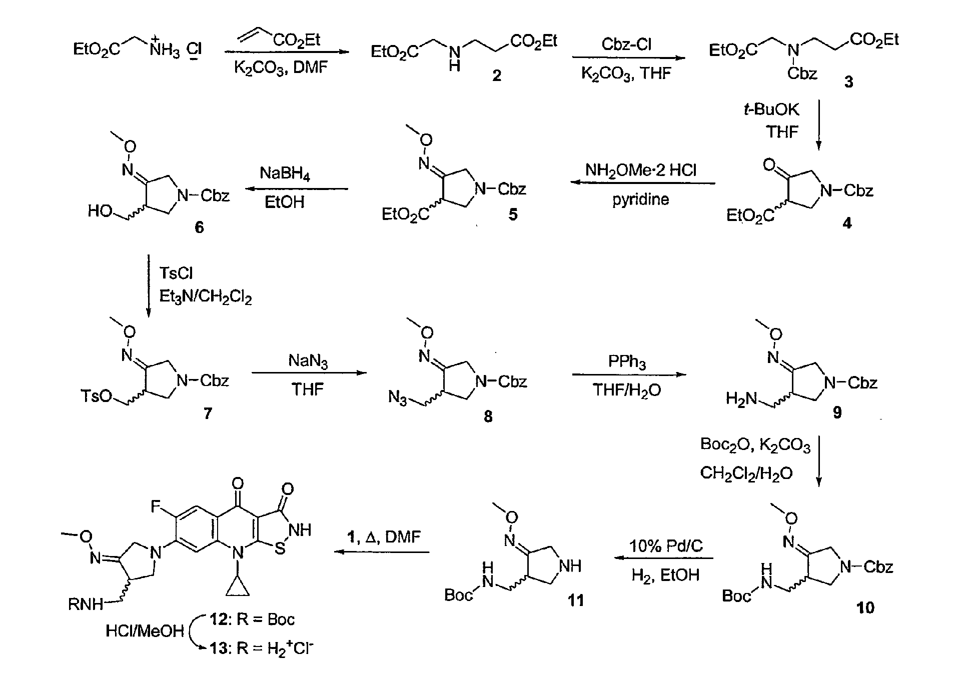

### Step 2. 3-(Ethoxycarbonylmethyl-amino)-propionic acid ethyl ester (2).

A mixture of ethyl glycinate hydrochloride (25.0 g, 179 mmol) and K₂CO₃ (55.0 g, fine powder, 362 mmol) in DMF (300 ml) is stirred for 30 min at room temperature. Ethyl acrylate (19.7 g, 197 mmol) is added slowly (∼30 min.) and the reaction mixture is then stirred at 80 °C for 8 hr. DMF is removed *in vacuo* and the residue extracted (2 x 200 mL) with EtOAc/H₂O. The organic layer is washed with water (50 mL) and brine (50 mL), dried (Na₂SO₄), and the EtOAc removed *in vacuo.* The residue is distilled (3 mm Hg, 100-105 °C) to afford **2** a clear oil. LCMS (API) m/z calc'd for C₉H₁₇NO₄ 203 ([M⁺]); found 204 ([M+H]⁺. ¹H NMR (300 MHz, CDCl₃): δ 1.27 (m, 6H), 2.24 (bs, 1H), 2.52 (t, *J*=5.1 Hz, 2H), 2.92 (t, *J*=6.9 Hz, 2H), 3.43 (s, 2H), 4.17 (m, 4H).

### Step 3. 3-(Benzyloxycarbonyl-ethoxycarbonylmethyl-amino)-propionic acid ethyl ester (3).

A mixture of Compound **2** (20.0 g, 98 mmol) and K₂CO₃ (20.4 g, 148 mmol, fine powder) in CH₃CN (500 ml), is stirred for 30 min. Cbz-Cl (14 ml, 99 mmol) is then added slowly. The reaction mixture is refluxed for 5 hr, additional Cbz-Cl (7 ml, 49 mmol) is added, and the mixture is refluxed for 15 hr. The solvent is *removed in vacuo,* extracted (2 x 200 mL) with CHCl₃/H₂O. The organic layer is washed with water (50 mL) and brine (50 mL), dried (Na₂SO₄), and the CHCl₃ removed *in vacuo.* The residue is purified by flash chromatography (EtOAc/hexanes: 1/4) to afford **3** as a clear oil. LCMS (API) m/z calcd for C₁₇H₂₃NO₆ 337 ([M⁺]); found 338 ([M+H]⁺). ¹H NMR (300 MHz, CDCl₃):451.24 (m, 6H), 3.90-4.30 (m, 10H), 5.12 (s, 2H), 7.34-7.38 (m, 5H).

### Step 4. 4-Oxo-pyrrolidine-1,3-dicarboxylic acid 1-benzyl ester 3-ethyl ester (4).

*t*-BuOK 9.6g (86 mmol) is added to a mixture of **3** (12.0 g, 36 mmol) in THF (300 ml). The reaction mixture is stirred for 2 hr, satu rated aq NH₄Cl (50 ml) is then added. The THF is removed *in vacuo.* The residue is extracted with (2 x 150 mL) CHCl₃/H₂O and the organic layer washed with water (50 mL) and brine (50 mL), dried (Na₂SO₄), and the CHCl₃ is removed *in vacuo.* The residue is purified by flash chromatography (EtOAc) to afford **4** as a white solid. LCMS (API) m/z calcd for C₁₅H₁₇NO₅ 291 ([M⁺]); found 292 ([M+H]⁺). ¹H NMR (300 MHz, CDCl₃): δ 1.25-1.34 (m, 3H), 3.93-4.32 (m, 7H), 5.18 (s, 2H), 7.34-7.40 (m, 5H).

### Step. 5. 4-Methoxyimino-pyrrolidine-1,3-dicarboxylic acid 1-benzyl ester 3-ethyl ester (5).

A mixture of **4** (2.0 g, 6.9 mmol) and O-methylhydroxylamine dihydrochloride (860 mg, 10.3 mmol) in pyridine (20 mL) is stirred at 80 °C for 4 **hr**. The pyridine is removed, toluene (20 ml) is added and then removed *in vacuo.* The residue is extracted with (2 x 50 mL) CHCl₃/H₂O, the organic layer is washed with water (10 mL) and brine (10 mL), dried (Na₂SO₄), and the CHCl₃ removed *in vacuo.* The residue is purified by flash chromatography (EtOAc/hexanes: 1/4) to afford **5** (1.82 g, 83 %) as a clear oil. LCMS (API) m/z calcd for C₁₆H₂₀N₂O₅ 320 ([M⁺]); found 321 ([M+H]⁺). ¹H NMR (300 MHz, CDCl₃): δ 1.24-1.33 (m, 3H), 3.70-4.05 (m, 6H), 4.15-4.28. (m, 4H), 5.18 (s, 2H), 7.34-7.40 (m, 5H). *Step 6. 3-Hydroxymethyl-4-methoxyimino-pyrrolidine-1-carboxylic acid benzyl ester* (**6**)*.*

NaBH₄ (327 mg, 8.6 mmol) is added to a solution of 5 (1.39 g, 4.3 mmol) in EtOH (20 ml) at 0 °C and then stirred for 5 hr at room temperature. Saturated aqueous NaHCO₃ (2 ml) and EtOAc (2 ml) are then added, and the reaction mixture is stirred for additional 1 hr at room temperature. The solvents are then *removed in vacuo.* The residue is extracted with EtOAc/H₂O (2 x 50 mL) and organic layer is washed with water (10 mL) and brine (10 mL), dried (Na₂SO₄), and the EtOAc *removed in vacuo.* The residue is purified by flash chromatography (EtOAc/hexanes: 1/1) to afford 6 as a clear oil. Starting material 5 is also recovered (700 mg, 50 %). LCMS (API) m/z calcd for C₁₄H₁₈N₂O₄ 278 ([M⁺]); found 279 ([M+H]⁺). ¹H NMR (300 MHz, CDCl₃): δ 2.55 (bs, 1H), 3.14 (m, 1H), 3.40 (m, 1H), 3.76 (d, *J*=6.8 Hz, 2H), 3.85 (s, 3H), 3.90-3.95 (m, 1H), 4.20 (m, 2H), 5.17 (s, 2H), 7.31-7.41 (m, 5H).

### Step 7. 3-Methoxyimino-4-(toluene-4-sulfonyloxymethyl)-pyrrolidine-1-carboxylic acid benzyl ester (7).

A mixture of 6 (600 mg, 2.2 mmol), Et₃N (2 ml, 14.3 mmol), TsCl (492 mg, 2.6 mmol) in CH₂Cl₂ (20 ml) is stirred for 2 days at room temperature. The solvents are then removed *in vacuo.* The residue is purified by flash chromatography (EtOAc/CHCl₃: 1/15) to afford **7** as a pale yellow oil. Starting material **6** is also recovered. LCMS (API) m/z calcd for C₂₁H₂₄N₂O₆S 432 ([M⁺]); found 433 ([M+H]⁺). ¹H NMR (300 MHz, CDCl₃): δ 2.45 (s, 3H), 3.22 (m, 1H), 3.42 (m, 1H), 3.78-3.89 (m, 1H), 3.82 (s, 3H), 3.97-4.33 (m, 4H), 5.13 (s, 2H), 7.28-7.40 (m, 7H), 7.78 (d, *J*=8.2 Hz, 2H).

### Step 8. 3-Azidomethyl-4-methoxyimino-pyrrolidine-1-carboxylic acid benzyl ester (8).

A mixture of 7 (340 mg, 0.79 mmol) and NaN₃ (102 mg, 1.57 mmol) in DMF (5 ml) is stirred for 2 hr at 80 °C. The solvents are then *removed in vacuo.* The residue is extracted with EtOAc/H₂O (2 x 30 mL) and the organic layer is washed with water (5 mL) and brine (5 mL), dried (Na₂SO₄), and the EtOAc removed *in vacuo.* The residue is used in the next reaction without any purification. LCMS (API) m/z calcd for C₁₄H₁₇N₅O₃ 303 ([M⁺]); found 276 ([M-N₂+H]⁺). ¹H NMR (300 MHz, CDCl₃): δ 3.12 (m, 1H), 3.47 (m, 2H), 3.57-3.71 (m, 1H), 3.89 (s, 3H), 3.89-3.90 (m, 1H), 4.17 (m, 2H), 5.15 (s, 2H), 7.30-7.39 (m, 5H).

### Step 9. 3-Aminomethyl-4-methoxyimino-pyrrolidine-1-carboxylic acid benzyl ester (9).

A mixture of **8** (50 mg, 0.16 mmol) and PPh₃ (250 mg, 0.95 mmol) in aqueous THF. (THF/H₂O: 8.5 ml/1.5 ml) is refluxed for 3 hr. The solvents are then removed *in vacuo.* The residue is purified by flash chromatography (EtOAc, CHCl₃/MeOH: 10/1) to afford 9 (37 mg, 81 %) as a clear oil to solid. LCMS (API) m/z calcd for C₁₄H₁₉N₃O₃ 277 ([M⁺]); found 278 ([M+H]⁺). ¹H NMR (300 MHz, CDCl₃): δ 2.88-3.16 (m, 2H), 3.38 (m, 1H), 3.68 (m, 2H), 3.88 (s, 3H), 4.17 (m, 2H), 4.17 (m, 2H), 5.14 (s, 2H), 7.27-7.40 (m, 5H).

### Step 10. 3-(tert-Butoxycarbonylamino-methyl)-4-methoxyimino-pyrrolidine-1-carboxylic acid benzyl ester (10).

A mixture of **9** (35 mg, 0.13 mmol), K₂CO₃ (35 mg, 0.25 mmol), Boc₂O (29 mg, 16.6 mmol) in CH₂Cl₂/H₂O (5 ml/5 ml) is stirred for 15 hr at room temperature. The organic layer is separated, dried (Na₂SO₄), and the solvent removed *in vacuo.* The residue is purified by flash chromatography (EtOAc/hexanes: 1/3, 1/2) to afford **10** as a clear oil. LCMS (API) m/z calcd for C₁₉H₂₇N₃O₅ 377 ([M⁺]); found 378 ([M+H]⁺). ¹H NMR (300 MHz, CDCl₃): δ 1.43 (s, 9H), 3.02 (m, 1H), 3.33 (m, 3H), 3.84 (m, 1H), 3.88 (s, 3H), 4.16 (m, 2H), 4.98 (bs, 1H), 5.14 (s, 2H), 7.27-7.41 (m, 5H).

### Step 11. (4-Methoxymino-pyrrolidin-3-ylmethyl)-carbamic acid tert-butyl ester (11).

A mixture of **10** (240 mg, 0.64 mmol) and Pd/C (20 mg) in EtOH is stirred under H₂ (1 atm) for 4 hr. at room temperature. The mixture is filtered and the residue (156 mg) is used for next reaction without any purification. LCMS (API) m/z calcd for C₁₁H₂₁N₃O₃ 243 ([M⁺]); found 244 ([M+H]⁺). ¹H NMR (300 MHz, CDCl₃): δ 1.45 (s, 9H), 3.09 (m, 2H), 3.25-3.61 (m, 3H), 3.89 (s, 3H), 3.94 (m, 1H), 5.22 (bs, 1H), 6.45 (bs, 2H). *Step 12. 7-(3-Aminomethyl-4-methoxyimino-pyrrolidin-1-yl)-9-cyclopropyl-6-fluoro-9H-isothiazolo[5,4-b]quinoline-3,4-dione* (**13**).
A solution of 12 (300 mg, 0.58 mmol) in HCl/MeOH (∼1.25 M, 4.6 mL.) is stirred for 24 hr at room temperature. Et₂O (50 ml) is added to the reaction mixture and the precipitate is collected and washed with Et₂O (3 x 30 mL). The solid is dried *in vacuo* to give **13**. LCMS (API) m/z calcd for C₁₉H₂₁CLFN₅O₃S 417 ([M⁺]), found 418 (M+1). ¹H NMR (300 MHz, DMSO-d₆) δ 1.23 (m, 2H), 1.39 (m, 2H), 3.21 (m, 2H), 3.49 (m, 3H), 3.87 (s, 3H), 4.01 (m, 1H), 4.38 (s, 2H), 7.09 (d, 1H, *J=6.6* Hz), 7.73 (d, 1H, *J=*14.4 Hz). ¹⁹F NMR (350 MHz, DMSO-d₆) δ 130 (s, 1F). Anal. Calcd for C₁₉H₂₁ClFN₅O₃S•1.5H₂O: C, 47.45; H, 5.03; N, 14.56. Found: C, 47.04; H, 4.52; N, 14.35.
*Alternate preparations of 7-[(4Z)-3-Aminomethyl-4-methoxyimino-pyrrolidin-1-yl]]-9-cyclopropyl-6-fluoro-9H-isothiazolo[5,4-b]quinoline-3,4-dione* (**13**).

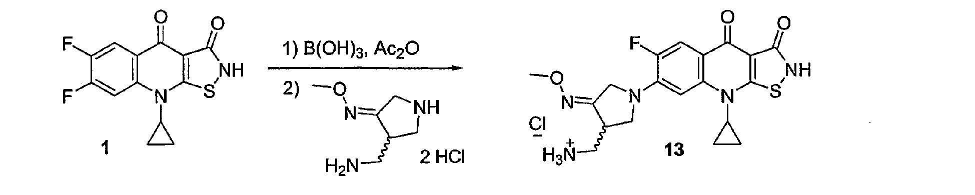

A mixture of boric acid (43 mg, 0.70 mmole), acetic anhydride (0.22 ml, 2.33 mmole) and acetic acid (0.13 ml) in toluene (0.43 ml) is refluxed for 3 hours. Intermediate **1** (172 mg, 0.58 mmole) is then added to the reaction mixture and refluxed another 4 hours. The volatiles are then removed *in vacuo.* 4-Aminomethyl-pyrrolidin-3-one O-methyl-oxime dihydrochloride (Tyger Scientific Inc., 380 mg, 1.76 mmole), triethyl amine (1 ml), and acetonitrile (10 ml) are then added to the residue, and the reaction mixture is refluxed for 4 hours. The volatiles are then removed *in vacuo.* The residue is washed with diethyl ether (1 x mL), a mixture of chloroform and methanol (1 x mL), and water (1 x mL). The residue is then purified by reverse phase HPLC (conditions) to afford **13**.

### EXAMPLE 2. ADDITIONAL COMPOUNDS

The following compounds of TABLE I are made by the methods given in Example 1. Those having skill in the art will recognize that the starting materials and reaction conditions may be varied and additional steps employed to produce the compounds shown in TABLE I.

Unless designated, the stereochemistry of the R7 group oxime double bond can be either Z or E, and the stereochemistry of other chiral carbons within the group can be racemic, R, or S.

| **Cpd. #** | **Structure** | **Name** | **Parent MW** | **Salt** | **MS** | **H-NMR** | **F-NMR** |
|---|---|---|---|---|---|---|---|
| 13 | 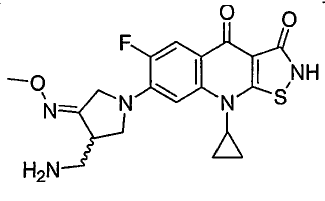 | 7-[(4Z)-3-Aminomethyl-4-methoxyimino-pyrrolidin-1- 9H-yl)]-9-cyclopropyl-6-fluoro-9H-isothiazolo[5,4-b]quinoline-3,4-dione | 417.5 | HCl l | LCMS *m*/*z* calcd for C₁₉H_{20F}N₅O₃ S([M]⁺)417, found M⁺+1 | ¹H-NMR (DMSO- D₆): 1.23 (2H, m), 1.38-1.40 (2H,m), 1.38-1.40 (2H, m), 3.22 (2H, m), 336- 3.61 (3H, m), 3.81 (7H, m) 7.10 (1H, d, J = 6.1 Hz), 7.73 (1H, d, J = 14.3 Hz) | ¹⁹F (DMSO-D₆): δ - 130.0 |
| 14 | 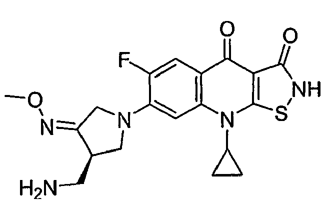 | 7-[(3R,4Z)-3-Aminomethyl-4-methoxyimino-pyrrolidin-1-yl)]-9-cyclopropyl-6-fluoro-9H-isothiazolo[5,4-b]quinoline-3,4-dione | | | | | |
| 15 | 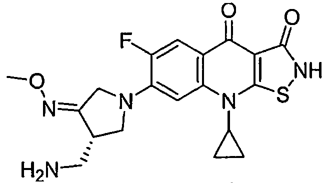 | 7-[(3 S,4Z)-3-Aminomethyl-4-methoxyimino-pyrrolidin-1-yl)]-9-cyclopropyl-6-fluoro-9H-isothiazolo[5,4-b]quinoline-3,4-dione | | | | | |
| 16 | 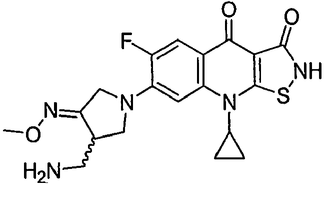 | 7-[(4E)-3-Aminomethyl-4-methoxyimino-pyrrolidin-1-yl)]-9-cyclopropyl-6-fluoro-9H-isothiazolo[5,4-b]quinoline-3,4-dione | | | | | |
| 17 | 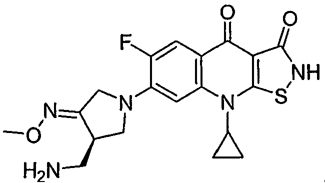 | 7-[(3R,4E)-3-Aminomethyl-4-methoxyimino-pyrrolidin-1-yl)]-9-cyclopropyl-6-fluoro-9H-isothiazolo[5,4-b]quinoline-3,4-dione | | | | | |
| 18 | 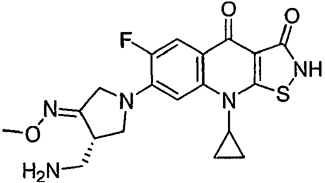 | 7-[(3S,4E)-3-Aminomethyl-4-methoxyimino-pyrrolidin-1-yl)]-9-cyclopropyl-6-fluoro-9H-isothiazolo[5,4-b]quinoline-3,4-dione | | | | | |
| 19 | 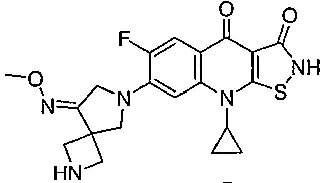 | 9-Cyclopropyl-6-fluoro-7-(8-methoxyimino-2,6-diaza-spiro[3.4]oct-6-yl)-9H-isothiazolo[5,4-b]quinoline-3,4-dione | | | | | |
| 20 | 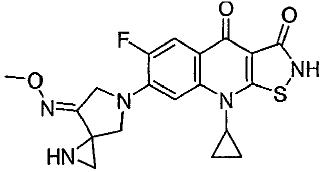 | 9-Cyclopropyl-6-fluoro-7-(7-methoxyimino-1,5-diaza-spiro[2.4]hept-5-yl)-9H-isothiazolo[5,4-b]quinoline-3,4-dione | | | | | |
| 21 | 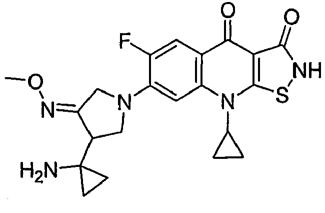 | 7-[3-(1-Amino-cyclopropyl)-4-methoxyimino-pyrrolidin-1-yl]-9-cyclopropyl-6-fluoro-9H-isothiazolo[5,4-b]quinoline-3,4-dione | | | | | |
| 22 | 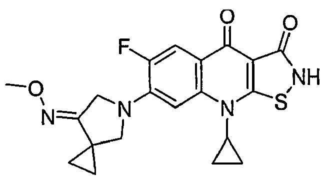 | 9-Cyclopropyl-6-fluoro-7-(7-methoxyimino-5-aza-spiro[2.4]hept-5-yl)-9H-isothiazolo[5,4-b]quinoline-3,4-dione | | | | | |
| 23 | 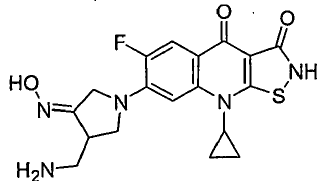 | 7-(3-Aminomethyl-4-hydroxyimino-pyrrolidin-1-yl)-9-cyclopropyl-6-fluoro-9H-isothiazolo[5,4-b]quinoline-3,4-dione | | | | | |
| 24 | 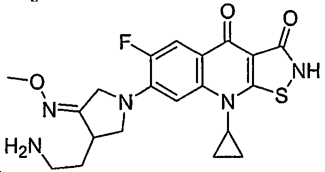 | 7-[3-(2-Amino-ethyl)-4-methoxyimino-pyrrolidin-1-yl]-9-cyclopropyl-6-fluoro-9H-isothiazolo[5,4-b]quinoline-3,4-dione | | | | | |
| 25 | 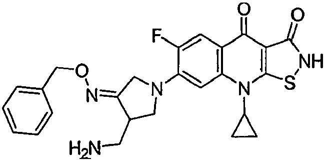 | 7-(3-Aminomethyl-4-benzyloxyimino-pyrrolidin-1-yl)-9-cyclopropyl-6-fluoro-9H-isothiazolo[5,4-b]quinoline-3,4-dione | | | | | |
| 26 | 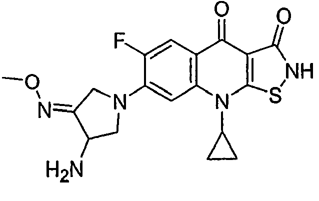 | 7-(3-Amino-4-methoxyimino-pyrrolidin-1-yl)-9-cyclopropyl-6-fluoro-9H-isothiazolo[5,4-b]quinoline-3,4-dione | | | | | |
| 27 | 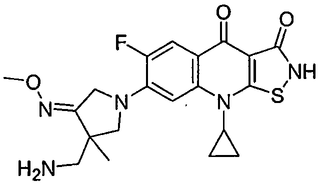 | 7-(3-Aminomethyl-4-methoxyimino-3-methyl-pyrrolidin-1-yl)-9-cyclopropyl-6-fluoro-9H-isothiazolo[5,4-b]quinoline-3,4-dione | 431.5 | HCl | LCMS *m*/*z* calcd for C₂₀H₂₂FN₅O₃ S([M]⁺)431, found M⁺+1 | ¹H-NMR (DMSO- ₆): δ 1.23 (2H, m), 1.36-1.42 (5H, m), 3.04-3.20 (3H, m), 3.48-3.60 (3H, m), 3.18-3.87 (1H,m), 3.18-3.87 (1H, m), 3.89 (3H, s), 4.41 (1H, m), 7.14 (1H, d, J = 7.0 Hz), 7.79 (1H, d, J = 14.4Hz), 8.13 (3H, m) | ¹⁹F (DMSO-D₆): δ-130.2 |
| 28 | 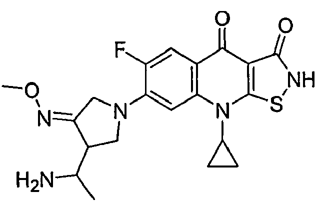 | 7-[3-(1-Amino-ethyl)-4-methoxyimino-pyrrolidin-1-yl]-9-cyclopropyl-6-fluoro-9H-isothiazolo[5,4-b]quinoline-3,4-dione | | | | | |
| 29 | 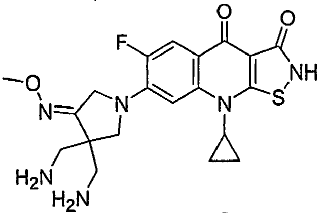 | 7-(3,3-Bis-aminomethyl-4-methoxyimino-pyrrolidin-1-yl)-9-cyclopropyl-6-fluoro-9H-isothiazolo[5,4-b]quinoline-3,4-dione | | | | | |
| 30 | 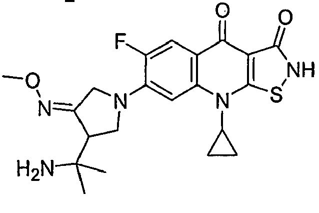 | 7-[3-(1-Amino-1-methyl-ethyl)-4-methoxyimino-pyrrolidin-1-yl]-9-cyclopropyl-6-fluoro-9H-isothiazolo[5,4-b]quinoline-3,4-dione | | | | | |
| 31 | 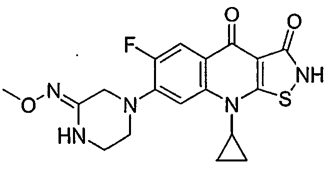 | 9-Cyclopropyl-6-fluoro-7-(3-methoxyimino-piperazin-1-yl)-9H-isothiazolo[5,4-b] quinoline-3,4-dione | | | | | |
| 32 | 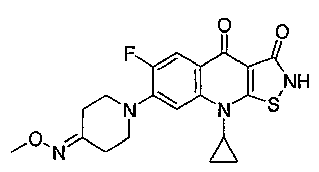 | 9-Cyclopropyl-6-fluoro-7-(4-methoxyimino-piperidin-1-yl)-9H-isothiazolo[5,4-b]quinoline-3,4-dione | | | | | |
| 33 | 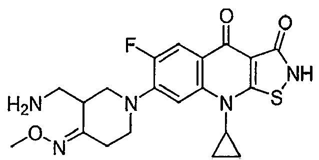 | 7-(3-Aminomethyl-4-methoxyimino-piperidin-1-yl)-9-cyclopropyl-6-fluoro-9H-isothiazolo[5,4-b]quinoline-3,4-dione | | | | | |
| 34 | 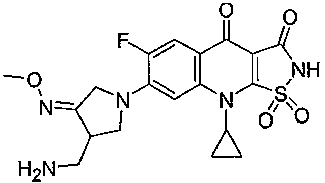 | 7-(3-Aminomethyl-4-methoxyimino-pyrrolidin-1-yl)-9-cyclopropyl-6-fluoro-1,1-dioxo-1,2-dihydro-9H-116-isothiazolo[5,4-b]quinoline-3,4-dione | | | | | |
| 35 | 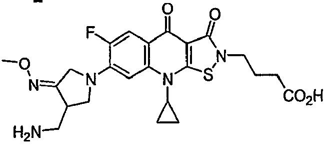 | 4-[7-(3-Aminomethyl-4-methoxyimino-pyrrolidin-1-yl)-9-cyclopropyl-6-fluoro-3,4-dioxo-4,9-dihydro-3H-isothiazolo [5,4-b] quinolin-2-yl]-butyric acid | | | | | |
| 36 | 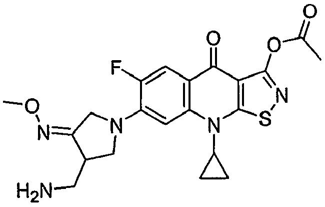 | Acetic acid 7-(3-aminomethyl-4-methoxyimino-pyrrolidin-1-yl)-9-cyclopropyl-6-fluoro-4-oxo-4,9-dihydro-isothiazolo[5,4-b]quinolin-3-yl ester | | | | | |
| 37 | 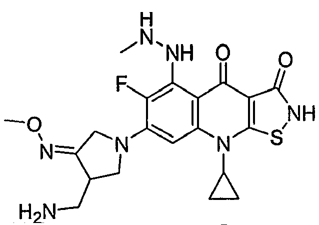 | 7-(3-Aminomethyl-4-methoxyimino-pyrrolidin-1-yl)-9-cyclopropyl-6-fluoro-5-(N'-methyl-hydrazino)-9H-isothiazolo [5,4-b] quinoline- 3,4-dione | | | | | |
| 38 | 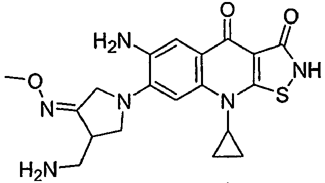 | 6-Amino-7-(3-aminomethyl-4-methoxyimino-pyrrolidin-1-yl)-9-cyclopropyl-9H-isothiazolo[5,4-b]quinoline-3,4-dione | | | | | |
| 39 | 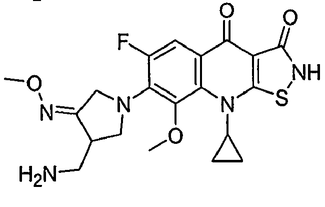 | 7-(3-Aminomethyl-4-methoxyimino-pyrrolidin-1-yl)-9-cyclopropyl-6-fluoro-8-methoxy-9H-isothiazolo[5,4-b]quinoline-3,4-dione | 447.5 | HCl | LCMS *m*/*z* calcd for 3.22-3.33 (4H, m), C₂₀H₂₂FN₅O₄ S ([M]⁺) 447, found M⁺+1 | ¹H-NMR (DMSO-D₆): δ 0.97 (2H, m), 1.18-1.26 (2H, m), 3.51-3.64 (5H, m), 3.72-3.88 (5H, m), 3.95-4.04 (1H, m), 4.17-4.23 (2H, m), 7.42 (1H, d, J = 13.1 Hz) | ¹⁹F (DMSO-D₆): δ -124.1 |
| 40 | 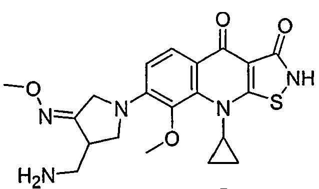 | 7-(3-Aminomethyl-4-methoxyimino-pyrrolidin-1-yl)-9-cyclopropyl-8-methoxy-9H-isothiazolo[5,4- b]quinoline-3,4-dione | | | | | |
| 41 | 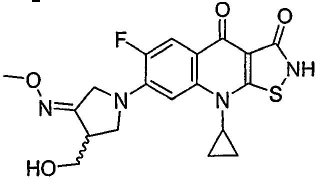 | 9-Cyclopropyl-6-fluoro-7-(3-hydroxymethyl-4-methoxyimino-pyrrolidin-1-yl)-9H-isothiazolo[5,4-b]quinoline-3,4-dione | 418.4 | N/A | LCMS *m*/*z* calcd for C₁₉H₁₉FN₄O₄ S ([M]⁺) 418, found M⁺+1 | ¹H-NMR (DMSO-D₆): δ 1.15 (2H, m), 1.27 (2H, m), 3.08 (1H, m), 3.40-3.50 (3H, m), 3.57-3.68 (3H, m), 3.78 (3H, s), 4.26 (2H, m), 7.04 (1H, d, J = 7.2 Hz), 7.70 (1H,d,J=14.5 Hz), 11.29(1H, brs) | ¹⁹F (DMSO-D₆):δ- 130.7 |
| 42 | 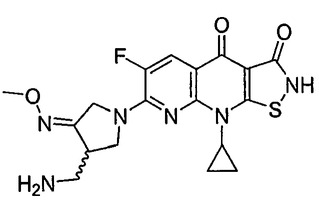 | 7-(3-Aminomethyl-4-methoxyimino-pyrrolidin-1-yl)-9-cyclopropyl-6-fluoro-9H-1-thia-2,8,9-triaza-cyclopenta[b]naphthalene-3,4-dione | 418.4 | HCl | LCMS *m*/*z* calcd for C₁₈H₁₉FN₆O₃ S([M]⁺) 418, found M⁺+1 | ¹H-NMR (DMSO- ₆): δ 1.25 (m, 4H, *c*-Pr CH₂), 3.30 (m, 1H, *c*-Pr CH), 3.44 (m, 2H), 3.54(m, 1H), 3.85 (m, 1H) 3.95 (s, 3H, OCH₃), 4.39 (m, 1H), 4.59 (m, 2H), 7.81 (d, *J*_{H-F} = 13.0 Hz, 1H, H-5) | ¹⁹F (DMSO-D₆): δ- 139.4.1 |
| 43 | 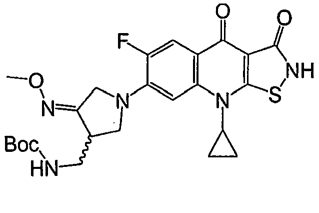 | [1-(9-Cyclopropyl-6-fluoro-3,4-dioxo-2,3,4,9-tetrahydroisothiazolo[5,4-b]quinolin-7-yl)-4-methoxyimino-pyrrolidin-3-ylmethyl]-carbamic acid ter-butyl ester | 517.57 | N/A | LCMS *m*/*z* calcd for C₂₄H₂₈FN₅O₅ S ([M]⁺) 517, found M⁺ +1 | ¹H-NMR (DMSO-D₆): δ 1.73 (2H, m), 1.36 (2H, m), 1.39 (9H, s), 3.11 (2H, bs), 3.31 (2H, m), 3.48 (1H, m), 3.60 (1H,m), 3.80 (1H, m), 3.84 (3H, s), 4.23-4.46 (2H, m), 7.11 (1H, m), 7.70 (1H, m), 8.32 (1H, s) | ¹⁹F (DMSO-D₆): δ -130.7 |

### EXAMPLE 3. ANTIMICROBIAL ACTIVITY OF COMPOUNDS

The antimicrobial activity of the compounds of the invention may be evaluated by a number of methods, including the following visual minimum inhibitory concentration (MIC) assay. This assay determines the minimum concentration of compound required to inhibit growth of a bacterial strain.

### MINIMUM INHIBITORY CONCENTRATION (MIC) ASSAY

Whole-cell antibacterial activity is determined by broth microdilution using conditions recommended by the NCCLS (see National Committee for Clinical Laboratory Standards. 2001. Performance standards for antimicrobial susceptibility testing: 11th informational supplement. Vol. 21, no. 1, M100-S11. National Committee for Clinical Laboratory Standards, Wayne, PA). Test compounds are dissolved in DMSO and diluted 1:50 in Mueller-Hinton II broth (Becton-Dickinson) to produce a 256 µg/ml stock solution. In a 96-well microtiter plate, the compound solution is serially two-fold diluted in Mueller-Hinton II broth. After the compounds are diluted, a 50 µl aliquot of the test organism (∼1 × 10⁶ cfu/mL) is added to each well of the microtiter plate. The final test concentrations ranges from 0.125-128 µg/mL. Inoculated plates are incubated in ambient air at 37°C for 18 to 24 hours. The organisms selected for testing included laboratory strains *S*. *aureus* ATCC 29213 and *E*. *coli* ATCC 25922 (strains purchased from American Type Culture Collection, Manassas, VA). The minimum inhibitory concentration (MIC) is determined as the lowest concentration of compound that inhibited visible growth of the test organism.

Table II below shows the minimum inhibitory concentration required to kill S. *aureus* and *E*. *coli* cells for several compounds of Formula I.

| **TABLE II** | | |
|---|---|---|
| **COMPOUND** | **MIC S. aureus (ug/ml)** | **MIC E. coli (ug/ml)** |
| 13 | <0.05 | <0.05 |
| 27 | <0.05 | <0.05 |
| 32 | <0.05 | <0.05 |
| 39 | <0.05 | 0.125 |
| 41 | <0.05 | <0.10 |
| 42 | <0.10 | <0.125 |

## Claims

1. A compound of Formula I or Formula II 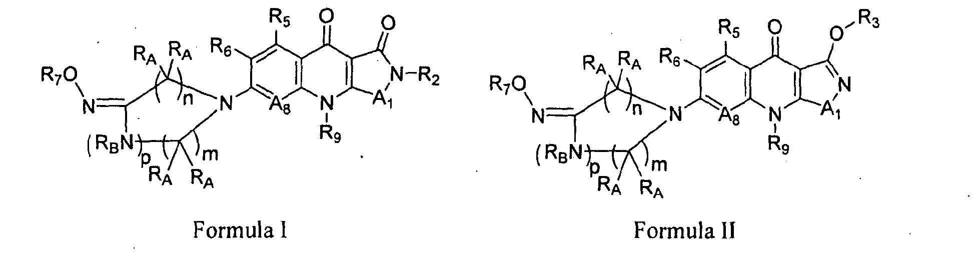 or a pharmaceutically acceptable salt, solvate, tautomer or optical isomer thereof, wherein
A₁ is S, O, SO, or SO₂;
R₂ is hydrogen, or
R₂ is C₁-C₈alkyl, C₂-C₆alkenyl, C₂-C₆alkynyl, (C₃-C₇cycloalkyl)C₀-C₄carbohydryl, (C₄-C₇cycloalkenyl)C₀-C₄carbohydryl, (aryl)C₀-C₄carbohydryl, or (C₂-C₆heterocycloalkyl)C₀-C₄carbohydryl, each of which is substituted with 0 to 5 substituents independently chosen from halogen, hydroxy, amino, cyano, nitro, C₁-C₄alkyl, C₁-C₄alkoxy, C₁-C₂haloalkyl, C₁-C₂haloalkoxy, mono- and di-C₁-C₄alkylamino, C₂-C₄alkanoyl, C₁-C₄alkylthio, -O(C=O)R₁₀, -(C=O)NR₁₀R₁₁, -O(C=O)NR₁₀R₁₁, -(C=O)OR₁₀, -(C=O)NR₁₀OR₁₁, -NR₁₀(C=0)R_{10,} -NR₁₀(C=O)OR₁₁, -NR₁₀(C=O)NR₁₁,R₁₂, -NR₁₀(C=S)NR₁₁R₁₂, -NR₁₀NR₁₁R₁₂, -SO₃R₁₀, -(S=O)OR₁₀, -SO₂R₁₃, -SO₂NR₁₀OR₁₁, =N-OR₁₀ and -NR₁₀SO₂R₁₃; where each R₁₀, R₁₁, and R₁₂ is independently hydrogen, C₁-C₄alkyl, or aryl, and each R₁₃ is independently C₁-C₄alkyl or aryl;
R₃ is hydrogen, C₁-C₆alkyl, C₁-C₆alkanoyl, mono- or di-C₁-C₆alkylcarbamate, C₁-C₆alkylphosphate, or C₁-C₆alkylsulfonate; each of which is substituted with 0 to 3 substituents independently chosen from halogen, hydroxy, amino, cyano, nitro, C₁-C₄alkoxy, mono- and di-C₁-C₄alkylamino, C₁-C₂haloalkyl, and C₁-C₂haloalkoxy;
R₅ is hydrogen, halogen, hydroxy, amino, cyano, nitro, -NHNH₂, C₁-C₂haloalkyl, or C₁-C₂haloalkoxy; or
R₅ is C₁-C₄alkyl, C₁-C₄alkoxy, mono- or di-C₁-C₄alkylamino, mono-, di-, or tri-C₁-C₄ alkylhydrazinyl, C₂-C₄alkanoyl, or C₁-C₄alkylester; each of which is substituted with 0 to 3 substituents independently chosen from hydroxy, amino, halogen, oxo, C₁-C₄alkoxy, C₁-C₂haloalkyl, C₁-C₂haloalkoxy, and mono- and di-C₁-C₄alkylamino;
R₆ is hydrogen, halogen, hydroxy, amino, cyano,C₁-C₄alkyl, C₁-C₄alkoxy, mono- or di-C₁-C₄alkylamino, -SO₃R₁₀, -SO₂R₁₀, -SO₂NR₁₀R₁₁;
n is 1, 2, or 3; m is 1, 2, or 3; p is 0 or 1;
each R_{A} is independently (i), (ii), or (iii); where
(i) is hydrogen, hydroxy, amino, or cyano,
(ii) is C₁-C₆alkyl, C₂-C₆alkenyl, C₂-C₆alkynyl, C₁-C₆alkoxyC₀-C₄alkyl, mono- or di-C₁-C₄alkylamino, C₁-C₂haloalkoxy, (C₃-C₇cycloalkyl)C₀-C₄carbohydryl, (C₄-C₇cycloalkenyl)C₀-C₄carbohydryl, (aryl)C₀-C₆carbohydryl, (aryl)C₁-C₄alkoxy, (C₂-C₆heterocycloalkyl)C₀-C₄carbohydryl, (heteroaryl)C₀-C₆carbohydryl, C₁-C₆alkylthio, - (C₀-C₄alkyl)O(C=O)R₁₀, -(C₀-C₄alkyl)(C=O)NR₁₀R₁₁, -(C₀-C₄alkyl)O(C=O)NR₁₀R₁₁, - (C₀-C₄alkyl) (C=O)OR₁₀, -(C₀-C₄alkyl)NR₁₀(C=O)R₁₁, -(C₀-C₄alkyl)NR₁₀(C=O)OR₁₁,(C₀-C₄alkyl)NR₁₀(C=O)NR₁₁R₁₂, -(C₀-C₄alkyl)NR₁₀(C=S)NR₁₁R₁₂, -(C₀-C₄alkyl)NR₁₀NR₁₁R₁₂, -(C₀-C₄alkyl)N=NR₁₃, -(C₀-C₄alkyl)SO₃R₁₀, -(Co-C₄alkyl)(S=O)OR₁₀, -(C₀-C₄alkyl)SO₂R₁₃, -(C₀-C₄alkyl)SO₂NR₁₀R₁₁, -(C₀-C₄alkyl)NR₁₀SO₂R₁₃; =N-OH, or =N-O(C₀-C₄alkyl);
(iii) is -OR_{D}, -(C=O)R_{D}, -SO₂R_{D}, -SO₃R_{D}, or -NR₁₀SO₂R_{D}, where R_{D} is C₁-C₄alkyl, (C₃-C₇cycloalkyl)C₀-C₂alkyl, (C₂-C₆heterocycloalkyl)C₀-C₂alkyl, (aryl)C₀-C₂alky), or (heteroaryl)C₀-C₂alkyl;
where each of (ii) and (iii) is substituted with 0 to 3 substituents independently chosen from halogen, hydroxy, amino, cyano, nitro, -COOH, -(C=O)OCH₃,-CONH₂, C₁-C₄alkyl, C₂-C₄alkenyl, C₂-C₄alkynyl, C₁-C₄alkoxy, (C₃-C₇cycloalkyl)C₀-C₄carbohydryl, (C₃-C₇cycloalkyl)C₀-C₄alkoxy, mono- and di-C₁-C₄alkylamino, C₁-C₂haloalkyl, C₁-C₂haloalkoxy, and C₂-C₄alkanoyl; or
any two R_{A} bound to the same carbon atom may be joined to form a C₃-C₇cycloalkyl, or a 3- to 7- membered heterocycloalkyl group; having 1 or 2 heteroatoms independently chosen from N, O, and S; each of which is substituted with 0 to 2 substituents independently chosen from C₁-C₂alkyl, and C₁-C₂alkoxy; or
any two R_{A} bound to different carbon atoms may be joined to form a C₃-C₇cycloalkyl or a 3- to 7-membered heterocycloalkyl group having 1 or 2 heteroatoms independently chosen from N, O, and S; each of which is substituted with 0 to 2 substituents independently chosen from C₁-C₂alkyl, and C₁-C₂alkoxy;
R_{B} is hydrogen or C₁-C₄alkyl;
R₇ is hydrogen, or
R₇ is C₁-C₈alkyl, C₂-C₆alkenyl, C₂-C₆alkynyl, C₂-C₆alkanoy, (C₃-C₇cycloalkyl)C₀-C₄carbohydryl, (C₄-C₇cycloalkenyl)C₀-C₄carbohydryl, (aryl)C₀-C₄carbohydryl, (aryl)(C=O)-, mono- or di-(C₁-C₆alkyl)carboxamide, C₁-C₆alkylester, or (C₂-C₆heterocycloalkyl)C₀-C₄carbohydryl, each of which is substituted with 0 to 5 substituents independently chosen from halogen, hydroxy, amino, cyano, nitro, C₁-C₄alkyl, C₁-C₄alkoxy, C₁-C₂haloalkyl, C₁-C₂haloalkoxy, mono- and di-C₁-C₄alkylamino, C₂-C₄alkanoyl, C₁-C₄alkylthio, -O(C=O)R₁₀, -(C=O)NR₁₀R₁₁, -O(C=O)NR₁₀R₁₁, -(C=O)OR₁₀, -(C=O)NR₁₀OR₁₁, -NR₁₀(C=O)R₁₁, -NR₁₀(C=O)OR₁₁, -NR₁₀(C=O)NR₁₁R₁₂, -NR₁₀(C=S)NR₁₁R₁₂, -NR₁₀NR₁₁R₁₂, -SO₃R₁₀, -(S=O)OR₁₀, -SO₂R₁₃, -SO₂NR₁₀R₁₁, or -NR₁₀SO₂R₁₃
A₈ is nitrogen or CR₈; where
R₈ is hydrogen, halogen, hydroxy, amino, cyano, nitro, or -NHNH₂, or
R₈ is C₁-C₄alkyl, C₁-C₄alkoxy, mono- or di-C₁-C₄alkylamino, mono-, di-, or tri-C₁-C₄ alkylhydrazinyl, C₂-C₄alkanoyl, C₁-C₄alkylester, C₁-C₂haloalkyl, or C₁-C₂haloalkoxy, each of which is substituted with 0 to 3 substituents independently chosen from hydroxy, amino, halogen, oxo, C₁-C₄alkoxy, C₁-C₂haloalkyl, C₁-C₂haloalkoxy, and mono- and di-C₁-C₄alkylamino; and
R₉ is C₁-C₈alkyl, (C₃-C₇cycloalkyl)C₀-C₄alkyl, or phenyl, each of which is substituted with 0 to 3 substituents independently chosen from halogen, hydroxy, amino, cyano, nitro, -COOH, -CONH₂, C₁-C₄alkyl, C₂-C₄alkenyl, C₂-C₄alkynyl, C₁-C₄alkoxy, (C₃-C₇cycloalkyl)C₀-C₄alkyl, (C₃-C₇cycloalkyl)C₀-C₄alkoxy, mono- and di-C₁-C₄alkylamino, C₁-C₂haloalkyl, C₁-C₂haloalkoxy, and C₂-C₄alkanoyl.

2. A compound or salt of Claim 1 of the formula (a) 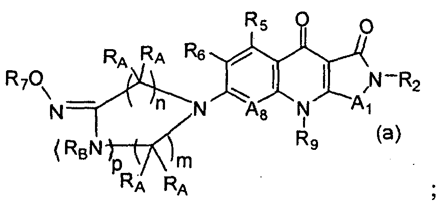 or of the formula (b)

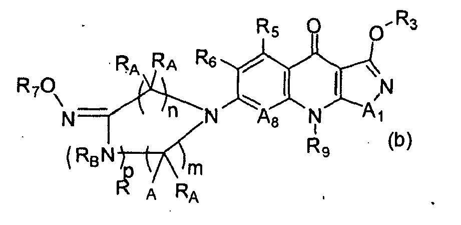

3. A compound or salt of any one of Claims 1 or 2, wherein A₁ is S, SO, SO₂, or O.

4. A compound or salt of Claim 2 or 3, wherein the compound or salt is of formula (a), and R₂ is hydrogen, or
R₂ is C₁-C₆alkyl or (C₃-C₇cycloalkyl)C₀-C₄alkyl, each of which is substituted with at least one substituent chosen from hydroxy, amino, -COOH, -(C=O)NR₁₀OR₁₁, and -CONH₂;
and is substituted with 0 to 3 substituents independently chosen from halogen, hydroxy, amino, cyano, nitro, -COOH, -CONH₂, C₁-C₄alkyl, C₁-C₄alkoxy, C₁-C₂haloalkyl, C₁-C₂haloalkoxy, and mono- and di-C₁-C₄alkylamino, and C₂-C₄alkanoyl.

5. A compound or salt of Claim 4 wherein R₂ is hydrogen.

6. A compound or salt of any one of Claims 2 to 3 wherein the compound is a compound of formula (b) and wherein R₃ is C₁-C₄alkyl or C₂-C₄alkanoyl.

7. A compound or salt of any one of Claims I to 6 wherein
R₅ is hydrogen, amino, C₁-C₂alkyl, C₁-C₂alkoxy, mono- or di-C₁-C₄alkylamino, or mono- or di-C₁-C₄ alkylhydrazinyl.

8. A compound or salt of Claim 7 wherein
R₅ is hydrogen, amino, mono- or di-C₁-C₂alkylamino, or mono- or di-C₁-C₂ alkylhydrazinyl.

9. A compound or salt of Claim 7 wherein R₅ is hydrogen.

10. A compound or salt of any one of Claims I to 8 wherein: R₆ is hydrogen, halogen, or amino.

11. A compound or salt of Claim 10 wherein R₆ is fluoro.

12. A compound or salt of any one of Claims 1 to 11 wherein A₈ is nitrogen or CR₈.

13. A compound or salt of Claim 12 in which A₈ is CR₈ and wherein R₈ is hydrogen, halogen, C₁-C₂alkyl, C₁-C₂alkoxy, C₁-C₂haloalkyl, or C₁-C₂haloalkoxy.

14. A compound or salt of Claim 13 in which R₈ is hydrogen or methoxy.

15. A compound or salt of any one of Claims 1 to 14 wherein
R₉ is C₁-C₄alkyl, cyclopropyl, or phenyl, each of which is substituted with 0 to 3 substituents independently chosen from halogen, hydroxy, amino, C₁-C₂alkyl, C₁-C₂alkoxy, mono- and di-C₁-C₂alkylamino, C₁-C₂haloalkyl, and C₁-C₂haloalkoxy.

16. A compound or salt of Claim 15 wherein
R₉ is C₁-C₄alkyl or cyclopropyl, or
R₉ is phenyl substituted with 2 substituents chosen from halogen, hydroxy, amino, C₁-C₂alkyl, C₁-C₂alkoxy, mono- and di-C₁-C₂alkylamino, C₁-C₂haloalkyl, and C₁-C₂alkoxy.

17. A compound or salt of Claim 15 wherein
R₉ is ethyl, t-butyl, cyclopropyl, or 2,4-difluorophenyl.

18. A compound or salt of Claim 15, wherein R₉ is cyclopropyl.

19. A compound or salt of any one of Claims 1 to 18 wherein
R₇ is hydrogen or
R₇ is C₁-C₈alkyl, C₂-C₆alkanoyl, (C₃-C₇cycloalkyl)C₀-C₄carbohydryl, (aryl)C₀-C₄carbohydryl, (aryl)(C=O)-, mono- or di-(C₁-C₆alkyl)carboxamide, C₁-C₆alkylester,or (C₂-C₆heterocycloalkyl)C₀-C₄carbohydryl, each of which is substituted with 0 to 5 substituents independently chosen from halogen, hydroxy, amino, cyano, nitro, C₁-C₄alkyl, C₁-C₄alkoxy, C₁-C₂haloalkyl, C₁-C₂haloalkoxy, mono- and di-C₁-C₄alkylamino, C₂-C₄alkanoyl, C₁-C₄alkylthio, -O(C=O)R₁₀, -(C=O)NR₁₀R₁₁, -O(C=O)NR₁₀R₁₁, -(C=O)OR₁₀, -(C=O)NR₁₀OR₁₁, -NR₁₀(C=O)R₁₁, -NR₁₀(C=O)OR₁₁, -NR₁₀(C=O)NR₁₁R₁₂, -NR₁₀(C=S)NR₁₁R₁₂, -NR₁₀NR₁₁R₁₂, -SO₃R₁₀, -(S=O)OR₁₀, -SO₂R₁₃, -SO₂NR₁₀R₁₁, or -NR₁₀SO₂R₁₃.

20. A compound or salt of Claim 19 wherein
R₇ is hydrogen, or
R₇ is C₁-C₈alkyl, (C₃-C₇cycloalkyl)C₀-C₄carbohydryl, (aryl)C₀-C₄carbohydryl, or (C₂-C₆heterocycloalkyl)C₀-C₄carbohydryl, each of which is substituted with 0 to 5 substituents independently chosen from halogen, hydroxy, amino, cyano, C₁-C₄alkyl, C₁-C₄alkoxy, C₁-C₂haloalkyl, C₁-C₂haloalkoxy, mono- and di-C₁-C₄alkylamino, C₂-C₄alkanoyl , -(C=O)NR₁₀R₁₁, -NR₁₀(C=O)R₁₁.

21. A compound or salt of Claim 20 wherein
R₇ is hydrogen, or
C₁-C₈alkyl, or (aryl)C₀-C₄alkyl, each of which is substituted with 0 to 5 substituents independently chosen from halogen, hydroxy, amino, cyano, C₁-C₄alkyl, C₁-C₄alkoxy, C₁-C₂haloalkyl, C₁-C₂haloalkoxy, mono- and di-C₁-C₄alkylamino, C₂-C₄alkanoyl, -(C=O)NR₁₀R₁₁, -NR₁₀(C=O)R₁₁.

22. A compound or salt of Claim 20 wherein R₇ is hydrogen, C₁-C₂alkyl, or benzyl.

23. A compound or salt of any one of Claims 1 to 22 wherein n is 1; m is 2, p is 1 and R_{B} is hydrogen.

24. A compound or slat of any one of Claims 1 to 22 wherein p is 0.

25. A compound or salt of Claim 24 wherein n is 1 and m is 2 or wherein n is 2 and m is 2.

26. A compound or salt of any one of Claims 1 to 25 wherein
each R_{A} is independently (i), (ii), or (iii); where
(i) is hydrogen, hydroxy, amino, or cyano,
(ii) is C₁-C₆alkyl, C₂-C₆alkenyl, C₂-C₆alkynyl, (C₁-C₆alkoxy)C₀-C₄alkyl, mono- or di-C₁-C₄alkylamino, C₁-C₂haloalkoxy, (C₃-C₇cycloalkyl)C₀-C₄carbohydryl, (C₄-C₇cydoalkenyl)C₀-C₄carbohydryl, (aryl)C₀-C₆carbohydryl, (aryl)C₁-C₄alkoxy, (C₂-C₆heterocycloalkyl)C₀-C₄carbohydryl, (heteroaryl)C₀-C₆carbohydryl, C₁-C₆alkylthio, (C₀-C₄alkyl)(C=O)NR₁₀R₁₁, -(C₀-C₄alkyl)NR₁₀(C=O)R₁₁, =N-OH, or =N-O(C₀-C₄alkyl);
(iii) is -OR_{D} or -(C=O)R_{D}, where R_{D} is C₁-C₄alkyl, (C₃-C₇cycloalkyl)C₀-C₂alkyl, (C₂-F₆heterocycloalkyl)C₀-C₂alkyl, (aryl)C₀-C₂alkyl, or (heteroatyl)C₀-C₂alkyl;
where each of (ii) and (iii) is substituted with 0 to 3 substituents independently chosen from halogen, hydroxy, amino, cyano, nitro, -COOH, -(C=O)OCH₃, - CONH₂, C₁-C₄alkyl, C₂-C₄alkenyl, C₂-C₄alkynyl, C₁-C₄alkoxy, (C₃-C₇cycloalkyl)C₀-C₄carbohydryl, (C₃-C₇cycloalkyl)C₀-C₄alkoxy, mono- and di-C₁-C₄alkylamino, C₁-C₂haloalkyl, C₁-C₂haloalkoxy, and C₂-C₄alkanoyl; or
any two R_{A} bound to the same carbon atom may be joined to form a C₃-C₇cycloalkyl or a 3- to 7-membered heterocycloalkyl group having 1 or 2 heteroatoms independently chosen from N, O, and S; each of which is substituted with 0 to 2 substituents independently chosen from C₁-C₂alkyl, and C₁-C₂alkoxy; or
any two R_{A} bound to different carbon atoms may be joined to form a C₃-C₇cycloalkyl or a 3- to 7-membered heterocycloalkyl group having 1 or 2 heteroatoms independently chosen from N, O, and S; each of which is substituted with 0 to 2 substituents independently chosen from C₁-C₂alkyl and C₁-C₂alkoxy.

27. A compound or salt of Claim 26 wherein
each R_{A} is independently (i) or (ii); where
(i) is hydrogen or amino,
(ii) is C₁-C₆alkyl, (C₁-C₆alkoxy)C₀-C₄alkyl, mono- or di-C₁-C₄alkylamino, (C₃-C₇cycloalkyl)C₀-C₄carbohydryl, (aryl)C₀-C₆carbohydryl, (C₂-C₆heterocycloakyl)C₀-C₄carbohydryl, (heteroaryl)C₀-C₆carbohydryl, -(C₀-C₄alkyl)(C=O)NR₁₀R₁₁, -(Co-C₄alkyl)NR₁₀(C=O)R₁₁, =N-OH, or =N-O(C₀-C₄alkyl);
where each of (ii) is substituted with 0 to 3 substituents independently chosen from halogen, hydroxy, amino, cyano, nitro, -COOH, -(C=O)OCH₃, -CONH₂, C₁-C₄alkyl, C₂-C₄alkenyl, C₂-C₄alkynyl, C₁-C₄alkoxy, (C₃-C₇cycloalkyl)C₀-C₄carbohydryl, (C₃-C₇cycloalkyl)C₀-C₄alkoxy, mono- and di-C₁-C₄alkylamino, C₁-C₂haloalkyl, C₁-C₂haloalkoxy, and C₂-C₄alkanoyl; or
any two R_{A} bound to the same carbon atom may be joined to form a C₃-C₇cycloalkyl or a 3- to 7-membered heterocycloalkyl group having I or 2 heteroatoms independently chosen from N, O, and S; each of which is substituted with 0 to 2 substituents independently chosen from C₁-C₂alkyl, and C₁-C₂alkoxy; or
any two R_{A} bound to different carbon atoms may be joined to form a C₃-C₇cycloalkyl or heterocycloalkyl group having 1 or 2 heteroatoms independently chosen from N, O, and S; each of which is substituted with 0 to 2 substituents independently chosen from C₁-C₂alkyl, and C₁-C₂alkoxy.

28. A compound or salt of Claim 26 wherein
each R_{A} is independently (i) or (ii); where
(i) is hydrogen or amino;
(ii) is C₁-C₆alkyl, mono- or di-C₁-C₄alkylamino, (C₃-C₇cycloalkyl)C₀-C₄alkyl, (C₂-C₆heterocycloalkyl)C₀-C₄alkyl, -(C₀-C₄alkyl)(C=O)NR₁₀R₁₁, -(C₀-C₄alkyl)NR₁₀(C=O)R₁₁, =N-OH, or =N-O(C₀-C₄alkyl);
where each of (ii) is substituted with 0 to 3 substituents independently chosen from hydroxy, amino, cyano, and -CONH₂.
any two R_{A} bound to the same carbon atom may be joined to form a C₃-C₇cycloalkyl or heterocycloalkyl group having 1 or 2 heteroatoms independently chosen from N, O, and S; each of which is substituted with 0 to 2 substituents independently chosen from C₁-C₂alkyl and C₁-C₂alkoxy.

29. A compound or salt of Claim 26 wherein
each R_{A} is independently (i) or (ii); where
(i) is hydrogen or amino,
(ii) is C₁-C₆alkyl, (C₃-C₇cycloalkyl)C₀-C₂alkyl, or mono- or di-C₁-C₄alkylamino,
where each of (ii) is substituted with 0 to 3 amino,
any two R_{A} bound to the same carbon atom may be joined to form a C₃-C₇cycloalkyl or a 3- to 7-membered heterocycloalkyl group having 1 or 2 heteroatoms independently chosen from N, O, and S; each of which is substituted with 0 to 2 substituents independently chosen from C₁-C₂alkyl and C₁-C₂alkoxy; or
any two R_{A} bound to different carbon atoms may be joined to form a C₃-C₇cycloalkyl or a membered heterocycloalkyl group having 1 or 2 heteroatoms independently chosen from N, O, and S; each of which is substituted with 0 to 2 substituents independently chosen from C₁-C₂alkyl and C₁-C₂alkoxy.

30. A compound or salt of Claim 26 wherein
each R_{A} is independently hydrogen, C₁-C₄alkyl, or C₁-C₄alkyl substituted with one amino substituent.

31. A compound or salt of Claim 30, wherein: one or two R_{A} is C₁-C₄alkyl substituted with one amino substituent and the remaining R_{A} are hydrogen.

32. A compound or salt of Claim 26 wherein
two R_{A} bound to the same carbon atom are joined to form a C₃-C₇cycloalkyl or a 3- to 7-membered heterocycloalkyl group having 1 or 2 heteroatoms independently chosen from N, O, and S; each of which is substituted with 0 to 2 substituents independently chosen from C₁-C₂alkyl and C₁-C₂alkoxy; and
the remaining R_{A} are independently chosen from hydrogen, C₁-C₂alkyl and C₁C₂alkoxy.

33. A compound or salt of Claim 32,
two R_{A} bound to the same carbon atom are joined to form a C₃-C₄cycloalkyl or a 3- to 4-membered heterocycloalkyl group having I N atom; each of which is substituted with 0 to 2 substituents independently chosen from C₁-C₂alkyl and C₁-C₂alkoxy; and
the remaining R_{A} are independently chosen from hydrogen, C₁-C₂alkyl and C₁-C₂alkoxy.

34. A compound or salt of formula (a) of Claim 2 wherein:
R₂ is hydrogen or C₁-C₆alkyl substituted with one substituent chosen from hydroxy, amino, -COOH, -(C=O)NR₁₀OR₁₁, and -CONH₂;
R₅ is hydrogen;
R₆ is halogen or amino;
R₇ is hydrogen, C₁-C₂alkyl, or benzyl;
A₈ is nitrogen or CR₈, and when A₈ is CR₈, R₈ is hydrogen or methoxy
R₉ is ethyl, t-butyl, cyclopropyl, or 2,4-difluorophenyl; and
Each R_{A} is independently hydrogen or C₁-C₄alkyl optionally substituted with one amino substituent; or
any two R_{A} bound to the same carbon atom may be joined to form a C₃-C₇cycloalkyl or a 3- to 7-membered heterocycloalkyl group having I or 2 heteroatoms independently chosen from N, O, and S; each of which is substituted with 0 to 2 substituents independently chosen from C₁-C₂alkyl, and C₁-C₂alkoxy; or
any two R_{A} bound to different carbon atoms may be joined to form a C₃-C₇cycloalkyl or heterocycloalkyl group having I or 2 heteroatoms independently chosen from N, O, and S; each of which is substituted with 0 to 2 substituents independently chosen from C₁-C₂alkyl, and C₁-C₂alkoxy.

35. A compound or salt of Claim 1 wherein the compound is
7-[(4Z)-3-Aminomethyl-4-methoxyimino-pyrrolidin-1-yl)]-9-cyclopropyl-6-fluoro-9H-isothiazolo[5,4-b]quinoline-3,4-dione;
7-[(3R,4Z)-3-Aminomethyl-4-methoxyimino-pyrrolidin-1-yl)]-9-cyclopropyl-6-fluoro-9H-isothiazolo[5,4-b]quinoline-3,4-dione;
7-[(3S,4Z)-3-Aminomethyl-4-methoxyimino-pyrrolidin-1-yl)]-9-cyclopropyl-6-fluoro-9H-isothiazolo[5,4-b]quinoline-3,4-dione;
7-[(4E)-3-Aminomethyl-4-methoxyimino-pyrrolidin-1-yl)]-9-cyclopropyl-6-fluoro-9H-isothiazolo[5,4-b]quinoline-3,4-dione;
7-[(3R,4E)-3-Aminomethyl-4-methoxyimino-pyrrolidin-1-yl)]-9-cyclopropyl-6-fluoro-9H-isothiazolo[5,4-b]quinoline-3,4-dione;
7-[(3S,4E)-3-Aminomethyl-4-methoxyimino-pyrrolidin-1-yl)]-9-cyclopropyl-6-fluoro-9H-isothiazolo[5,4-b]quinoline-3,4-dione;
9-Cyclopropyl-6-fluoro-7-(8-methoxyimino-2,6-diaza-spiro[3.4]oct-6-yl)-9H-isothiazolo[5,4-b]quinoline-3,4-dione;
9-Cyclopropyl-6-fluoro-7-(7-methoxyimino-1,5-diaza-spiro[2.4]hept-5-yl)-9H-isothiazolo[5,4-b]quinoline-3,4-dione;
7-[3-(1-Amino-cyclopropyl)-4-methoxyimino-pyrrolidin-1-yl]-9-cyclopropyl-6-fluoro-9H-isothiazolo[5,4-b]quinoline-3,4-dione;
9-Cyclopropyl-6-fluoro-7-(7-methoxyimino-5-aza-spiro[2.4]hept-5-yl)-9H-isothiazolo[5,4-b]quinoline-3,4-dione;
7-(3-Aminomethyl-4-hydroxyimino-pyrrolidin-1-yl)-9-cyclopropyl-6-fluoro-9H-isothiazolo[5,4-b]quinoline-3,4-dione;
7-[3-(2-Amino-ethyl)-4-methoxyimino-pyrrolidin-1-yl]-9-cyclopropyl-6-fluoro-9H-isothiazolo[5,4-b]quinoline-3,4-dione;
7-(3-Aminomethyl-4-benzyloxyimino-pyrrolidin-1-yl)-9-cyclopropyl-6-fluoro-9H-isothiazolo[5,4-b]quinoline-3,4-dione;
7-(3-Amino-4-methoxyimino-pyrrolidin-1-yl)-9-cyclopropyl-6-fluoro-9H-isothiazolo[5,4-b]quinoline-3,4-dione;
7-(3-Aminomethyl-4-methoxyimino-3-methyl-pyrrolidin-1-yl)-9-cyclopropyl-6-fluoro-9H-isothiazolo[5,4-b]quinoline-3,4-dione;
7-[3-(1-Amino-ethyl)-4-methoxyimino-pyrrolidin-1-yl]-9-cyclopropyl-6-fluoro-9H-isothiazolo[5,4-b]quinoline-3,4-dione;
7-(3,3-Bis-aminomethyl-4-methoxyimino-pyrrolidin-1-yl)-9-cyclopropyl-6-fluoro-9H-isothiazolo[5,4-b]quinoline-3,4-dione;
7-[3-(1-Amino-1-methyl-ethyl)-4-methoxyimino-pyrrolidin-1-yl]-9-cyclopropyl-6-fluoro-9H-isothiazolo[5,4-b]quinoline-3,4-dione;
9-Cyclopropyl-6-fluoro-7-(3-methoxyimino-piperazin-1-yl)-9H-isothiazolo[5,4-b]quinoline-3,4-dione;
9-Cyclopropyl-6-fluoro-7-(4-methoxyimino-piperidin-1-yl)-9H-isothiazolo[5,4-b]quinoline-3,4-dione;
7-(3-Aminomethyl-4-methoxyimino-piperidin-1-yl)-9-cyclopropyl-6-fluoro-9H-isothiazolo[5,4-b]quinol ine-3,4-dione;
7-(3-Aminomethyl-4-methoxyimino-pyrrolidin-1-yl)-9-cyclopropyl-6-fluoro-1,1-dioxo-1,2-dihydro-9H-116-isothiazolo[5,4-b]quinoline-3,4-dione;
4-[7-(3-Aminomethyl-4-methoxyimino-pyrrolidin-1-yl)-9-cyclopropyl-6-fluoro-3,4-dioxo-4,9-dihydro-3H-isothiazolo[5,4-b]quinolin-2-yl]-butyric acid;
Acetic acid 7-(3-aminomethyl-4-methoxyimino-pyrrolidin-1-yl)-9-cyclopropyl-6-fluoro-4-oxo-4,9-dihydro-isothiazolo[5,4-b]quinolin-3-yl ester;
7-(3-Aminomethyl-4-methoxyimino-pyrrolidin-1-yl)-9-cyclopropyl-6-fluoro-5-(N'-methyl-hydrazino)-9H-isothiazolo[5,4-b]quinoline-3,4-dione;
6-Amino-7-(3-aminomethyl-4-methoxyimino-pyrrolidin-1-yl)-9-cyclopropyl-9H-isothiazolo[5,4-b]quinoline-3,4-dione;
7-(3-Aminomethyl-4-methoxyimino-pyrrolidin-1-yl)-9-cyclopropyl-6-fluoro-8-methoxy-9H-isothiazolo[5,4-b]quinoline-3,4-dione; and
7-(3-Aminomethyl-4-methoxyimino-pyrrolidin-1-yl)-9-cyclopropyl-8-methoxy-9H-isothiazolo[5,4-b]quinoline-3,4-dione.

36. An anti-bacterial composition comprising a compound or salt of any one of Claims I to 35, together with a carrier, diluent, or excipient.

37. A pharmaceutical composition comprising a compound or salt of any one of Claims I to 35, together with a pharmaceutically acceptable carrier, diluent, or excipient.

38. A pharmaceutical composition of Claim 36, wherein the composition is formulated as an injectable fluid, an aerosol, a cream, a gel, a pill, a capsule, a tablet, a syrup, a transdermal patch, or an ophthalmic solution.

39. A composition comprising a compound or salt of any one of Claim 1 to 35 in combination with another one ore more antibacterial agent, antiprotozoal agent, antifungal agent, antiviral agent, interferon, efflux-pump inhibitor, or beta-lactamase inhibitor.

40. A package comprising the pharmaceutical composition of Claim 39 in a container and further comprising instruction for using the composition to treat a patient suffering from microorganism infection.

41. A package of Claim 40 wherein the instruction are instruction for using the composition to treat a patient suffering from a bacterial infection.

42. The compound or salt of any one of Claim 1 to 35 for use in a method for treating or preventing a bacterial or protozoal infection.

43. The compound of Claim 42 wherein the bacterial or protozoal infection is a urinary tract infection, pyelonephritis, lower respiratory tract infection, skin infection, skin-structure infection, urethral gonococcal infection, cervical gonococcal infection, urethral chlamydial infection, cervical chlamydial infection, bone infection, joint infection, gram-negative bacterial infection, infectious diarrhea, typhoid fever, prostatitis, acute sinusitis, acute exacerbation of chronic bronchitis, pneumonia, intra-abdominal infection, gynecologic infection, or pelvic infection.

44. The compound of Claim 42 wherein the animal is a fish, amphibian, reptile, bird, or mammal.

45. The compound of Claim 44 wherein the animal is a mammal.

46. The compound of Claim 45 wherein the mammal is a human.

47. A compound or salt of any one of Claim 1 to 35 that exhibits an MIC of 10 µg/ ml or less against *S*. *aureus* or *E. coli.*

48. A compound or salt of any one of Claim 1 to 35 that exhibits an MIC of I µg/ ml or less against *S*. *aureus* or *E. coli.*

49. The use of a compound of any one of Claims 1 to 35 for the manufacture of a medicament for the treatment of a bacterial or protozoal infection.

50. The use of Claim 49 wherein the bacterial or protozoal infection is a urinary tract infection, pyelonephritis, lower respiratory tract infection, skin infection, skin-structure infection, urethral gonococcal infection, cervical gonococcal infection, urethral chlamydial infection, cervical chlamydial infection, bone infection, joint infection, gram-negative bacterial infection, infectious diarrhea, typhoid fever, prostatitis, acute sinusitis, acute exacerbation of chronic bronchitis, pneumonia, intra-abdominal infection, gynecologic infection, or pelvic infection.

51. The use of Claim 49 wherein the bacteria infection is an S. aureaus or E. coli infection.

## Patentansprüche

1. Verbindung der Formel I oder Formel II 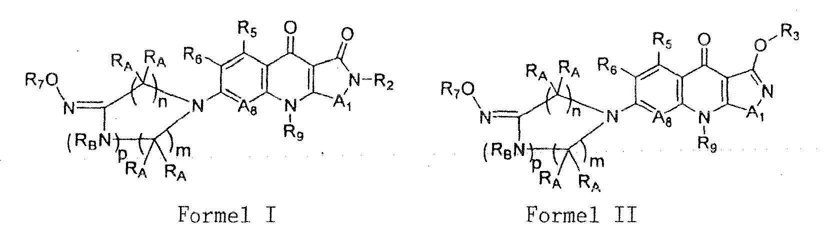 oder ihr pharmazeutisch zulässiges Salz, Solvat, Tautomer oder optisches Isomer, worin
A₁ S, 0, SO oder SO₂ ist,
R₂ Wasserstoff ist oder R₂ C₁-C₈-Alkyl, C₂-C₆-Alkenyl, C₂-C₆-Alkynyl, (C₃-C₇-Cycloalkyl)-C₀-C₄-carbohydryl, (C₄-C₇-Cycloalkenyl)-C₀-C₄-carbohydryl, (Aryl)-C₀-C₄-carbohydryl oder (C₂-C₆-Heterocycloalkyl)-C₀-C₄-carbohydryl ist, von denen jedes mit 0 bis 5 Substituenten substituiert ist, die unabhängig ausgewählt sind unter Halogen, Hydroxy, Amino, Cyano, Nitro, C₁-C₄-Alkyl, C₁-C₄-Alkoxy, C₁-C₂-Haloalkyl, C₁-C₂-Haloalkoxy, Mono- und Di-C₁-C₄-alkylamino, C₂-C₄-Alkanoyl, C₁-C₄-Alkylthio, -O(C=O)R₁₀, -(C=O)NR₁₀R₁₁, -O(C=O)NR₁₀R₁₁, -(C=O)OR₁₀, -(C=O)NR₁₀R₁₁, -NR₁₀(C=O)R₁₁, -NR₁₀(C=O)OR₁₁, -NR₁₀(C=O)NR₁₁R₁₂, -NP₁₀(C=S)NR₁₁P₁₂, -NR₁₀NR₁₁R₁₂, -SO₃R₁₀, -(S=O)OR₁₀, -SO₂R₁₃, -SO₂NR₁₀R₁₁, =N-OR₁₀ und -NR₁₀SO₂R₁₃, worin jedes R₁₀, R₁₁ und R₁₂ unabhängig Wasserstoff, C₁-C₄-Alkyl oder Aryl ist und jedes R₁₃ unabhängig C₁-C₄-Alkyl oder Aryl ist,
R₃ Wasserstoff, C₁-C₆-Alkyl, C₁-C₆-Alkanoyl, Mono- oder Di-C₁-C₆-alkyl-carbamat, C₁-C₆-Alkylphosphat oder C₁-C₆-Alkylsulfonat ist, von denen jedes mit 0 bis 3 Substituenten substituiert ist, die unabhängig unter Halogen, Hydroxy, Amino, Cyano, Nitro, C₁-C₄-Alkoxy, Mono- und Di-C₁-C₄-alkylamino, C₁-C₂-Haloalkyl und C₁-C₂-Haloalkoxy ausgewählt sind
R₅ Wasserstoff, Halogen, Hydroxy, Amino, Cyano, Nitro, -NHNH₂, C₁-C₂-Haloalkyl oder C₁-C₂-Haloalkoxy ist oder
R₅ C₁-C₄-Alkyl, C₁-C₄-Alkoxy, Mono- oder Di-C₁-C₄-alkylamino, Mono-, Di- oder Tri-C₁-C₄-alkylhydrazinyl, C₂-C₄-Alkanoyl oder C₁-C₄Alkylester ist, von denen jedes mit 0 bis 3 Substituenten substituiert ist, die unabhängig unter Hydroxy, Amino, Halogen, Oxo, C₁-C₄-Alkoxy, C₁-C₂-Haloalkyl, C₁-C₂-Haloalkoxy und Mono- und Di-C₁-C₄-Alkylamino ausgewählt sind,
R₆ Wasserstoff, Halogen, Hydroxy, Amino, Cyano, C₁-C₄-Alkyl, C₁-C₄-Alkoxy, Mono- oder Di-C₁-C₄-alkylamino, -SO₃R_{10,} -SO₂R₁₀, -SO₂NR₁₀R₁₁ ist,
n 1, 2 oder 3 ist, m 1, 2 oder 3 ist, p 0 oder 1 ist,
jedes R_{A} unabhängig (i), (ii) oder (iii) ist, wobei
(i) Wasserstoff, Hydroxy, Amino oder Cyano ist,
(ii) C₁-C₆-Alkyl, C₂-C₆-Alkenyl, C₂-C₆-Alkynyl, C₁-C₆-Alkoxy-C₀-C₄-alkyl, Mono- oder Di-C₁-C₄-alkylamino, C₁-C₂-Haloalkoxy, (C₃-C₇-Cycloalkyl)-C₀-C₄-carbohydryl, (C₄-C₇-Cycloalkenyl)-C₀-C₄-carbohydryl, (Aryl)-C₀-C₆-carbohydryl, (Aryl)-C₁-C₄-alkoxy, (C₂-C₆-Heterocycloalkyl)-C₀-C₄-carbohydryl, (Heteroaryl)-C₀-C₆-carbohydryl, C₁-C₆-Alkylthio, -(C₀-C₄Alkyl)O(C=O)R₁₀, -(C₀-C₄Alkyl)-(C=O)NR₁₀R₁₁, -(C₀-C₄Alkyl)O(C=O)NR₁₀R₁₁, -(C₀-C₄Alkyl)(C=O)OR₁₀, -(C₀-C₄Alkyl)NR₁₀(C=O)R₁₁, -(C₀-C₄Alkyl)NR₁₀(C=O)OR₁₁, -(C₀-C₄Alkyl)-NR₁₀(C=O)NR₁₁R₁₂, -(C₀-C₄Alkyl)NR₁₀(C=S)NR₁₁R₁₂, -(C₀-C₄Alkyl)NR₁₀NR₁₁R₁₂ -(C₀-C₄Alkyl)N=NR₁₃, -(C₀-C₄Alkyl)SO₃R₁₀, -(C₀-C₄Alkyl)(S=O)OR₁₀, -(C₀-C₄Alkyl)SO₂R₁₃, -(C₀-C₄Alkyl)SO₂ NR₁₀R₁₁, -(C₀-C₄Alkyl)NR₁₀SO₂R₁₃, =N-OH oder =N-O(C₀-C₄Alkyl) ist,
(iii) -OR_{D}, -(C=O) R_{D}, -SO₂ R_{D}, -SO₃ R_{D} oder -NR₁₀SO₂ R_{D} ist,
worin R_{D} C₁-C₄Alkyl,, (C₃-C₇Cycloalkyl)C₀-C₂alkyl, (C₂-C₆Heterocycloalkyl)C₀-C₂alkyl, (Aryl)C₀-C₂alkyl oder (Heteroaryl)C₀-C₂-alkyl ist,
wobei jedes der (i) und (ii) mit 0 bis 3 Substituenten substituiert ist, die unabhängig ausgewählt sind unter Halogen, Hydroxy, Amino, Cyano, Nitro, -COOH, -(C=O)OCH₃, -CONH₂, C₁-C₄Alkyl, C₂-C₄Alkenyl, C₂-C₄Alkynyl, C₁-C₄Alkoxy, (C₃-C₇-Cycloalkyl)C₀-C₄carbohydryl, (C₃-C₇Cycloalkyl)C₀-C₄alkoxy, Mono- und Di-C₁-C₄alkylamino, C₁-C₂Haloalkyl, C₁-C₂Haloalkoxy und C₂-C₄Alkanoyl, oder
zwei beliebige, an dasselbe Kohlenstoffatom gebundene R_{A} können verbunden sein unter Bildung eines C₃-C₇Cycloalkyls oder einer 3- bis 7-gliedrigen Heterocycloalkylgruppe mit 1 oder 2 Heteroatomen, die unabhängig unter N, 0 und S ausgewählt sind, wobei jede Gruppe mit 0 bis 2 Substituenten substituiert ist, die unter C₁-C₂Alkyl und C₁-C₂-Alkoxy unabhängig ausgewählt sind, oder
zwei beliebige, an verschiedene Kohlenstoffatome gebundene R_{A} können verbunden sein unter Bildung einer C₃-C₇Cycloalkyl- oder einer 3- bis 7-gliedrigen Heterocycloalkylgruppe mit 1 oder 2 Heteroatomen, die unabhängig unter N, 0 und S ausgewählt sind, wobei jede Gruppe mit 0 bis 2 Substituenten substituiert ist, die unabhängig unter C₁-C₂Alkyl und C₁-C₂Alkoxy ausgewählt sind,
R_{B} Wasserstoff oder C₁-C₄Alkyl ist,
R₇ Wasserstoff ist oder
R₇ C₁-C₈Alkyl, C₂-C₆Alkenyl, C₂-C₆Alkynyl, C₂-C₆Alkanoyl, (C₃-C₇Cycloalkyl)C₀-C₄carbohydryl, (C₄-C₇Cycloalkenyl)C₀-C₄carbohydryl, (Aryl)-C₀-C₄carbohydryl, (Aryl)(C=O)-, Mono- oder Di-(C₁-C₆alkyl)carboxamid, C₁-C₆Alkvlester oder (C₂-C₆Heterocycloalkyl)C₀-C₄carbohydryl ist, von denen jedes mit 0 bis 5 Substituenten substituiert ist, die unabhängig ausgewählt sind unter Halogen, Hydroxy, Amino, Cyano, Nitro, C₁-C₄-Alkyl, C₁-C₄Alkoxy, C₁-C₂Haloalkyl, C₁-C₂Haloalkoxy, Mono- und Di-C₁-C₄alkylamino, C₂-C₄Alkanoyl, C₁-C₄Alkylthio, -O(C=O)R₁₀, -(C=O)NR₁₀R₁₁, -O(C=O)NR₁₀R₁₁, -(C=O)OR₁₀, -(C=O)NR₁₀R₁₁, -NR₁₀(C=O)R₁₁, -NR₁₀(C=O)OR₁₁, -NR₁₀(C=O)NR₁₁R₁₂, -NR₁₀(C=S)NR₁₁R₁₂, -NR₁₀NR₁₁R₁₂, -SO₃R₁₀, -(S=O)OR₁₀, -SO₂R₁₃, -SO₂NR₁₀R₁₁ oder -NR₁₀SO₂R₁₃,
A₈ Stickstoff oder CR₈ ist, worin
R₈ Wasserstoff, Halogen, Hydroxy, Amino, Cyano, Nitro oder -NHNH₂ ist oder
R₈ C₁-C₄Alkyl, C₁-C₄Alkoxy, Mono- oder Di-C₁-C₄alkylamino, Mono-, Di- oder Tri-C₁-C₄alkylhydrazinyl, C₂-C₄ Alkanoyl, C₁-C₄Alkylester, C₁-C₂-Haloalkyl oder C₁-C₂Haloalkoxy ist, von denen jedes mit 0 bis 3 Substituenten substituiert ist, die unabhängig ausgewählt sind unter Hydroxy, Amino, Halogen, Oxo, C₁-C₄Alkoxy, C₁-C₂Haloalkyl, C₁-C₂-Haloalkoxy und Mono- und Di-C₁-C₄Alkylamino, und
R₉ C₁-C₈ Alkyl, (C₃-C₇ Cycloalkyl1)C₀-C₄ alkyl oder Phenyl ist, von denen jedes mit 0 bis 3 Substituienten substituiert ist, die unabhängig ausgewählt sind unter Halogen, Hydroxy, Amino, Cyano, Nitro, -COOH, -CONH₂, C₁-C₄Alkyl, C₂-C₄Alkenyl, C₂-C₄Alkynyl, C₁-C₄Alkoxy, (C₃-C₇-Cycloalkyl)C₀-C₄alkyl, (C₃-C₇Cycloalkyl)C₀-C₄alkoxy, Mono- und Di-C₁-C₄alkylamino, C₁-C₂Haloalkyl, C₁-C₂Haloalkoxy und C₂-C₄Alkanoyl.

2. Verbindung oder Salz des Anspruchs 1 der Formel (a) 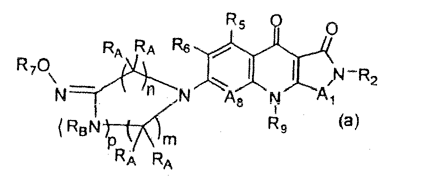 oder der Formel (b)

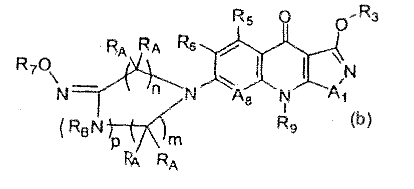

3. Verbindung oder Salz eines der Ansprüche 1 oder 2, bei denen A₁ S, SO, SO₂ oder 0 ist.

4. Verbindung oder Salz des Anspruchs 2 oder 3, bei denen die Verbindung oder das Salz die Formel (a) hat und R₂ Wasserstoff ist oder R₂ C₁-C₆Alkyl oder (C₃-C₇-Cycloalkyl)C₀-C₄alkyl ist, von denen jedes mit mit wenigstens einem Substituenten substituiert ist, der unter Hydroxy, Amine, -COOH, -(C=O)NR₁₀R₁₁ und -CONH₂ ausgewählt ist,
und mit 0 bis 3 Substituenten substituiert ist, die unabhängig ausgewählt sind unter Halogen, Hydroxy, Amino, Cyano, Nitro, -COOH, -CONH₂, C₁-C₄-Alkyl, C₁-C₄Alkoxy, C₁-C₂Haloalkyl, C₁-C₂Haloalkoxy und Mono- und Di-C₁-C₄Alkylamino und C₂-C₄Alkanoyl.

5. Verbindung oder Salz des Anspruchs 4, bei denen R₂ Wasserstoff ist.

6. Verbindung oder Salz eines der Ansprüche 2 bis 3, bei denen die Verbindung eine Verbindung der Formel (b) ist und R₃ C₁-C₄Alkyl oder C₂-C₄Alkanoyl ist.

7. Verbindung oder Salz eines der Ansprüche 1 bis 6, bei denen R₅ Wasserstoff, Amino, C₁-C₂Alkyl, C₁-C₂Alkoxy, Mono- oder Di-C₁-C₄-alkylamino oder Mono- oder Di-C₁-C₄alkylhydrazinyl ist.

8. Verbindung oder Salz des Anspruchs 7, bei denen R₅ Wasserstoff, Amino, Mono- oder Di-C₁-C₂alkylamino oder Mono- oder Di-C₁-C₂alkylhydrazinyl ist.

9. Verbindung oder Salz des Anspruchs 7, bei denen R₅ Wasserstoff ist.

10. Verbindung oder Salz eines der Ansprüche 1 bis 8, bei denen R₆ Wasserstoff, Halogen oder Amino ist.

11. Verbindung oder Salz des Anspruchs 10, bei denen R₆ Fluoro ist.

12. Verbindung oder Salz eines der Ansprüche 1 bis 11, bei denen A₈ Stickstoff oder CR₈ ist.

13. Verbindung oder Salz des Anspruchs 12, in denen A₈ CR₈ ist und R₈ Wasserstoff, Halogen, C₁-C₂Alkyl, C₁-C₂Alkoxy, C₁-C₂Haloalkyl oder C₁-C₂Haloalkoxy ist.

14. Verbindung oder Salz des Anspruchs 13, bei denen R₈ Wasserstoff, oder Methoxy ist.

15. Verbindung oder Salz eines der Ansprüche 1 bis 14, bei denen R₉ C₁-C₄Alkyl, Cyclopropyl oder Phenyl ist, von denen jedes mit 0 bis 3 Substituenten substituiert ist, die unabhängig ausgewählt sind unter Halogen, Hydroxy, Amino, C₁-C₂Alkyl, C₁-C₂Alkoxy, Mono- und Di-C₁-C₂-alkylamino, C₁-C₂Haloalkyl und C₁-C₂Haloalkoxy.

16. Verbindung oder Salz des Anspruchs 15, bei denen
R₉ C₁-C₄Alkyl oder Cyclopropyl ist oder
R₉ Phenyl ist, das mit 2 Substituenten substituiert ist, die unter Halogen, Hydroxy, Amino, C₁-C₂Alkyl, C₁-C₂Alkoxy, Mono- und Di-C₁-C₂-alkylamino, C₁-C₂-Haloalkyl und C₁-C₂Alkoxy ausgewählt sind.

17. Verbindung oder Salz des Anspruchs 15, bei denen R₉ Ethyl, t-Butyl, Cyclopropyl oder 2,4-Difluorophenyl ist.

18. Verbindung oder Salz des Anspruchs 15, bei denen R₉ Cyclopropyl ist.

19. Verbindung oder Salz eines der Ansprüche 1 bis 18, bei denen
R₇ Wasserstoff ist oder
R₇ C₁-C₈Alkyl, C₂-C₆Alkanoyl, (C₃-C₇Cycloalkyl)C₀-C₄carbohydryl, (Aryl)-C₀-C₄carbohydryl, (Aryl)(C=O)-, Mono- oder Di-(C₁-C₆alkyl)carboxamid, C₁-C₆Alkylester oder (C₂-C₆Heterocycloalkyl)C₀-C₄carbohydryl ist, von denen jedes mit 0 bis 5 Substituenten substituiert ist, die unabhängig ausgewählt sind unter Halogen, Hydroxy, Amino, Cyano, Nitro, C₁-C₄-Alkyl, C₁-C₄Alkoxy, C₁-C₂Haloalkyl, C₁-C₂Haloalkoxy, Mono- und Di-C₁-C₄alkylamino, C₂-C₄Alkanoyl, C₁-C₄Alkylthio, -O(C=C)R₁₀, -(C=O)NR₁₀R₁₁, -O(C=O)NR₁₀R₁₁, -(C=O)OR₁₀, -(C=O)NR₁₀OR₁₁, -NR₁₀(=C)R₁₁, -NR₁₀(C=O)OR₁₁, -NR₁₀(C=O)NR₁₁R₁₂, -NR₁₀(C=S)NR₁₁R₁₂, -NR₁₀NR₁₁R₁₂, -SO₃R₁₀, -(S=O)OR₁₀, -SO₂R₁₃, -SO₂NR₁₀R₁₁ oder -NR₁₀SO₂R₁₃.

20. Verbindung oder Salz des Anspruchs 19, bei denen
R₇ Wasserstoff ist oder
R₇ C₁-C₈Alkyl, (C₃-C₇Cycloalkyl)C₀-C₄carbohydryl, (Aryl)C₀-C₄carbohydryl oder (C₂-C₆Heterocycloalky)C₀-C₄carbohydryl ist, von denen jedes mit 0 bis 5 Substituenten substituiert ist, die unabhängig ausgewählt sind unter Halogen, , Hydroxy, Amino, Cyano, C₁-C₄Alkyl, C₁-C₄Alkoxy, C₁-C₂Haloalkyl, C₁-C₂Haloalkoxy, Mono- und Di-C₁-C₄alkylamino, C₂-C₄Alkanoyl, -(C=O)NR₁₀R₁₁, -NR₁₀(C=O)R₁₁.

21. Verbindung oder Salz des Anspruchs 20, bei denen
R₇ Wasserstoff oder C₁-C₈Alkyl oder (Aryl)C₀-C₄alkyl ist, von denen jedes mit 0 bis 5 Substituenten substituiert ist, die unabhängig ausgewählt sind unter Halogen, Hydroxy, Amino, Cyano, C₁-C₄Alkyl, C₁-C₄Alkoxy, C₁-C₂Haloalkyl, C₁-C₂Haloalkoxy, Mono- und Di-C₁-C₄-alkylamino, C₂-C₄Alkanoyl, -(C=O)NR₁₀R₁₁, -NR₁₀(C=O)R₁₁.

22. Verbindung oder Salz des Anspruchs 20, bei denen R₇ Wasserstoff, C₁-C₂Alkyl oder Benzyl ist.

23. Verbindung oder Salz eines der Ansprüche 1 bis 22, bei denen n 1 ist, m 2 ist, p 1 ist und R₈ Wasserstoff ist.

24. Verbindung oder Salz eines der Ansprüche bis 22, bei denen p 0 ist.

25. Verbindung oder Salz des Anspruchs 24, bei denen n 1 ist und m 2 ist oder bei denen n 2 ist und m 2 ist.

26. Verbindung der Salze eines der Ansprüche 1 bis 25, bei denen
jedes R_{A} unabhängig (i), (ii) oder (iii) ist, wobei
(i) Wasserstoff, Hydroxy, Amino oder Cyano ist,
(ii) C₁-C₆-Alkyl, C₂-C₆-Alkeny, C₂-C₆-Alkynyl, C₁-C₆-Alkoxy-C₀-C₄-alkyl, Mono- oder Di-C₁-C₄-alkylamino, C₁-C₂-Haloalkoxy, (C₃-C₇-Cycloalkyl)-C₀-C₄-carbohydryl, (C₄-C₇-Cycloalkenyl)-C₀-C₄-carbohydryl, (Aryl)-C₀-C₆-carbohydryl, (Aryl)-C₁-C₄-alkoxy, (C₂-C₆-Heterocycloalkyl)-C₀-C₄-carbohydryl, (Heteroaryl)-C₀-C₆-carbohydryl, C₁-C₆-Alkylthio, -(C₀-C₄Alkyl)O(C=O)R₁₀, -(C₀-C₄Alkyl)-(C=O)NR₁₀R₁₁, -(C₀-C₄Alkyl)O(C=O)NR₁₀R₁₁, -(C₀-C₄Alkyl)(C=O)OR₁₀, -(C₀-C₄Alkyl)NR₁₀(C=O)R₁₁, -(C₀-C₄Alkyl)NR₁₀(C=O)OR₁₁, -(C₀-C₄Alkyl)-NR₁₀(C=O)NR₁₁R₁₂, -(C₀-C₄Alkyl)NR₁₀(C=S)NR₁₁R₁₂, -(C₀-C₄Alkyl)NR₁₀NR₁₁R₁₂ -(C₀-C₄Alkyl)N=NR₁₃, -(C₀-C₄Alkyl)SO₃R₁₀, -(C₀-C₄Alkyl)(S=O)OR₁₀, -(C₀-C₄ Alkyl)SO₂R₁₃, -(C₀-C₄Alkyl)SO₂NR₁₀R₁₁, -(C₀-C₄ Alkyl)NR₁₀SO₂R₁₃, =N-OH oder =N-O(C₀-C₄Alkyl) ist,
(iii) -OR_{D}, -(C=O)R_{D}, -SO₂R_{D}, -SO₃R_{D} oder -NR₁₀SO₂ R_{D} ist,
worin R_{D} C₁-C₄Alkyl,, (C₃-C₇Cycloalkyl)C₀-C₂alkyl, (C₂-C₆Heterocycloalkyl)C₀-C₂alkyl, (Aryl)C₀-C₂alkyl oder (Heteroaryl)C₀-C₂-alkyl ist,
wobei jedes der (i) und (ii) mit 0 bis 3 Substituenten substituiert ist, die unabhängig ausgewählt sind unter Halogen, Hydroxy, Amino, Cyano, Nitro, -COOH, -(C=O)OCH₃, -CONH₂, C₁-C₄Alkyl, C₂-C₄Alkenyl, C₂-C₄Alkynyl, C₁-C₄Alkoxy, (C₃-C₇-Cycloalkyl)C₀-C₄carbohydryl, (C₃-C₇Cycloalkyl)C₀-C₄alkoxy, Mono- und Di-C₁-C₄alkylamino, C₁-C₂Haloalkyl, C₁-C₂Haloalkoxy und C₂-C₄Alkanoyl, oder
zwei beliebige, an dasselbe Kohlenstoffatom gebundene R_{A} können verbunden sein unter Bildung eines C₃-C₇Cycloalkyls oder einer 3- bis 7-gliedrigen Heterocycloalkylgruppe mit 1 oder 2 Heteroatomen, die unabhängig unter N, 0 und S ausgewählt sind, wobei jede Gruppe mit 0 bis 2 Substituenten substituiert ist, die unter C₁-C₂Alkyl und C₁-C₂-Alkoxy unabhängig ausgewählt sind, oder
zwei beliebige, an verschiedene Kohlenstoffatome gebundene R_{A} können verbunden sein unter Bildung einer C₃-C₇Cycloalkyl- oder einer 3- bis 7-gliedrigen Heterocycloalkylgruppe mit 1 oder 2 Heteroatomen, die unabhängig unter N, 0 und S ausgewählt sind, wobei jede Gruppe mit 0 bis 2 Substituenten substituiert ist, die unabhängig unter C₁-C₂Alkyl und C₁-C₂Alkoxy ausgewählt sind,

27. Verbindung oder Salz des Anspruchs 26, bei denen
jedes R_{A} unabhängig (i) oder (ii) ist, wobei
(i) Wasserstoff oder Amino ist,
(ii) C₁-C₆Alkyl, (C₁-C₆Alkaxy)C₀-C₄alkyl, Mono- oder Di-C₁-C₄alkylamino, (C₃-C₇Cycloalkyl)C₀-C₄carbohydryl, (Aryl)C₀-C₆carbohydril, (C₂-C₆Heterocycloalkyl1)C₀-C₄carbohydryl, (Heteroaryl)C₀-C₆carbohydryl, -(C₀-C₄Alkyl)(C=O)NR₁₀R₁₁, -(C₀-C₄Alkyl)NR₁₀(C=O)R₁₁, =N-OH oder =N-O(C₀-C₄alkyl) ist,
wobei jedes der (ii) mit 0 bis 3 Substituenten substituiert ist, die unabhängig ausgewählt sind unter Halogen, Hydroxy, Amino, Cyano, Nitro, -COOH, -(C=O)OCH₃, -CONH₂, C₁-C₄Alkyl, C₂-C₄Alkenyl, -C₂₋C₄Alkynyl, C₁-C₄Alkoxy, (C₃-C₇-Cycloalkyl)C₀-C₄carbohydryl, (C₃-C₇Cycloalkyl)C₀-C₄alkoxy, Mono- und Di-C₁-C₄alkylamino, C₁-C₂Haloalkyl, C₁-C₂Haloalkoxy und C₂-C₄Alkanoyl, oder
zwei beliebige, an dasselbe Kohlenstoffatom gebundene R_{A} können verbunden sein unter Bildung eines C₃-C₇Cycloalkyls oder einer 3- bis 7-gliedrigen Heterocycloalkylgruppe mit 1 oder 2 Heteroatomen, die unabhängig unter N, 0 und S ausgewählt sind, wobei jede Gruppe mit O bis 2 Substituenten substituiert ist, die unter C₁-C₂Alkyl und C₁-C₂-Alkoxy unabhängig ausgewählt sind, oder
zwei beliebige, an verschiedene Kohlenstoffatome gebundene R_{A} können verbunden sein unter Bildung einer C₃-C₇Cycloalkyl- oder einer Heterocycloalkylgruppe mit 1 oder 2 Heteroatomen, die unabhängig unter N, 0 und S ausgewählt sind, wobei jede Gruppe mit 0 bis 2 Substituenten substituiert ist, die unabhängig unter C₁-C₂Alkyl und C₁-C₂Alkoxy ausgewählt sind,

28. Verbindung oder Salz des Anspruchs 26, bei denen
jedes R_{A} unabhängig (i) oder (ii) ist, wobei
(i) Wasserstoff oder Amino ist,
(ii) C₁₋C₆Alkyl, Mono- oder Di-C₁-C₄Alkylamino, (C₃-C₇Cycloalkyl)-C₀-C₄alkyl, (C₂-C₆Heterocycloalkyl)C₀-C₄alkyl, -(C₀-C₄Alkyl)-(C=O)NR₁₀R₁₁, -(C₀-C₄Alkyl)NR₁₀(C=O)R₁₁, =N-OH oder =N-O(C₀-C₄alkyl) ist,
wobei jedes der (ii) mit 0 bis 3 Substituenten substituiert ist, die unabhängig unter Hydroxy, Amino, Cyano und -CONH₂ ausgewählt sind, und
zwei beliebige, an das gleiche Kohlenstoffatom gebundene R_{A} verbunden sein können unter Bildung einer C₃-C₇Cycloalkyl- oder Heterocycloalkylgruppe mit 1 oder 2 Heteroatomen, die unabhängig unter N, O und S ausgewählt sind, wobei jede Gruppe mit 0 bis 2 Substituenten substituiert ist, die unabhängig unter C₁-C₂Alkyl und C₁-C₂Alkoxy ausgewählt sind.

29. Verbindung oder Salz des Anspruchs 26, bei denen
jedes R_{A} unabhängig (i) oder (ii) ist, wobei
(i) Wasserstoff oder Amino ist,
(ii) C₁-C₆Alkyl, (C₃-C₇Cycloalkyl)C₀-C₂alkyl oder Mono- oder Di-C₁-C₄alkylamino ist, wobei jedes der (ii) mit 0 bis 3 Amino substituiert ist,
zwei beliebige an das gleiche Kohlenstoffatom gebundene R_{A} verbunden sein können unter Bildung einer C₃-C₇Cycloalkyl- oder einer 3- bis 7-gliedrigen Heterocycloalkylgruppe mit 1 oder 2 Heteroatomen, die unabhängig unter N, 0 und S ausgewählt sind, wobei jede Gruppe mit 0 bis 2 Substituenten substituiert ist, die unter C₁-C₂Alkyl und C₁-C₂-Alkoxy unabhängig ausgewählt sind, oder
zwei beliebige, an verschiedene Kohlenstoffatome gebundene R_{A} verbunden sein können unter Bildung einer C₃-C₇Cycloalkyl- oder einer 3- bis 7-gliedrigen Heterocycloalkylgruppe mit 1 oder 2 Heteroatomen, die unabhängig unter N, 0 und S ausgewählt sind, wobei jede Gruppe mit 0 bis 2 Substituenten substituiert ist, die unabhängig unter C₁-C₂Alkyl und C₁-C₂Alkoxy ausgewählt sind.

30. Verbindung oder Salz des Anspruchs 26, bei denen jedes R_{A} unabhängig Wasserstoff, C₁-C₄Alkyl oder mit einem Amino-Substituenten substituiertes C₁-C₄Alkyl ist

31. Verbindung oder Salz des Anspruchs 30, bei denen ein oder zwei R_{A} mit einem Amino-Substituenten substituierte C₁-C₄Alkyl sind und die übrigen R_{A} Wasserstoff sind.

32. Verbindung oder Salz des Anspruchs 26, bei denen
zwei an das gleiche Kohlenstoffatome gebundene R_{A} verbunden sind unter Bildung einer C₃-C₇Cycloalkyl-oder einer 3- bis 7-gliedrigen Heterocycloalkylgruppe mit 1 oder 2 Heteroatomen, die unabhängig unter N, O und S ausgewählt sind, von denen jedes mit 0 bis 2 Substituenten substituiert ist, die unabhängig unter C₁-C₂Alkyl und C₁-C₂Alkoxy augewählt sind, und
die übrigen R_{A} unabhängig unter Wasserstoff, C₁-C₂Alkyl und C₁-C₂Alkoxy ausgewählt sind.

33. Verbindung oder Salz des Anspruchs 32, bei denen
zwei an das gleiche Kohlenstoffatom gebundene R_{A} verbunden sind unter Bildung einer C₃-C₄Cycloalkyl- oder einer 3- bis 4-gliedrigen Heterocycloalkylgruppe mit 1 N-Atom, von denen jede mit 0 bis 2 Substituenten substituiert ist, die unabhängig unter C₁-C₂Alkyl und C₁-C₂Alkoxy ausgewählt sind, und
die übrigen R_{A} unabhängig unter Wasserstoff, C₁-C₂Alkyl und C₁-C₂Alkoxy ausgewählt sind.

34. Verbindung oder Salz der Formel (a) von Anspruch 2, bei denen
R₂ Wasserstoff oder C₁-C₆Alkyl ist, das mit einem Substituenten substituiert ist, der unter Hydroxy, Amino, -COOH, -(C=O)NR₁₀OR₁₁ und -CONH₂ ausgewählt ist.
R₅ Wasserstoff ist,
R₆ Halogen oder Amino ist,
R₇ Wasserstoff, C₁-C₂Alkyl oder Benzyl ist,
A₈ Stickstoff oder CR₈ ist und R₈ Wasserstoff oder Methoxy ist, wenn A₈ CR₈ ist,
R₉ Ethyl, t-Butyl, Cyclopropyl oder 2,4-Difluorophenyl ist und
jedes R_{A} unabhängig Wasserstoff oder C₁-C₄Alkyl ist, das wahlweise mit einem Amino-Substituenten substituiert ist, oder
zwei beliebige an das gleiche Kohlenstoffatom gebundene R_{A} verbunden sein können unter Bildung einer C₃-C₇Cycloalkyl- oder einer 3- bis 7-gliedrigen Heterocycloalkylgruppe mit 1 oder 2 Heteroatomen, die unabhängig unter N, O und S ausgewählt sind, wobei jede Gruppe mit 0 bis 2 Substituenten substituiert ist, die unter C₁-C₂Alkyl und C₁-C₂-Alkoxy unabhängig ausgewählt sind, oder
zwei beliebige, an verschiedene Kohlenstoffatome gebundene R_{A} verbunden sein können unter Bildung einer C₃-C₇Cycloalkyl- oder einer Heterocycloalkylgruppe mit 1 oder 2 Heteroatomen, die unabhängig ausgewählt sind unter N, O und S, wobei jede Gruppe mit 0 bis 2 Substituenten substituiert ist, die unabhängig unter C₁-C₂Alkyl und C₁-C₂Alkoxy ausgewählt sind.

35. Verbindung oder Salz des Anspruchs 1, bei denen die Verbindung
7-[(4Z)-3-Aminomethyl-4-methoxyimino-pyrrolidin-1-yl)]-9-cyclopropyl-6-fluoro-9H-isothiazolo[5,4-b] chinolin-3,4-dion,
7-[(3R,4Z)-3-Aminomethyl-4-methoxyimino-pyrrolidin-1-yl)]-9-cyclopropyl-6-fluoro-9H-isothiazolo[5,4-b] chinolin-3,4-dion,
7-[(3S,4Z)-3-Aminomethyl-4-methoxyimino-pyrrolidin-1-yl)]-9-cyclopropyl-6-fluoro-9H-isothiazolo[5,4-b] chinolin-3,4-dion,
7-[(4E)-3-Aminomethyl-4-methoxyimino-pyrrolidin-1-yl)]-9-cyclopropyl-6-fluoro-9H-isothiazolo[5,4-b] chinolin-3, 4-dion,
7-[(3R,4E)-3-Aminomethyl-4-methoxyimino-pyrrolidin-1-yl)]-9-cyclopropyl-6-fluoro-9H-isothiazolo[5,4-b] chinolin-3,4-dion,
7-[(3S,4E)-3-Aminomethyl-4-methoxyimino-pyrrolidin-1-yl)]-9-cyclopropyl-6-fluoro-9H-isothiazolo[5,4-b] chinolis-3,4-dion,
9-Cyclopropyl-6-fluoro-7-(8-methoxyimino-2,6-diaza-spiro[3.4]oct-6-yl)-9H-isothiazolo[5,4-b]chinolin-3,4-dion,
9-Cyclopropyl-6-fluoro-7-(7-methoxyimino-1,5-diaza-spiro[2.4]hept-5-yl)-9H-isothiazolo[5,4-b]chinolin-3,4-dion,
7-[3-(1-Amino-cyclopropyl)-4-methoxyimino-pyrrolidin-1-y]-9-cyclopropyl-6-fluoro-9H-isothiazolo[5,4-b] chinolin-3,4-dion,
9-Cyclopropyl-6-fluoro-7-(7-methoxyimino-5-aza-spiro[2.4]hept-5-yl)-9H-isothiazolo[5,4-b]chinolin-3,4-dion,
7-(3-Aminomethyl-4-hydroxyimino-pyrrolidin-1-yl)-9-cyclopropyl-6-fluoro-9H-isothiazolo[5,4-b]chinolin-3,4-dion,
7-[3-(2-Amino-ethyl)-4-methoxyimino-pyrrolidin-1-yl]-9-cyclopropyl-6-fluoro-9H-isothiazolo[5,4-b]chinolin-3,4-dion,
7-(3-Aminomethyl-4-benzyloxyimino-pyrrolidin-1-yl)-9-cyclopropyl-6-fluoro-9H-isothiazolo[5,4-b]chinolin-3,4-dion,
7-(3-Amino-4-methoxyimino-pyrrolidin-1-yl)-9-cyclopropyl-6-fluoro-9H-isothiazolo[5,4-b]chinolin-3,4-dion;
7-(3-Aminomethyl-4-methoxyimino-3-methyl-pyrrolidin-1-yl)-9-cyclopropyl-6-fluoro-9H-isothiazolo[5,4-b] chinolin-3,4-dion,
7-[3-(1-Amino-ethyl)-4-methoxyimino-pyrrolidin-1-yl]-9-cyclopropyl-6-fluoro-9H-isothiazolo[5,4-b] chinolin-3,4-dion,
7-(3,3-Bis-aminomethyl-4-methoxyimino-pyrrolidin-1-y)-9-cyclopropyl-6-fluoro-9H-isothiazolo[5,4-b] chinolin-3,4-dion,
7-[3-(1-Amino-1-methyl-ethyl)-4-methoxyimino-pyrrolidin-1-yl]-9-cyclopropyl-6-fluoro-9H-isothiazolo[5,4-b] chinolin-3,4-dion,
9-Cyclopropyl-6-fluoro-7-(3-methoxyimino-piperazin-1-yl)-9H-isothiazolo[5,4-b] chinolin-3,4-dion,
9-Cyclopropyl-6-fluoro-7-(4-methoxyimino-piperidin-1-yl)-9H-isothiazolo[5,4-b] chinolin-3,4-dien,
7-(3-Aminomethyl-4-methoxyimino-piperidin-1-yl)-9-cyclopropyl-6-fluoro-9H-isothiazolo[5,4-b]chinolin-3,4-dion,
7-(3-Aminomethyl-4-methoxyimino-pyrrolidin-1-yl)-9-cyclopropyl-6-fluoro-1,1-dioxo-1,2-dihydro-9H-116-isothiazolo[5,4-b] chinolin-3,4-dion,
4-[7-(3-Aminomethyl-4-methoxyimino-pyrrolidin-1-yl)-9-cyclopropyl-6-fluoro-3,4-dioxo-4,9-dihydro-3H-isothiazolo[5,4-b]chinolin-2-yl]-buttersäure,
Essigsäure-7-(3-aminomethyl-4-methoxyimino-pyrrolidin-1-yl)-9-cyclopropyl-6-fluoro-4-oxo-4,9-dihydro-isothiazolo[5,4-b]chinolin-3-yl-ester,
7-(3-Aminomethyl-4-methoxyimino-pyrrolidin-1-yl)-9-cyclopropyl-6-fluoro-5-(N'-methyl-hydrazino)-9H-isothiazolo-[5,4-b]chinolin-3,4-dion,
6-Amino-7-(3-aminomethyl-4-methoxyimino-pyrrolidin-1-yl)-9-cyclopropyl-9H-isothiazolo[5,4-b]chinolin-3,4-dion,
7-(3-Aminomethyl-4-methoxyimino-pyrrolidin-1-yl)-9-cyclopropyl-6-fluoro-8-methoxy-9H-isothiazolo[5,4-b]chinolin-3,4-dion und
7-(3-Aminomethyl-4-methoxyimino-pyrrolidin-1-yl)-9-cyclopropyl-8-methoxy-9H-isothiazolo[5,4-b]chinolin-3,4-dion ist.

36. Antibakterielle Zusammensetzung, die eine Verbindung oder ein Salz eines der Ansprüche 1 bis 35 zusammen mit einem Träger, Verdünnungsmittel oder Bindemittel enthält.

37. Pharmazeutische Zusammensetzung, die eine Verbindung oder ein Salz eines der Ansprüche 1 bis 35 zusammen mit einem pharmazeutisch zulässigen Träger, Verdünnungsmittel oder Bindemittel enthält.

38. Pharmazeutische Zusammensetzung des Anspruchs 36, bei der die Zusammensetzung als injizierbare Flüssigkeit, ein Aerosol, eine Creme, ein Gel, eine Pille, eine Kapsel, eine Tablette, ein Sirup, ein transdermales Pflaster oder eine augenmedizinische Lösung formuliert ist.

39. Zusammensetzung mit einer Verbindung oder einem Salz eines der Ansprüche 1 bis 35 in Kombination mit wenigstens einem anderen antibakteriellen Mittel, Antiprotozoenmittel, fungiziden Mittel, antiviralen Mittel, Interferon, Effluxpumpen-Inhibitor oder Beta-Lactamase-Inhibitor.

40. Packung mit der pharmazeutischen Zusammensetzung des Anspruchs 39 in einem Behälter und ferner mit Anweisungen zur Anwendung der Zusammensetzung zur Behandlung eines an Mikroorganismeninfektion leidenden Patienten.

41. Packung des Anspruchs 40, bei der die Anweisungen solche zur Anwendung der Zusammensetzung zur Behandlung eines an bakterieller Infektion leidenden Patienten sind.

42. Die Verbindung oder das Salz eines der Ansprüche 1 bis 35 für die Anwendung bei einer Methode zur Behandlung oder Vorbeugung einer bakteriellen oder durch Protozoen erregten Infektion.

43. Verbindung des Anspruchs 42, bei der die bakterielle oder durch Protozoen erregte Infektion eine Harnweginfektion, Nierenbeckenentzündung, untere Atemwegsinfektion, Hautinfektion, Hautstrukturinfektion, Gonokokken-Harnröhreninfektion, Gonokokken-Gebärmutterhalsinfektion, Chlamydien-Harnröhreninfektion, Chlamydien-Gebärmutterhalsinfektion, Knocheninfektion, Gelenkinfektion, gramnegative bakterielle Infektion, infektiöser Durchfall, Abdominaltyphus, Prostataentzündung, akute Sinusitis, akute Verschlimmerung chronischer Bronchitis,Bauchhöhlen-infektion, gynäkologische Infektion oder Beckeninfektion ist.

44. Verbindung des Anspruchs 42, bei der das Lebewesen ein Fisch, eine Amphibie, ein Reptil, ein Vogel oder ein Säuger ist.

45. Verbindung des Anspruchs 44, bei der das Lebewesen ein Säuger ist.

46. Verbindung des Anspruchs 45, bei der das Lebewesen ein Mensch ist.

47. Verbindung oder Salz eines der Ansprüche 1 bis 35, die eine MIC von 10 µg/ml oder weniger gegen S. aureus oder E. coli zeigen.

48. Verbindung oder Salz eines der Ansprüche 1 bis 35, die eine MIC von 1 µg/ml oder weniger gegen S. aureus oder E. coli zeigen.

49. Verwendung einer Verbindung eines der Ansprüche 1 bis 35 für die Herstellung eines Heilmittels für die Behandlung einer bakteriellen oder durch Protozoen erregten Infektion.

50. Verwendung nach Anspruch 49, bei der die bakterielle oder durch Protozoen erregte Infektion eine Harnweginfektion, Nierenbeckenentzündung, untere Atemwegsinfektion, Hautinfektion, Hautstrukturinfektion, Gonokokken-Harnröhreninfektion, Gonokokken-Gebärmutterhalsinfektion, Chlamydien-Harnröhreninfektion, Chlamydien-Gebärmutterhalsinfektion, Knocheninfektion, Gelenkinfektion, gramnegative bakterielle Infektion, infektiöser Durchfall, Abdominaltyphus, Prostataentzündung, akute Sinusitis, akute Verschlimmerung chronischer Bronchitis,Bauchhöhlen-infektion, gynäkologische Infektion oder Beckeninfektion ist.

51. Verwendung nach Anspruch 49, bei der die Bakterieninfektion eine Infektion durch S. aureaus oder E. coli ist.

## Revendications

1. Composé de formule I ou de formule II 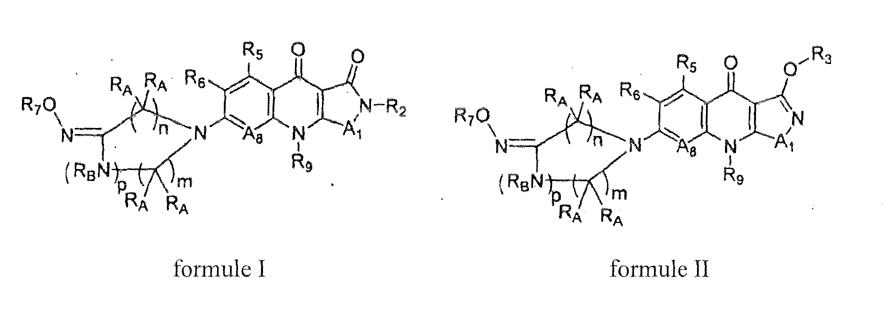 ou sel pharmaceutiquement acceptable, solvate, tautomère ou isomère optique de celui-ci, où
A₁ est S, O, SO ou SO₂ ;
R₂ est l'hydrogène, ou
R₂ est C₁-C₈alkyle, C₂-C₆alcényle, C₂-C₆alcynyle, (C₃-C₇cycloalkyle)-C₀-C₄carbohydryle, (C₄-C₇cycloalcényle)-C₀-C₄carbohydryle, (aryle)-C₀-C₄carbohydryle ou (C₂-C₆hétérocycloalkyle)-C₀-C₄carbohydryle, substitués chacun avec 0 à 5 substituant choisis indépendamment parmi halogène, hydroxy, amino, cyano, nitre, C₁-C₄alkyle, C₁-C₄alcoxy, C₁-C₂haloalkyle, C₁-C₂haloalcoxy, mono- et di-C₁-C₄alkylamino, C₂-C₄alcanoyle, C₁-C₄alkylthio,
-O(C=O)R₁₀, -(C=O)NR₁₀R₁₁, -O(C=O)NR₁₀R₁₁, -(C=O)OR₁₀, -(C=O)NR₁₀OR₁₁, -NR₁₀(C-O)R₁₁, -NR₁₀(C-O)OR₁₁, -NR₁₀(C=O)NR₁₁R₁₂, -NR₁₀(C=S)NR₁₁R₁₂, -NR₁₀NR₁₁R₁₂, -SO₃R₁₀, -(S=O)OR₁₀, -SO₂R₁₃, -SO₂NR₁₀R₁₁, =N-OR₁₀ et -NR₁₀SO₂R_{13;} où chaque R₁₀, R₁₁ et R₁₂ est indépendamment l'hydrogène, C₁-C₄alkyle ou aryle, et chaque R₁₃ est indépendamment C₁-C₄alkyle ou aryle;
R₃ est l'hydrogène, C₁-C₆alkyle, C₁-C₆alcanoyle, mono- ou di-C₁-C₆alkylcarbamate, C₁-C₆alkylphosphate ou C₁-C₆alkylsulfonate; substitués chacun avez 0 à 3 substituants choisis indépendamment parmi halogène, hydroxy, amino, cyano, nitro, C₁-C₄alcoxy, mono- et di-C₁-C₄alkylamino, C₁-C₂haloalkyle et C₁-C₂haloalcoxy ;
R₅ est l'hydrogène, un halogène, hydroxy, amino, cyano, nitro, -NHNH₂, C₁-C₂haloalkyle ou C₁-C₂haloalcoxy ; ou
R₅ est C₁-C₄alkyle, C₁-C₄alcoxy, mono- ou di-C₁-C₄alkylamino, mono-, di- ou triC₁-C₄alkylhydrazinyle, C₂-C₄alcanoyle ou C₁-C₄alkylester; substitués chacun avec 0 à 3 substituants choisis indépendamment parmi hydroxy, amino, halogène, oxo, C₁-C₄alcoxy, C₁-C₂haloalkyle, C₁-C₂haloalcoxy et mono- et di-C₁-C₄alkylamino ;
R₆ est l'hydrogène, un halogène, hydroxy, amino, cyano, C₁-C₄alkyle, C₁-C₄alcoxy, mono- ou di-C₁-C₄alkylamino, -SO₃R₁₀, -SO₂R₁₀,
-SO₂NR₁₀R₁₁;
n est 1, 2 ou 3 ; m est 1, 2 ou 3 ; p est 0 ou 1 ;
chaque R_{A} est indépendamment (i), (ii) ou (iii) ; où
(i) est l'hydrogène, hydroxy, amino ou cyano,
(ii) est C₁-C₆alkyle_{,} C₂-C₆alcényle, C₂-C₆alcynyle, C₁-C₆alcoxy-C₀-C₄alkyle, mono- ou di-C₁-C₄alkylamino, C₁-C₂haloalcoxy, (C₃-C₇cycloalkyle)-C₀-C₄carbohydryle, (C₄-C₇cycloalcényle)-C₀-C₄carbohydryle, (aryle)-C₀-C₆carbobydryle, (aryle)-C₁-C₄alcoxy, (C₂-C₆hétérocycloalkyle)-C₀-C₄carbohydryle, (hétéroaryle)-C₀-C₆carbohydryle, C₁-C₆alkylthio, -(C₀-C₄alkyle)O(C=C)R₁₀, -(C₀-C₄alkyle)(C=O)NR₁₀R₁₁, -(C₀-C₄alkyle)O(C=O)NR₁₀R₁₁, -(C₀-C₄alkyle)(C=O)OR₁₀, -(C₀-C₄alkyle)NR₁₀(C=O)R₁₁, -(C₀-C₄alkyle)NR₁₀(C=O)OR₁₁, -(C₀-C₄alkyle)NR₁₀(C=O)NR₁₁R₁₂, -(C₀-C₄alkyle)NR₁₀(C=S)NR₁₁R₁₂, -(C₀-C₄alkyle)NR₁₀NR₁₁R₁₂, -(C₀-C₄alkyle)N=NR_{13;} -(C₀-C₄alkyle)SO₃R₁₀, -(C₀-C₄alkyle)(S=O)OR₁₀, -(C₀-C₄alkyle)SO₂R₁₃, -(C₀-C₄alkyle)SO₂NR₁₀R₁₁, -(C₀-C₄alkyle)NR₁₀SO₂R₁₃; -N-OH ou =N-O(C₀-C₄alkyle) ;
(iii) est -OR_{D}, -(C=O)R_{D}, -SO₂R_{D}, -SO₃R_{D} ou -NR₁₀SO₂R_{D}, où R_{D} est C₁-C₄alkyle, (C₃-C₇cycloalkyle-C₀-C₂alkyle, (C₂-C₆hétérocycloalkyle)-C₀-C₂alkyle, (aryle)-C₀-C₂alkyle ou (hétéroaryle)-C₀-C₂alkyle ;
où chacun de (ii) et (iii) est substitué avec 0 à 3 substituants choisis indépendamment parmi halogène, hydroxy, amino, cyano, nitro, -COOH, -(C=O)OCH₃, -CONH₂, C₁-C₄alkyle, C₂-C₄alcényle, C₂-C₄alcynyle, C₁-C₄alcoxy, (C₃-C₇cycloalkyle)-C₀-C₄carbahydryle, (C₃-C₇cycloalkyle)-C₀-C₄alcoxy, mono- et di-C₁-C₄alkylamino, C₁-C₂haloalkyle, C₁-C₂haloalcoxy et C₂-C₄alcanoyle ; ou
deux R_{A} quelconques liés au même atome de carbone peuvent être joints pour former un groupe C₃-C₇cycloalkyle ou un groupe hétérocycloalkyle à 3 à 7 chaînons ayant 1 ou 2 hétéroatomes choisis indépendamment parmi N, O et S ; substitués chacun avec 0 à 2 substituants choisis indépendamment parmi C₁-C₂alkyle et C₁-C₂alcoxy ; ou
deux R_{A} quelconques liés à des atomes de carbone différents peuvent être joints pour former un groupe C₃-C₇cycloalkyle ou un groupe hétérocycloalkyle à 3 à 7 chaînons ayant 1 ou 2 hétéroatomes choisis indépendamment parmi N. O et S ; substitués chacun avec 0 à 2 substituants choisis indépendamment parmi C₁-C₂alkyle et C₁-C₂alcoxy ;
R_{B} est l'hydrogène ou C₁-C₄alkyle ;
R₇ est l'hydrogène, ou
R₇ est C₁-C₈alkyle, C₂-C₆alcényle, C₂-C₆alcynyle, C₂-C₆alcanoyle, (C₃-C₇cycloalkyle)-C₀-C₄carbohydryle, (C₄-C₇cycloalcényle)-C₀-C₄carbohydryle, (aryle)-C₀-C₄carbohydryle, (aryle)(C=O)-, mono- ou di-(C₁-C₆alkyl)-carboxamide, C₁-C₆alkylester ou (C₂-C₆hétérocycloalkyle)-C₀-C₄carbohydryle, substitués chacun avec 0 à 5 substituants choisis indépendamment parmi halogène, hydroxy, amino, cyano, nitro, C₁-C₄alkyle, C₁-C₄alcoxy, C₁-C₂haloalkyle, C₁-C₂haloalcoxy, mono- et di-C₁-C₄alkylamino, C₂-C₄alcanoyle, C₁-C₄alkylthio,
-O(C=O)R₁₀, -(C=O)NR₁₀R₁₁, -O(C=O)NR₁₀R₁₁, -(C=O)OR₁₀, -(C=O)NR₁₀OR₁₁, -NR₁₀(C=O)R₁₁, -NR₁₀(C=O)OR₁₁, -NR₁₀(C=O)NR₁₁R₁₂, -NR₁₀(C=S)NR₁₁R₁₂, -NR₁₀NR₁₁R₁₂, -SO₃R₁₀, -(S=O)OR₁₀, -SO₂R₁₃, -SO₂NR₁₀R₁₁ ou -NR₁₀SO₂R₁₃ ;
A₈ est l'azote ou CR₈ ; où
R₈ est l'hydrogène, un halogène, hydroxy, amino, cyano, nitro ou -NHNH₂ ; ou
R₈ est C₁-C₄alkyle, C₁-C₄alcoxy, mono- ou di-C₁-C₄alkylamino, mono-, di- ou triC₁-C₄alkylhydrazinyle, C₂-C₄alcanoyle, C₁-C₄alkylester, C₁-C₂haloalkyle ou C₁-C₂haloalcoxy, substitués chacun avec 0 à 3 substituants choisis indépendamment parmi hydroxy, amino, halogène, oxo, C₁-C₄alcoxy, C₁-C₂haloalkyle, C₁-C₂haloalcoxy et mono- et di-C₁-C₄alkylamino ; et
R₉ est C₁-C₈alkyle, (C₃-C₇cycloalkyle)C₀-C₄alkyle ou phényle, substitués chacun avec 0 à 3 substituants choisis indépendamment parmi halogène, hydroxy, amino, cyano, nitro, -COOH, -CONH₂, C₁-C₄alkyle, C₂-C₄alcényle, C₂-C₄alcynyle, C₁-C₄alcoxy, (C₃-C₇cycloalkyle)-C₀-C₄alkyle, (C₃-C₇cycloalkyle)-C₀-C₄alcoxy, mono- et di-C₁-C₄alkylamino, C₁-C₂haloalkyle, C₁-C₂haloalcoxy et C₂-C₄alcanoyle.

2. Composé ou sel selon la revendication 1 de formule (a) 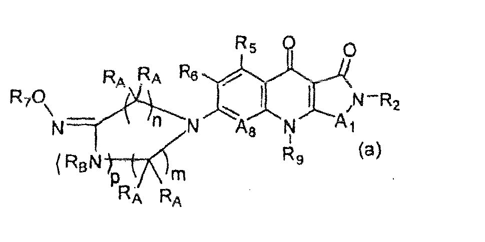 ou de formule (b)

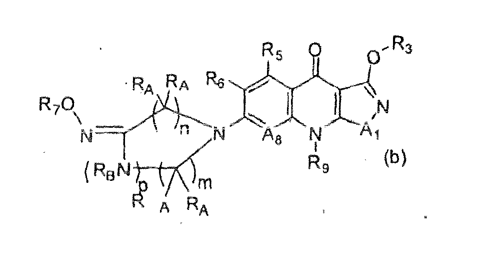

3. Composé ou sel selon l'une quelconque des revendications 1 et 2 où A₁ est S, SO, SO₂ ou O.

4. Composé ou sel selon la revendication 2 ou 3 où le composé ou sel est de formule (a) et R₂ est l'hydrogène, ou
R₂ est C₁-C₆alkyle ou (C₃-C₇cycloalkyle)-C₀-C₄alkyle, substitués chacun avec au moins un substituant choisi parmi hydroxy, amino, -COOH, -(C=O)NR₁₀OR₁₁ et -CONH₂;
et est substitué avec 0 à 3 substituants choisis indépendamment parmi halogène; hydroxy, amino, cyano, nitro, -COOH, -CONH₂, C₁-C₄alkyle, C₁-C₄alcoxy, C₁-C₂haloalkyle, C₁-C₂haloalcoxy, et mono- et di-C₁-C₄alkylamino et C₂-C₄alcanoyle.

5. Composé ou sel selon la revendication 4 où R₂ est l'hydrogène.

6. Composé ou sel selon l'une quelconque des revendications 2 à 3 où le composé est un composé de formule (b) et où R₃ est C₁-C₄alkyle ou C₂-C₄alcanoyle.

7. Composé ou sel selon l'une quelconque des revendications 1 à 6 où R₅ est l'hydrogène, amino, C₁-C₂alkyle, C₁-C₂alcoxy, mono- ou di-C₁-C₄alkylamino ou mono- ou di-C₁-C₄alkylhydrazinyle.

8. Composé ou sel selon la revendication 7 où
R₅ est l'hydrogène, amino, mono- ou di-C₁-C₂alkylamino ou mono- ou di-C₁-C₂alkylhydrazinyle.

9. Composé ou sel selon la revendication 7 où R₅ est l'hydrogène.

10. Composé ou sel selon l'une quelconque des revendications 1 à 8 où : R₆ est l'hydrogène, un halogène ou amino.

11. Composé ou sel selon la revendication 10 où R₆ est fluoro.

12. Composé ou sel selon l'une quelconque des revendications 1 à 11 où A₈ est l'azote ou CR₈.

13. Composé ou sel selon la revendication 12 où A₈ est CR₈ et où R₈ est l'hydrogène, un halogène, C₁-C₂alkyle, C₁-C₂alcoxy, C₁-C₂haloalkyle ou C₁-C₂haloalcoxy.

14. Composé ou sel selon la revendication 13 où R₈ est l'hydrogène ou méthoxy.

15. Composé ou sel selon l'une quelconque des revendications 1 à 14 où
R₉ est C₁-C₄alkyle, cyclopropyle ou phényle, substitués avec 0 à 3 substituants choisis indépendamment parmi halogène, hydroxy, amino, C₁-C₂alkyle, C₁-C₂alcoxy, mono- et di-C₁-C₂alkylamino, C₁-C₂haloalkyle et C₁-C₂haloalcoxy.

16. Composé ou sel selon la revendications 15 où
R₉ est C₁-C₄alkyle ou cyclopropyle, ou
R₉ est phényle substitué avec 2 substituants choisis parmi halogène, hydroxy, amino, C₁-C₂alkyle, C₁-C₂alcoxy, mono- et di-C₁-C₂alkylamino, C₁-C₂haloalkyle et C₁-C₂alcoxy.

17. Composé ou sel selon la revendication 15 où
R₉ est éthyle, t-butyle, cyclopropyle ou 2,4-difluorophényle.

18. Composé ou sel selon la revendication 15 où R₉ est cyclopropyle.

19. Composé ou sel selon l'une quelconque des revendications 1 à 18 où
R₇ est l'hydrogène, ou
R₇ est C₁-C₈alkyle, C₂-C₆alcanoyle, (C₃-C₇cycloalkyle)-C₀-C₄carbohydryle, (aryle)-C₀-C₄carbohydryle, (aryle)(C=O)-, mono- ou di-(C₁-C₆alkyl)-carboxamide, C₁-C₆alkylester ou (C₂-C₆hétérocycloalkyle)-C₀-C₄carbohydryle, substitués chacun avec 0 à 5 substituants choisis indépendamment parmi halogène, hydroxy, amino, cyano, nitro, C₁-C₄alkyle, C₁-C₄alcoxy, C₁-C₂haloalkyle, C₁-C₂haloalcoxy, mono- et di-C₁-C₄alkylamino, C₂-C₄alcanoyle, C₁-C₄alkylthio, -O(C=O)R₁₀, -(C=C)NR₁₀R₁₁, -O(C=O)NR₁₀R₁₁, = -(C=O)OR₁₀, -(C=O)NR₁₀OR₁₁, -NR₁₀(C=O)R₁₁, -NR₁₀(C=O)OR₁₁, -NR₁₀(C=O)NR₁₁R₁₂, -NR₁₀(C=S)NR₁₁,R₁₂, -NR₁₀NR₁₁R₁₂, -SO₃R₁₀, -(S=O)OR₁₀, -SO₂R₁₃, -SO₂NR₁₀R₁₁, ou -NR₁₀SO₂R₁₃.

20. Composé ou sel selon la revendication 19 où
R₇ est l'hydrogène, ou
R₇ est C₁-C₈alkyle, (C₃-C₇cycloalkyle)-C₀-C₄carbohydryle, (aryle)-C₀-C₄carbohydryle ou (C₂-C₆hétérocycloalkyle)-C₀-C₄carbohydryle, substitués chacun aven 0 à 5 substituants choisis indépendamment parmi halogène, hydroxy, amino, cyano, C₁-C₄alkyle, C₁-C₄alcoxy, C₁-C₂halo-alkyle, C₁-C₂haloalcoxy, mono- et di-C₁-C₄alkylamino, C₂-C₄alcanoyle, -(C=O)NR₁₀R₁₁, -NR₁₀(C=O)R₁₁

21. Composé ou sel selon la revendication 20 où
R₇ est l'hydrogène, ou
C₁-C₈alkyle ou (aryle)-C₀-C₄alkyle, chacun avec 0 à 5 substituants choisis indépendamment parmi halogène, hydroxy, amino, cyano, C₁-C₄alkyle, C₁-C₄alcoxy, C₁-C₂halkoalkyle, C₁-C₂haloalcoxy, mono- et di-C₁-C₄alkylamino, C₂-C₄alcanoyle, -(C=O)NR₁₀R₁₁, -NR₁₀(C=O)R₁₁.

22. Composé ou sel selon la revendication 20 où R₇ est l'hydrogène, C₁-C₂alkyle ou benzyle.

23. Composé ou sel selon l'une quelconque des revendications 1 à 22 où n est 1 ; m est 2, p est 1 et R_{B} est l'hydrogène.

24. Composé ou sel selon l'une quelconque des revendications 1 à 22 où p est 0.

25. Composé ou sel selon la revendication 24 où n est 1 et m est 2 ou bien où n est 2 et m est 2.

26. Composé ou sel selon l'une quelconque des revendications 1 à 25 où
chaque R_{A} est indépendamment (i), (ii) ou (iii) ; où
(i) est l'hydrogène, hydroxy, amino ou cyano,
(ii) est C₁-C₆alkyle, C₂-C₆alcényle, C₂-C₆alcynyle, (C₁-C₆alcoxy)-C₀-C₄alkyle, mono- ou di-C₁-C₄alkylamino, C₁-C₂haloalcoxy, (C₃-C₇cycloalkyle)-C₀-C₄carbohydryle, (C₄-C₇cycloalcényle)-C₀-C₄carbohydryle, (aryl)-C₀-C₆carbohydryle, (aryl)-C₁-C₄alcoxy, (C₂-C₆hétérocycloalkyle)-C₀-C₄carbohydryle, (hétéroaryle)-C₀-C₆carbohydryle, C₁-C₆alkylthio, -(C₀-C₄alkyle)(C=O)NR₁₀R₁₁, -(C₀-C₄alkyle)NR₁₀(C=O)R₁₁, =N-OH ou =N-O(C₀-C₄alkyle) ;
(iii) est -OR_{D} ou -(C=0)R_{D}, où R_{D} est C₁-C₄alkyle, (C₃-C₇cycloalkyle)-C₀-C₂alkyle, (C₂-C₆hétérocycloalkyle)-C₀-C₂alkyle; (aryle)-C₀-C₂alkyle ou (hétéroaryle)-C₀-C₂alkyle;
où chacun de (ii) et (iii) est substitué avec 0 à 3 substituants choisis indépendamment parmi halogène, hydroxy, amino, cyano, nitro, -COOH, -(C=O)OCH₃, -CONH₂, C₁-C₄alkyle, C₂-C₄alcényle, C₂-C₄alcynyle, C₁-C₄alcoxy, (C₃-C₇cycloalkyle)-C₀-C₄carbohydryle, (C₃-C₇cycloalkyle)-C₀-C₄alcoxy, mono- et di-C₁-C₄alkylamino, C₁-C₂haloalkyle, C₁-C₂haloalcoxy et C₂-C₄alcanoyle ; ou
deux R_{A} quelconques liés au même atome de carbone peuvent être joints pour former un groupe C₃-C₇cycloalkyle ou un groupe hétérocycloalkyle à 3 à 7 chaînons ayant 1 ou 2 hétéroatomes choisis indépendamment parmi N, O et S; substitués chacun avec 0 à 2 substituants choisis indépendamment parmi C₁-C₂alkyle et C₁-C₂alcoxy; ou
deux R_{A} quelconques liés à des atomes de carbone différents peuvent être joints pour former un groupe C₃-C₇cycloalkyle ou un groupe hétérocycloalkyle à 3 à 7 chaînons ayant 1 ou 2 hétéroatomes choisis indépendamment parmi N, O et S ; substitués chacun avec 0 à 2 substituants choisis indépendamment parmi C₁-C₂alkyle et C₁-C₂alcoxy.

27. Composé ou sel selon la revendication 26 où
chaque R_{A} est indépendamment (i) ou (ii) ; où
(i) est l'hydrogène ou amino,
(ii) est C₁-C₆alkyle, (C₁-C₆alcoxy)C₀-C₄alkyle, mono- ou di-C₁-C₄alkylamino, (C₃-C₇cycloalkyle)-C₀-C₄carbohydryle, (aryl)-C₀-C₆carbohydryle, (C₂-C₆hétérocycloalkyle)-C₀-C₄carbohydryle, (hétéroaryle)-C₀-C₆carbohydryle, -(C₀-C₄alkyle)(C=O)NR₁₀R₁₁, -(C₀-C₄alkyle)NR₁₀(C=O)R₁₁, =N-OH ou =N-O(C₀-C₄alkyle);
où chacun de (ii) est substitué avec 0 à 3 substituants choisis indépendamment parmi halogène, hydroxy, amino, cyano, nitro, -COOH, -(C=O)OCH₃, -CONH₂, C₁-C₄alkyle, C₂-C₄alcényle, C₂-C₄alcynyle, C₁-C₄alcoxy, (C₃-C₇cycloalkyle)-C₀-C₄carbohydryle, (C₃-C₇cycloalkyle)-C₀-C₄alcoxy, mono- et di-C₁-C₄alkyl-amino; C₁-C₂haloalkyle, C₁-C₂haloalcoxy et C₂-C₄alcanoyle ; ou
deux R_{A} quelconques liés au même atome de carbone peuvent être joints pour former un groupe C₃-C₇cyloalkyle ou un groupe hétérocycloalkyle à 3 à 7 chaînons ayant 1 ou 2 hétéroatomes choisis indépendamment parmi N, O et S ; substitués chacun aven 0 à 2 substituants choisis indépendamment parmi C₁-C₂-alkyle est C₁-C₂alcoxy ; ou
deux R_{A} quelconques liés à des atomes de carbone différents peuvent être joints pour former un groupe C₃-C₇cycloalkyle ou hétérocycloalkyle ayant 1 ou 2 hétéroatomes choisis indépendamment parmi N, O et S ; substitués chacun avec 0 à 2 substituants choisis ïndépendamment parmi C₁-C₂alkyle et C₁-C₂alcoxy.

28. Composé ou sel selon la revendication 26 où
chaque R_{A} est indépendamment (i) ou (ii) ; où
(i) est l'hydrogène ou amino ;
(ii) est C₁-C₆alkyle, mono- ou di-C₁-C₄alkylamino, (C₃-C₇cycloalkyle)-C₀-C₄alkyle, (C₂-C₆hétérocycloalkyle)-C₀-C₄alkyle, -(C₀-C₄alkyle)(C=O)NR₁₀R₁₁, -(C₀-C₄alkyle)NR₁₀(C=O)R₁₁, =N-OH ou =N-O(C₀-C₄alkyle) ;
où chacun de (ii) est substitué avec 0 à 3 substituants choisis indépendamment parmi hydroxy, amino, cyano et -CONH₂,
deux R_{A} quelconques liés au même atome de carbone peuvent être joints pour former un groupe C₃-C₇cycloalkyle ou hétérocycloalkyle ayant 1 ou 2 hétéroatomes choisis indépendamment parmi N, O et S ; substitués chacun avec 0 à 2 substituants choisis indépendamment parmi C₁-C₂alkyle et C₁-C₂alcoxy.

29. Composé ou sel selon la revendication 26 où
chaque R_{A} est indépendamment (i) ou (ii) ; où
(i) est l'hydrogène ou amino ;
(ii) est C₁-C₆alkyle, (C₃-C₇cycloalkyle)-C₀-C₂alkyle ou mono- ou di-C₁-C₄alkylamino, où chacun de (ii) est substitué avec 0 à 3 amino,
deux R_{A} quelconques liés au même atome de carbone peuvent être joints pour former un groupe C₃-C₇cycloalkyle ou un groupe hétérocycloalkyle à 3 à 7 chaînons ayant 1 ou 2 hétéroatomes choisis indépendamment parmi N, O et S ; substitués chacun avec 0 à 2 substituants choisis indépendamment parmi C₁-C₂alkyle et C₁-C₂alcoxy ; ou
deux R_{A} quelconques liés à des atomes de carbone différents peuvent être joints pour former un groupe C₃-C₇cycloalkyle ou un groupe hétérocycloalkyle à chaînons avant 1 ou 2 hétéroatomes choisis indépendamment parmi N, O et S ; substitués chacun avec 0 à 2 substituants choisis indépendamment parmi C₁-C₂-alkyle et C₁-C₂alcoxy.

30. Composé ou sel selon la revendication 26 où
chaque R_{A} est indépendamment l'hydrogène, C₁-C₄alkyle ou C₁-C₄alkyle substitué avec un substituant amino.

31. Composé ou sel selon la revendication 30 où : un ou deux R_{A} sont C₁-C₄alkyle substitué avec un substituant amino et les R_{A} restants sont l'hydrogène.

32. Composé ou sel selon la revendication 26 où
deux R_{A} liés au même atome de carbone sont joints pour former un groupe C₃-C₇cycloalkyle ou un groupe hétérocycloalkyle à 3 à 7 chaînons ayant 1 ou 2 hétéroatomes choisis indépendamment parmi N, O et S ; substitués chacun avec 0 à 2 substituants choisis indépendamment parmi C₁-C₂alkyle et C₁-C₂alcoxy ; et les R_{A} restants sont choisis indépendamment parmi l'hydrogène, C₁-C₂alkyle et C₁-C₂alcoxy,

33. Composé ou sel selon la revendication 32
deux R_{A} liés au même atome de carbone sont joints pour former un groupe C₃-C₄cycloalkyle ou un groupe hétérocycloalkyle à 3 à 4 chaînons ayant 1 atome N ; substitués chacun avec 0 à 2 substituants choisis indépendamment parmi C₁-C₂alkyle et C₁-C₂alcoxy et les R_{A} restants sont choisis indépendamment parmi l'hydrogène, C₁-C₂alkyle et C₁-C₂alcoxy.

34. Composé ou sel de formule (a) selon la revendication 2 où :
R₂ est l'hydrogène ou C₁-C₆alkyle substitué avec un substituant choisi parmi hydroxy, amino, -COOH, -(C=O)NR₁₀OR₁₁ et -CONH₂ ;
R₅ est l'hydrogène ;
R₆ est un halogène ou amino,
R₇ est l'hydrogène, C₁-C₂alkyle ou benzyle ;
A₈ est l'azote ou CR₈, et quant A₈ est CR₈, R₈ est l'hydrogène ou méthoxy
R₉ est éthyle, t-butyle, cyclopropyle ou 2,4-difluorophényle ; et
chaque R_{A} est l'hydrogène ou C₁-C₄alkyle éventuellement substitué avec un substituant amino ; ou
deux R_{A} quelconques liés au même atome de carbone peuvent être joints pour former un groupe C₃-C₇cycloalkyle ou un groupe hétérocycloalkyle à 3 à 7 chaînons ayant 1 ou 2 hétéroatomes choisis indépendamment parmi N, O et S ; substitués chacun avec 0 à 2 substituants choisis indépendamment parmi C₁-C₂alkyle et C₁-C₂alcoxy ; ou
deux R_{A} quelconques liés à des atomes de carbone différents peuvent être joints pour former un groupe C₃-C₇cycloalkyle ou hétérocycloalkyle ayant 1 ou 2 hétéroatomes choisis indépendamment parmi N, O et S ; substitués chacun avec 0 à 2 substituants choisis indépendamment parmi C₁-C₂alkyle et C₁-C₂alcoxy.

35. Composé ou sel selon la revendication 1 où le composé est
7-[(4Z)-3-aminométhyl-4-méthoxyimjno-pyrrolidin-1-yl)]-9-cyclopropyl-6-fluoro-9H-isothiazolo[5,4-b]quinoléine-3,4-dione,
7-[(3R,4Z)-3-aminométhyl-4-méthoxyimino-pyrrolidin-1-yl)]-9-cyclopropyl-6-fluoro-9H-isothiazolo[5,4-b]quinoléine-3,4-dione;
7-[(3S,4Z)-3-aminométhyl-4-méthoxyimino-pyrrolidin-1-yl)]-9-cyclopropyl-6-fluoro-9H-isothiazolo[5,4-b]quinoléine-3,4-dione,
7-[(4E)-3-aminométhyl-4-méthoxyimino-pyrrolidin-1-yl)]-9-cyclopropyl-6-fluoro-9H-isothiazolo[5,4-b]quinoléine-3,4-dione;
7-[(3R,4E)-3-aminométhyl-4-méthoxyimino-pyrrolidin-1-yl)]-9-cyclopropyl-6-fluoro-9H-isothiazolo[5,4-b]quinoléine-3,4-dione-,
7-[(3S,4E)-3-aminométhyl-4-méthoxyimino-pyrrolidin-1-yl)]-9-cyclopropyl-6-fluoro-9H-isothiazolo[5,4-b]quinoléine-3,4-dione;
9-cyclopropyl-6-fluoro-7-(8-méthoxyimino-2,6-diaza-spiro[3.4]oct-6-yl)-9H-isothiazolo[5,4-b]-quinoléine-3,4-dione;
9-cyclopropyl-6-fluoro-7-(7-méthoxyimino-1,5-diaza-spiro[2.4]hept-5-yl)-9H-isothiazolo[5,4-b]quinoléine-3,4-dione;
7-[3-(1-amino-cyclopropyl)-4-méthoxyimino-pyrrolidin-1-yl]-9-cyclopropyl-6-fluoro-9H-isothiazolo[5,4-b]quinoléine-3,4-dione;
9-cyclopropyl-6-fluoro-7-(7-méthoxyimino-5-aza-spiro[2.4]hept-5-yl)-9H-isothiazolo[5,4-b]quinoléine-3,4-dione;
7-(3-aminométhyl-4-hydroxyimino-pyrrolidin-1-yl)-9-cyclopropyl-6-fluoro-9H-isothiazolo[5,4-b]quinoléine-3,4-dione;
7-[3-(2-amino-éthyl)-4-méthoxyimino-pyrrolidin-1-yl]-9-cyclopropyl-6-fluoro-9H-isothiazolo[5,4-b]quinoléine-3,4-dione;
7-(3-aminométhyl-4-benzyloxyimino-pyrrolidin-1-yl)-9-cyclopropyl-6-fluoro-9H-isothiazolo[5,4-b]quinoléine-3,4-dione;
7-(3-amino-4-méthoxyimino-pyrrolidin-1-yl)-9-cyclopropyl-6-fluoro-9H-isothiazolo[5,4b]quinoléine-3,4-dione;
7-(3-aminométhyl-4-méthoxyimino-3-méthyl-pyrrolidin-1-yl)-9-cyclopropyl-6-fluoro-9H-isothiazolo[5,4-b]quinoléine-3,4-dione;
7-[3-(1-amino-éthyl)-4-méthoxyimino-pyrrolidin-1-yl]-9-cyclopropyl-6-fluoro-9H-isothiazolo-[5,4-b]quinoléine-3,4-dione;
7-(3,3-bis-aminométhyl-4-méthoxyimino-pyrrolidin-1-yl)-9-cyclopropyl-6-fluoro-9H-isothiazolo[5,4-b]quinoléine-3,4-dione;
7-[3-(1-amino-1-méthyl-éthyl)-4-méthoxyimino-pyrrolidin-1-yl]-9-cyclopropyl-6-fluoro-9H-isothiazolo[5,4-b]quinoléine-3,4-dione;
9-cyclopropyl-6-fluoro-7-(3-méthoxyimino-pipérazin-1-yl)-9H-isothiazolo[5,4-b]quinoléine-3,4-dione;
9-cyclopropyl-6-fluoro-7-(4-méthoxyimino-pipéridin-1-yl)-9H-isothiazolo[5,4-b]quinoléine-3,4-dione;
7-(3-aminométhyl1-4-méthoxyimino-pipéridin-1-yl)-9-cyclopropyl-6-fluro-9H-isothiazolo[5,4b]quinoléine-3,4-dione;
7-(3-aminométhyl-4-méthoxyimino-pyrrolidin-1-yl)-9-cyclopropyl-6-fluoro-1,1-dioxo-1,2-dihydro-9H-116-isothiazolo[5,4-b]quinoléine-3,4-dione;
acide 4-[7-(3-aminométhyl-4-méthoxyimino-pyrrolidin-1-yl)-9-cyclopropyl-6-fluoro-3,4-dioxo-4,9-dihydro-3H-isothiazolo[5,4-b]quinolin-2-yl]-butyrique ;
7-(3-aminométhyl-4-méthoxyimino-pyrrolidin-1-yl)-9-cyclopropyl-6-fluoro-4-oxo-4,9-dihydro-isothiazolo[5,4-b]quinolin-3-ylester de l'acide acétique;
7-(3-aminométhyl-4-méthoxyimino-pyrrolidin-1-yl)-9-cyclopropyl-6-fluoro-5-(N'-méthylhydrazino)-9H-isothiazolo[5,4-b]quinoléine-3,4-dione;
6-amino-7-(3-aminométhyl-4-méthoxyimino-pyrrolidin-1-yl)-9-cyclopropyl-9H-isothiazolo[5,4b]quinoléine-3,4-dione;
7-(3-aminométhyl-4-méthoxyimino-pyrrolidin-1-yl)-9-cyclopropyl-6-fluoro-8-méthoxy-9H-isothiazolo[5,4-b]quinoléine-3,4-dione; et
7-(3-aminométhyl-4-méthoxyimino-pyrrolidin-1-yl)-9-cyclopropyl-8-méthoxy-9H-isothiazolo[5,4-b]quinotéine-3,4-dione.

36. Composition antibactérienne comprenant un composé ou sel selon l'une quelconque des revendications 1 à 35, en même temps qu'un véhicule, diluant ou excipient.

37. Composition pharmaceutique comprenant un composé ou sel selon l'une quelconque des revendications 1 à 35, en même temps qu'un véhicule, diluant ou excipient pharmaceutiquement acceptable.

38. Composition pharmaceutique selon la revendication 36 où la composition est formulée sous forme d'un fluide injectable, d'un aérosol, d'une crème, d'un gel, d'une pilule, d'une capsule, d'un comprimé, d'un sirop, d'un timbre transdermique ou d'une solution ophtalmique.

39. Composition comprenant un composé ou sel selon l'une quelconque des revendications 1 à 35 en combinaison avec un ou plusieurs autres agents antibactériens, agents antiprotozoaires, agents antifongiques, agents antiviraux, interférons, inhibiteurs de pompe à efflux ou inhibiteurs de bêta-lactamase.

40. Conditionnement comprenant la composition pharmaceutique selon la revendication 39 dans un récipient et comprenant en outre des instructions pour utiliser la composition pour traiter un patient souffrant d'une infection par des micro-organismes.

41. Conditionnement selon la revendication 40 où les instructions sont des instructions pour utiliser la composition pour traiter un patient souffrant d'une infection bactérienne.

42. Composé ou sel selon l'une quelconque des revendications 1 à 35 destiné à être utilisé dans un procédé pour traiter ou prévenir une infection bactérienne ou par des protozoaires.

43. Composé selon la revendication 42 où l'infection bactérienne ou par des protozoaires est une infection des voies urinaires, une pyélonéphrite, une infection des voies respiratoires inférieures, une infection cutanée, une infection de structures cutanées, une infection gonococcique urétrale, une infection gonococcique cervicale, une infection à Chlamydia urétrale, une infection à Chlamydia cervicale, une infection osseuse, une infection articulaire, une infection par des bactéries gram-négatives, une diarrhée infectieuse, une fièvre typhoïde, prostatite, une sinusite aiguë, une exacerbation aiguë de bronchite chronique, une pneumonie, une infection infra-abdominale, une infection gynécologique ou une infection pelvienne.

44. Composé selon la revendication 42 où l'animal est un poisson, un amphibien, un reptile, un oiseau ou un mammifère.

45. Composé selon la revendication 44 où l'animal est un mammifère.

46. Composé selon la revendication 45 où le mammifère est un humain.

47. Composé ou sel selon l'une quelconque des revendications 1 à 35 qui présente une CIM de 10 µg/ml ou moins contre *S*. *aureus* ou *E. coli.*

48. Composé ou sel selon l'une quelconque des revendications 1 à 35 qui présente une CIM de 1 µg/ml ou moins contre *S. aureus* ou *E. coli.*

49. Utilisation d'un composé selon l'une quelconque des revendications 1 à 35 pour la fabrication d'un médicament pour le traitement d'une infection bactérienne ou par des protozoaires.

50. Utilisation selon la revendication 49 où l'infection bactérienne ou par des protozoaires est une infection des voies urinaires, une pyélonéphrite, une infection des voies respiratoires inférieures, une infection cutanée, une infection de structures cutanées, une infection gonococcique urétrale, une infection gonococcique cervicale, une infection à Chlamydia urétrale, une infection à Chlamydia cervicale, une infection osseuse, une infection articulaire, une infection par des bactéries gram-négatives, une diarrhée infectieuse, une fièvre typhoïde, une prostatite, une sinusite aiguë, une exacerbation aiguë de bronchite chronique, une pneumonie, une infection intra-abdominale, une infection gynécologique ou une infection pelvienne.

51. Utilisation selon la revendication 49 où l'infection bactérienne est une infection à S.aureus ou E. coli.
